(19) Europäisches Patentamt
European Patent Office
Office européen des brevets

(11) EP 2 111 861 A1

(12) EUROPEAN PATENT APPLICATION

(43) Date of publication:
**28.10.2009 Bulletin 2009/44**

(21) Application number: **08154898.4**

(22) Date of filing: **21.04.2008**

(51) Int Cl.:
***A61K 31/4174*** *(2006.01)* ***A61K 31/422*** *(2006.01)*
***A61K 31/46*** *(2006.01)* ***A61K 31/57*** *(2006.01)*
***A61K 45/06*** *(2006.01)* ***A61K 31/519*** *(2006.01)*
***A61P 29/00*** *(2006.01)* ***A61P 37/00*** *(2006.01)*
***A61P 11/00*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Ranbaxy Laboratories Limited**
**Haryana 122001, Delhi (IN)**

(72) Inventors:
- **Ray, Abhijit**
  **110070, Delhi (IN)**
- **Dastidar, Sunanda G.**
  **110044, Delhi (IN)**
- **Shirumalla, Rajkumar**
  **110018, Delhi (IN)**
- **Gupta, Suman**
  **122001, Gurgaon (IN)**

(74) Representative: **Cronin, Brian Harold John**
**CRONIN Intellectual Property**
**Chemin de Précossy 31**
**1260 Nyon (CH)**

# (54) Compositions of phosphodiesterase type IV inhibitors

(57) Provided herein are pharmaceutical compositions comprising one or more phosphodiesterase inhibitors of type IV ("PDE-IV"), and atleast one other active ingredients selected from muscarinic receptor antagonists (MRA), β2-agonists, p38 MAP Kinase inhibitors, and corticosteroids and optionally one or more pharmaceutically acceptable excipients and/or other therapeutic agents. In addition, methods of treating autoimmune, inflammatory or allergic diseases or disorders are provided.

EP 2 111 861 A1

## Description

### Field of the Invention

Provided herein are pharmaceutical compositions comprising one or more phosphodiesterase inhibitors of type IV ("PDE-IV"), and at least one other active ingredient such as muscarinic receptor antagonists (MRA), β2-agonists, p38 MAP Kinase inhibitors, or corticosteroids and optionally one or more pharmaceutically acceptable excipients. In addition, methods of treating autoimmune, inflammatory or allergic diseases or disorders are provided.

### Background of the Invention

It is known that cyclic adenosine-3', 5'-monophosphate (cAMP) exhibits an important role of acting as an intracellular secondary messenger. The intracellular hydrolysis of cAMP to adenosine 5'-monophosphate (AMP) causes a number of inflammatory conditions, which include, but are not limited to, psoriasis, allergic rhinitis, shock, atopic dermatitis, Crohn's disease, adult respiratory distress syndrome (ARDS), eosinophilic granuloma, allergic conjunctivitis, osteoarthritis, and ulcerative colitis. Cyclic nucleotide phosphodiesterases (PDE), a biochemically and functionally, highly variable superfamily of the enzyme, is the most important factor in the control of cAMP (as well as of cGMP) levels. Eight distinct families with more than 15 gene products are currently recognized. Although PDE I, PDE II, PDE III, PDE IV, and PDE VII all use cAMP as a substrate, only the PDE IV and PDE VII types are highly selective for hydrolysis of cAMP. Accordingly, inhibitors of PDE, particularly the PDE IV inhibitors, such as rolipram or Ro-1724, are known as cAMP-enhancers. Immune cells contain PDE IV and PDE III, of which PDE IV is prevalent in human mononuclear cells. Thus, the inhibition of phosphodiesterase type IV has been a target for modulation and, accordingly, for therapeutic intervention in a range of disease processes. The initial observation that xanthine derivatives, theophylline and caffeine inhibit the hydrolysis of cAMP led to the discovery of the required hydrolytic activity in the cyclic nucleotide phosphodiesterase (PDE) enzymes. More recently, distinct classes of PDE have been recognized, and their selective inhibition has led to improved drug therapy. Thus, it was recognized that inhibition of PDE IV could lead to inhibition of inflammatory mediator release and airway smooth muscle relaxation.

Particular 3-aryl-2-isoxazoline compounds are known as anti-inflammatory agents and particular isoxazoline compounds are known as inhibitors of TNF release. However, there remains a need for new selective inhibitors of phosphodiesterase (PDE) type IV, as well as compositions thereof in combination with one or more other therapeutic agents.

### Summary of the Invention

In one aspect, provided are pharmaceutical compositions comprising one or more phosphodiesterase inhibitors of type IV ("PDE-IV"), and at least one other active ingredient such as muscarinic receptor antagonists (MRA), β2-agonists, p38 MAP Kinase inhibitors, or corticosteroids and optionally one or more pharmaceutically acceptable excipients, wherein the PDE-IV inhibitor is one or more compounds having the structure of Formula Ia or Formula Ib, wherein:

a. Formula Ia is:

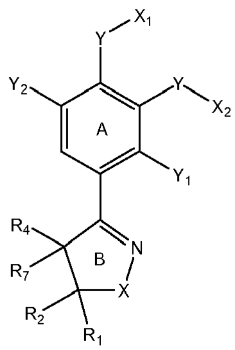

FORMULA Ia

and its pharmaceutically acceptable salts, pharmaceutically acceptable solvates, enantiomers, diastereomers or N-oxides, wherein

**When X is oxygen,**

**$R_1$** can be hydrogen, alkyl, heterocyclyl, $-(CH_2)_mC(=O)R_3$, or $(CH_2)_{1-4}OR'$, (wherein m is an integer 0-2, $R_3$ can be alkyl, cycloalkyl, heterocyclyl, or optionally substituted $R_p$ or $R_q$, wherein $R_p$ can be heterocyclyl or heteroaryl ring, wherein the rings are attached to $(CH_2)_mC(=O)$ through N, and $R_q$ can be heterocyclyl or heteroaryl ring wherein the rings are attached to $-(CH_2)_mC(=O)$ through C, and wherein R' can be can be alkyl, alkenyl, alkynyl, saturated or unsaturated cycloalkyl, aryl, heterocyclyl or heteroaryl);

**$R_2$** can be $(CH_2)_mC(=O)R_3$, $-(CH_2)_{1-4}OR'$, or $C(=O)NR_xR_y$ {where m, $R_3$ and R' are as defined above, and wherein $R_x$ and $R_y$ each independently can be hydrogen, alkyl, $C_3$-$C_6$ alkenyl, $C_3$-$C_6$ alkynyl, cycloalkyl, carboxy, $-S(O)_mR_5$ (wherein $R_5$ can be hydrogen, alkyl, alkenyl, alkynyl, aryl, cycloalkyl, alkaryl, heteroaryl, heteroarylalkyl, heterocyclyl or heterocyclylalkyl), aryl, alkaryl, heteroaryl, heterocyclyl, heteroarylalkyl, or heterocyclylalkyl}, or **$R_1$** and **$R_2$** together form an optionally substituted cycloalkyl or heterocyclyl ring wherein the optional substituent is oxo, alkyl, alkenyl, alkynyl, halogen, nitro, $-NH_2$, $-NHC(=O)OR_6$, $-C(=O)NR_xR_y$, cyano, hydroxy, alkoxy, or substituted amino (wherein $R_6$ can be alkyl, alkenyl, alkynyl, cycloalkyl, alkaryl, heteroarylalkyl or heterocyclylalkyl), with the proviso that if $R_1$ is $-(CH_2)_{1-4}OR'$, then $R_2$ is also $-(CH_2)_{1-4}OR'$, and with the proviso that if $R_1$ is $C(=O)NR_xR_y$, then $R_2$ is also $C(=O)NR_xR_y$;

**$R_4$** can be hydrogen; alkyl; $-OR_5$; halogen; $-NH_2$, substituted amino; cyano; carboxy; or $-C(=O)NR_xR_y$ (wherein $R_5$, $R_x$ and $R_y$ are as defined above); or **$R_2$** and **$R_4$** forms an optionally substituted 4-12 membered saturated or unsaturated monocyclic or bicyclic ring system fused to ring B having 0-4 heteroatom(s) selected from the group consisting of N, O and S, wherein the substituents can be one or more of alkyl, halogen, hydroxy, alkoxy, $-NH_2$ or substituted amino (wherein $R_3$ and $R_x$ and $R_y$ are as defined above), with the proviso that $R_2$ and $R_4$ together does not form $-CH_2-O-CH_2-O-CH_2-$;

**$R_7$** can be hydrogen, alkyl, alkenyl, alkynyl, $-OR_5$, halogen, cyano,$-NH_2$, or substituted amino;

**$X_1$** and **$X_2$** each independently can be hydrogen, alkyl, alkaryl, cycloalkyl, alkaryl, alkenyl, cycloalkylalkyl, heterocyclyl, heteroaryl, heterocyclylalkyl, $-(CH_2)_gC(=O)NR_xR_y$, $-(CH_2)_{g1}C(=O)OR_3$ or heteroarylalkyl; wherein $g_1$ can be an integer from 1-3 (wherein $R_x$, $R_y$, g and $R_3$ are as defined above);

**Y** can each independently be an oxygen atom; a sulphur atom; or -NR (wherein R can be can be hydrogen, acyl, aryl, or alkyl);

**$Y_1$** and **$Y_2$** each independently can be hydrogen; alkyl; -OR; -SR; or -NHR (wherein R is as defined above); wherein any of **$Y_1$** and **$X_2$** & **$X_1$** and **$Y_2$** together optionally form a ring fused with the ring A, the ring containing 3-5 carbon atoms within the ring and having 1-3 heteroatoms such as N, O and S, and **$X_1$** and **$X_2$** can together optionally form a ring fused with ring A, the ring containing 3-5 carbon atoms within the ring and having 2-3 heteroatoms such as N, O or S, and

**When X is $NR_7$, or S (wherein $R_7$, can be hydrogen, or $C_{1-6}$ alkyl)**

**$R_1$** and **$R_2$** can each independently be alkyl, alkenyl, alkynyl, alkoxy, hydroxy, cyano, nitro, halogen, heteroaryl, heterocyclyl, heteroarylalkyl, heterocyclylalkyl, $NH_2$, substituted amino, carboxy, $-(CH_2)_mC(=O)R_3$, $-C(=O)NR_xR_y$, or $(CH_2)_{1-4}OR'$, {wherein m is an integer 0-2, $R_3$ can be alkyl, cycloalkyl, heterocyclyl, or optionally substituted $R_p$ or $R_q$ (wherein $R_p$ can be heterocyclyl or heteroaryl ring, wherein the rings are attached to $(CH_2)_mC(=O)$ through N, and $R_q$ can be heterocyclyl or heteroaryl ring wherein the rings are attached to $-(CH2)_mC(=O)$ through C), wherein R' can be can be alkyl, alkenyl, alkynyl, saturated or unsaturated cycloalkyl, aryl, heterocyclyl or heteroaryl, and wherein $R_x$ and $R_y$ each independently can be hydrogen, alkyl, $C_3$-$C_6$ alkenyl, $C_3$-$C_6$ alkynyl, cycloalkyl, carboxy, $-S(O)_mR_5$ (wherein $R_5$ can be hydrogen, alkyl, alkenyl, alkynyl, aryl, cycloalkyl, alkaryl, heteroaryl, heteroarylalkyl, heterocyclyl or heterocyclylalkyl), aryl, alkaryl, heteroaryl, heterocyclyl, heteroarylalkyl, or heterocyclylalkyl}, or **$R_1$** and **$R_2$** together can form an optionally substituted cycloalkyl or heterocyclyl ring wherein the optional substituent is oxo, alkyl, alkenyl, alkynyl, halogen, nitro, $-NH_2$, $-NHC(=O)OR_6$ (wherein $R_6$ can be alkyl, alkenyl, alkynyl, cycloalkyl, alkaryl, heteroarylalkyl or heterocyclylalkyl), $-C(=O)NR_xR_y$, cyano, hydroxy, alkoxy, or substituted amino;

**$R_4$** can be hydrogen; alkyl; $-OR_5$; halogen; $-NH_2$, substituted amino; cyano; carboxy; or $-C(=O)NR_xR_y$ (wherein $R_5$, $R_x$ and $R_y$ are as defined above); or **$R_2$** and **$R_4$** forms an optionally substituted 4-12 membered saturated or unsaturated monocyclic or bicyclic ring system fused to ring B having 0-4 heteroatom(s) selected from the group consisting of N, O and S, wherein the substituents can be one

or more of alkyl, halogen, hydroxy, alkoxy or substituted amino (wherein $R_3$ and $R_x$ and $R_y$ are as defined above), with the proviso that $R_2$ and $R_4$ together does not form - $CH_2$-O-$CH_2$-O-$CH_2$-;

**$R_7$** can be hydrogen, alkyl, alkenyl, alkynyl, -$OR_5$, halogen, cyano,-$NH_2$, or substituted amino;

**$X_1$ and $X_2$** each independently can be alkyl, cycloalkyl, alkaryl, heteroaryl, heterocyclyl, heteroarylalkyl, or heterocyclylalkyl;

**Y** can each independently be an oxygen atom; a sulphur atom; or -NR (wherein R can be can be hydrogen, acyl, aryl, or alkyl);

**$Y_1$ and $Y_2$** each independently can be hydrogen, alkyl, -OR, -SR, or -NHR (wherein R is as defined above); wherein any of **$Y_1$** and **$X_2$** & **$X_1$** and **$Y_2$** together optionally form a ring fused with the ring A, the ring containing 3-5 carbon atoms within the ring and having 1-3 heteroatoms such as N, O and S;

**$X_1$** and **$X_2$** can together optionally forms a cyclic ring fused with the ring A, the ring containing 3-5 carbon atoms within the ring and having 2-3 heteroatoms such as N, O or S.

b. Formula Ib is:

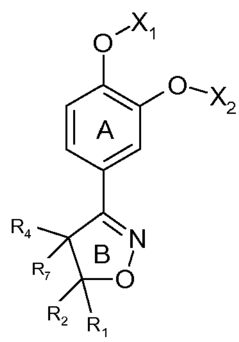

Formula Ib

and its pharmaceutically acceptable salts, pharmaceutically acceptable solvates, enantiomers, diastereomers or N-oxides, wherein

**$R_1$** and **$R_2$** together forms an optionally substituted cycloalkyl or heterocyclyl ring

wherein one or more optional substituent are oxo, alkyl, alkaryl, alkenyl, alkynes, heterocyclylalkyl, cycloalkylalkyl, -$SO_2NR_xR_y$, halogen, -$NH_2$, -$(CH_2)_gC(=O)NR_xR_y$, - NHC(=O)$OR_6$, -NHC(=O)$NR_xR_y$, -C(=O)$OR_3$, -NHC(=O)$R_x$, -$SO_2R_3$, cyano, hydroxy, alkoxy, substituted amino, or -C(=O)$R_3$ (wherein $R_xR_y$ g, $R_6$ and $R_3$ are as defined above);

**$R_4$** can be hydrogen; alkyl, hydroxyl, halogen, or carboxy;

**$R_7$** can be hydrogen, or alkyl;

**$R_1$** can be independently hydrogen or alkyl and **$R_2$** and **$R_4$** forms an optionally substituted 4-12 membered saturated or unsaturated monocyclic or bicyclic ring system fused to ring B having 0-4 heteroatom(s) selected from the group consisting of N, O and S, wherein the substituents is one or more of oxo, alkyl, -C(=O)$OR_3$, -$SO_2R_3$, halogen, hydroxy, alkoxy, -$NH_2$ or substituted amino (wherein $R_3$ is as defined below), with the proviso that $R_2$ and $R_4$ together does not form -$CH_2$-O-$CH_2$-O-$CH_2$-;

**$X_1$** and **$X_2$** can be hydrogen, alkyl, cycloalkyl, alkaryl, alkenyl, cycloalkylalkyl, heteroaryl, heterocyclyl, heteroarylalkyl, heterocyclylalkyl, -$(CH_2)_gC(=O)NR_xR_y$ or - $(CH_2)_{g1}C(=O)OR_3$ (wherein g can be an integer from 0-3 and $g_1$ can be an integer from 1-3, and $R_x$, $R_y$ and $R_3$ are as defined below);

**$X_1$** and **$X_2$** together can optionally form a cyclic ring fused with the ring A shown in Formula I, the ring containing 3-5 carbon atoms within the ring and having 2-3 heteroatoms N, O or S;

wherein **$R_3$** can be alkyl, cycloalkyl or heterocyclyl;

wherein the halogen can be F, Cl, Br, or I; **$R_x$** and **$R_y$** each independently can be hydrogen, alkyl, $C_3$-$C_6$ alkenyl, $C_3$-$C_6$ alkynyl, carboxy, cycloalkyl, -$S(O)_mR_5$, aryl, alkaryl, heteroaryl, heterocyclyl, heteroarylalkyl, and heterocyclylalkyl; **m** can be an integer between 0-2; **$R_6$** can be alkyl, alkenyl, alkynyl, cycloalkyl, alkaryl, heteroarylalkyl or heterocyclylalkyl; wherein **$R_5$** can be hydrogen, alkyl, alkenyl, alkynyl, aryl, cycloalkyl, alkaryl, heteroaryl, heteroarylalkyl, heterocyclyl or heterocyclylalkyl.

In another aspect, provided are pharmaceutical compositions comprising one or more phosphodiesterase inhibitors of type IV ("PDE-IV"), and at least one other active ingredient such as muscarinic receptor antagonists (MRA), β2-agonists, p38 MAP Kinase inhibitors, and corticosteroids and one or more pharmaceutically acceptable excipients, wherein the PDE-IV inhibitor is one or more compounds having the structure of Formula Ia and Formula Ib, as described

herein.

The pharmaceutical compositions of each of the above aspects can include one or more of the following embodiments. For example, the one or more compounds of Formula Ia and Formula Ib may be chosen from among members of a list of such compounds presented herein:

In another embodiment, β2-agonists can be selected from albuterol, salbutamol, biltolterol, pirbuterol, levosalbutamol, tulobuterol, terbutaline, bambuterol, metaproterenol, fenoterol, salmeterol, carmoterol, arformoterol, formoterol, or their pharmaceutically acceptable salts or solvates thereof. In yet another embodiment, corticosteroids can be selected from alclometasone, amcinonide, amelometasone, beclometasone, betamethasone, budesonide, ciclesonide, clobetasol, cloticasone, cyclomethasone, deflazacort, deprodone, dexbudesonide, diflorasone, difluprednate, fluticasone, flunisolide, halometasone, halopredone, hydrocortisone, hydrocortisone, methylprednisolone, mometasone, prednicarbate, prednisolone, rimexolone, tixocortol, triamcinolone, ulobetasol, or pharmaceutically acceptable salts or solvates thereof.

In another embodiment, p38 kinase inhibitors can be selected from 1-[5-tert-butyl-2-p-tolyl-2H-pyrazol-3-yl]-3-[4-(2-morpholin-4-ylethoxy)naphthalen-1-yl]urea; 1-[5-tert-butyl-2-p-tolyl-2H-pyrazol-3-yl]-3-[4-(2-(1-oxothiomorpholin-4-yl)ethoxy)naphthalen-l-yl]urea; 1-[5-tert-butyl-2-(2-methylpyridin-5-yl)-2H-pyrazol-3-yl]-3-[4-(2-pyridin-4-ylethoxy) naphthalen-1-yl]urea; 1-[5-tert-butyl-2-(2-methoxypyridin-5-yl)-2H-pyrazol-3-yl]-3-[4-(2-morpholin-4-ylethoxy)naphthalen-1-yl]urea; and 1-[5-tert-butyl-2-methyl-2H-pyrazol-3-yl]-3-[4-(2-morpholin-4-ylethoxy)naphthalen-1-yl]urea disclosed in our copending United States Patent Application No. 60/605,344;

Other p38 kinase inhibitors can be selected from for example members of a list of such compounds presented herein:

In another embodiment, corticosteroids can be selected from alclometasone, amcinonide, amelometasone, beclometasone, betamethasone, budesonide, ciclesonide, clobetasol, cloticasone, cyclomethasone, deflazacort, deprodone, dexbudesonide, diflorasone, difluprednate, fluticasone, flunisolide, halometasone, halopredone, hydrocortisone, hydrocortisone, methylprednisolone, mometasone, prednicarbate, prednisolone, rimexolone, tixocortol, triamcinolone, tolterodine, oxybutynin, ulobetasol, rofleponide, KSR 592, as disclosed in US Patent 4,285,937, ST-126, as disclosed in EP 1344526, dexamethasone and pharmaceutically acceptable salts, solvates thereof. Preferred corticosteroids include, for example, flunisolide, beclomethasone, triamcinolone, budesonide, fluticasone, mometasone, ciclesonide, and dexamethasone. Examples of possible salts or derivatives include: sodium salts, sulfobenzoates, phosphates, isonicotinates, acetates, propionates, dihydrogen phosphates, palmitates, pivalates, or furoates. In some cases, the corticosteroids may also occur in the form of their hydrates.

In another embodiment, muscarinic receptor antagonists can be selected from for example, tiotropium salts, methantheline, ipratropium, propantheline, dicyclomine, scopolamine, telenzepine, benztropine and atropine.

Other muscarinic receptor antagonists can be selected from for example, a list of such compounds presented herein.

The one or more PDE-IV inhibitors and one or more muscarinic receptor antagonists (MRA) can be present in compositions described herein in a ratio from 1:10 to 10:1.

The one or more PDE-IV inhibitors and one or more β2-agonist can be present in compositions described herein in compositions described herein in a ratio from 1:10 to 10:1.

The one or more PDE-IV inhibitors and one or more p38 MAP Kinase inhibitors can be present in compositions described herein in a ratio from 1:10 to 10:1.

The one or more PDE-IV inhibitors and one or more corticosteroids can be present in compositions described herein in a ratio from 1:10 to 10:1.

In another aspect, provided herein are methods of treating autoimmune, inflammatory or allergic diseases or disorders, comprising administering one or more pharmaceutical compositions described herein. The autoimmune, inflammatory or allergic diseases or disorders can be selected from respiratory disorder, asthma, chronic bronchitis, chronic obstructive pulmonary disease, whooping cough, eosinophilic granuloma, psoriasis and other benign or malignant proliferative skin diseases, eczema, inflammatory bowel disease, endotoxic shock, anaphylactic shock, laminitis in horses, septic shock, ulcerative colitis, Crohn's disease, reperfusion injury of the myocardium and brain, inflammatory arthritis, perodontitis, chronic glomerulonephritis, atopic dermatitis, urticaria, adult respiratory distress syndrome, infant respiratory distress syndrome, transplant rejection, rhinitis, pruritus, diabetes insipidus, eye diseases, allergic rhinitis, allergic conjunctivitis, vernal conjunctivitis, arterial restenosis, ortherosclerosis, atherosclerosis, neurogenic inflammation, pain, cough, rheumatoid arthritis, osteoporosis, osteoarthritis, inflammation, ankylosing spondylitis, transplant rejection, graft versus host disease, hypersecretion of gastric acid, bacterial, fungal induced sepsis, viral induced sepsis, fungal induced septic shock, viral induced septic shock, inflammation-mediated chronic tissue degeneration, cytokine-mediated chronic tissue degeneration, osteoarthritis, cancer, cachexia, muscle wasting, depression memory impairment, tumor growth, cancerous invasion of normal tissues Hashimoto's thyroiditis (underactive thyroid), Graves' disease (overactive thyroid), Lupus and acquired immuno deficiency syndrome.

Detailed Description of the Invention

In accordance with an aspect, provided herein are compositions comprising one or more PDE-IV inhibitors and at least one other active ingredient such as muscarinic receptor antagonists (MRA), β2-agonists, p38 MAP Kinase inhibitors and corticosteroids and optionally one or more pharmaceutically acceptable excipients wherein the PDE-IV inhibitor is one or more compound having the structure of Formula Ia or Formula Ib, wherein:

a. Formula Ia is:

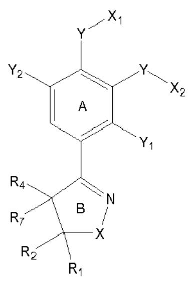

FORMULA Ia

and its pharmaceutically acceptable salts, pharmaceutically acceptable solvates, enantiomers, diastereomers or N-oxides, wherein

**When X is oxygen,**

**$R_1$** can be hydrogen, alkyl, heterocyclyl, $-(CH_1)_mC(=O)R_3$, or $(CH_2)_{1-4}OR'$, (wherein m is an integer 0-2, $R_3$ can be alkyl, cycloalkyl, heterocyclyl, or optionally substituted $R_p$ or $R_q$, wherein $R_p$ can be heterocyclyl or heteroaryl ring, wherein the rings are attached to $(CH_2)_mC(=O)$ through N, and $R_q$ can be heterocyclyl or heteroaryl ring wherein the rings are attached to $-(CH_2)_mC(=O)$ through C, and wherein R' can be can be alkyl, alkenyl, alkynyl, saturated or unsaturated cycloalkyl, aryl, heterocyclyl or heteroaryl);

**$R_2$** can be $(CH_1)_mC(=O)R_3$, $-(CH_2)_{1-4}OR'$, or $C(=O)NR_xR_y$ {where m, $R_3$ and R' are as defined above, and wherein $R_x$ and $R_y$ each independently can be hydrogen, alkyl, $C_3$-$C_6$ alkenyl, $C_3$-$C_6$ alkynyl, cycloalkyl, carboxy, $-S(O)_mR_5$ (wherein $R_5$ can be hydrogen, alkyl, alkenyl, alkynyl, aryl, cycloalkyl, alkaryl, heteroaryl, heteroarylalkyl, heterocyclyl or heterocyclylalkyl), aryl, alkaryl, heteroaryl, heterocyclyl, heteroarylalkyl, or heterocyclylalkyl}, or **$R_1$** and **$R_2$** together form an optionally substituted cycloalkyl or heterocyclyl ring wherein the optional substituent is oxo, alkyl, alkenyl, alkynyl, halogen, nitro, $-NH_2$, $-NHC(=O)OR_6$, $-C(=O)NR_xR_y$, cyano, hydroxy, alkoxy, or substituted amino (wherein $R_6$ can be alkyl, alkenyl, alkynyl, cycloalkyl, alkaryl, heteroarylalkyl or heterocyclylalkyl), with the proviso that if $R_1$ is $-(CH_2)_{1-4}OR'$, then $R_2$ is also $-(CH_2)_{1-4}OR'$, and with the proviso that if $R_1$ is $C(=O)NR_xR_y$, then $R_2$ is also $C(=O)NR_xR_y$;

**$R_4$** can be hydrogen; alkyl; $-OR_5$; halogen; $-NH_2$, substituted amino; cyano; carboxy; or $-C(=O)NR_xR_y$ (wherein $R_5$, $R_x$ and $R_y$ are as defined above); or **$R_2$** and **$R_4$** forms an optionally substituted 4-12 membered saturated or unsaturated monocyclic or bicyclic ring system fused to ring B having 0-4 heteroatom(s) selected from the group consisting of N, O and S, wherein the substituents can be one or more of alkyl, halogen, hydroxy, alkoxy, $-NH_2$ or substituted amino (wherein $R_3$ and $R_x$ and $R_y$ are as defined above), with the proviso that $R_2$ and $R_4$ together does not form $-CH_2-O-CH_2-O-CH_2-$;

**$R_7$** can be hydrogen, alkyl, alkenyl, alkynyl, $-OR_5$, halogen, cyano, $-NH_2$, or substituted amino;

**$X_1$ and $X_2$** each independently can be hydrogen, alkyl, alkaryl, cycloalkyl, alkaryl, alkenyl, cycloalkylalkyl, heterocyclyl, heteroaryl, heterocyclylalkyl, $-(CH_2)_gC(=O)NR_xR_y$, $-(CH_2)_{g1}C(=O)OR_3$ or heteroarylalkyl; wherein $g_1$ can be an integer from 1-3 (wherein $R_x$, $R_y$, g and $R_3$ are as defined above);

**Y** can each independently be an oxygen atom; a sulphur atom; or -NR (wherein R can be can be hydrogen, acyl, aryl, or alkyl);

**$Y_1$ and $Y_2$** each independently can be hydrogen; alkyl; -OR; -SR; or -NHR (wherein R is as defined above); wherein any of **$Y_1$** and **$X_2$** & **$X_1$** and **$Y_2$** together optionally form a ring fused with the ring A, the ring containing 3-5 carbon atoms within the ring and having 1-3 heteroatoms such as N, O and S, and **$X_1$**

and $X_2$ can together optionally form a ring fused with ring A, the ring containing 3-5 carbon atoms within the ring and having 2-3 heteroatoms such as N, O or S, and

**When X is $NR_7$, or S (wherein $R_7$, can be hydrogen, or $C_{1-6}$ alkyl)**

**$R_1$ and $R_2$** can each independently be alkyl, alkenyl, alkynyl, alkoxy, hydroxy, cyano, nitro, halogen, heteroaryl, heterocyclyl, heteroarylalkyl, heterocyclylalkyl, $NH_2$, substituted amino, carboxy, $-(CH_2)_mC(=O)R_3$, -C (=O)$NR_xR_y$, or $(CH_2)_{1-4}OR'$, {wherein m is an integer 0-2, $R_3$ can be alkyl, cycloalkyl, heterocyclyl, or optionally substituted Rp or $R_q$ (wherein $R_p$ can be heterocyclyl or heteroaryl ring, wherein the rings are attached to $(CH_2)_mC(=O)$ through N, and $R_q$ can be heterocyclyl or heteroaryl ring wherein the rings are attached to $-(CH_2)_mC(=O)$ through C), wherein R' can be can be alkyl, alkenyl, alkynyl, saturated or unsaturated cycloalkyl, aryl, heterocyclyl or heteroaryl, and wherein $R_x$ and $R_y$ each independently can be hydrogen, alkyl, $C_3$-$C_6$ alkenyl, $C_3$-$C_6$ alkynyl, cycloalkyl, carboxy, $-S(O)_mR_5$ (wherein $R_5$ can be hydrogen, alkyl, alkenyl, alkynyl, aryl, cycloalkyl, alkaryl, heteroaryl, heteroarylalkyl, heterocyclyl or heterocyclylalkyl), aryl, alkaryl, heteroaryl, heterocyclyl, heteroarylalkyl, or heterocyclylalkyl}, or **$R_1$** and **$R_2$** together can form an optionally substituted cycloalkyl or heterocyclyl ring wherein the optional substituent is oxo, alkyl, alkenyl, alkynyl, halogen, nitro, $-NH_2$, $-NHC(=O)OR_6$ (wherein $R_6$ can be alkyl, alkenyl, alkynyl, cycloalkyl, alkaryl, heteroarylalkyl or heterocyclylalkyl), - C(=O)$NR_xR_y$, cyano, hydroxy, alkoxy, or substituted amino;

**$R_4$** can be hydrogen; alkyl; $-OR_5$; halogen; $-NH_2$, substituted amino; cyano; carboxy; or -C(=O)$NR_xR_y$ (wherein $R_5$, $R_x$ and $R_y$ are as defined above); or **$R_2$** and **$R_4$** forms an optionally substituted 4-12 membered saturated or unsaturated monocyclic or bicyclic ring system fused to ring B having 0-4 heteroatom(s) selected from the group consisting of N, O and S, wherein the substituents can be one or more of alkyl, halogen, hydroxy, alkoxy or substituted amino (wherein $R_3$ and $R_x$ and $R_y$ are as defined above), with the proviso that $R_2$ and $R_4$ together does not form - $CH_2$-O-$CH_2$-O-$CH_2$-;

**$R_7$** can be hydrogen, alkyl, alkenyl, alkynyl, $-OR_5$, halogen, cyano,$-NH_2$, or substituted amino;

**$X_1$ and $X_2$** each independently can be alkyl, cycloalkyl, alkaryl, heteroaryl, heterocyclyl, heteroarylalkyl, or heterocyclylalkyl;

**Y** can each independently be an oxygen atom; a sulphur atom; or -NR (wherein R can be can be hydrogen, acyl, aryl, or alkyl);

**$Y_1$ and $Y_2$** each independently can be hydrogen, alkyl, -OR, -SR, or -NHR (wherein R is as defined above); wherein any of **$Y_1$** and **$X_2$** & **$X_1$** and **$Y_2$** together optionally form a ring fused with the ring A, the ring containing 3-5 carbon atoms within the ring and having 1-3 heteroatoms such as N, O and S; **$X_1$** and **$X_2$** can together optionally forms a cyclic ring fused with the ring A, the ring containing 3-5 carbon atoms within the ring and having 2-3 heteroatoms such as N, O or S.

b. Formula Ib is:

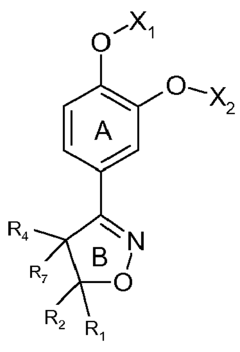

Formula Ib

and its pharmaceutically acceptable salts, pharmaceutically acceptable solvates, enantiomers, diastereomers or N-oxides, wherein
**$R_1$** and **$R_2$** together forms an optionally substituted cycloalkyl or heterocyclyl ring

wherein one or more optional substituent are oxo, alkyl, alkaryl, alkenyl, alkynes, heterocyclylalkyl, cycloalkylalkyl,

$-SO_2NR_xR_y$, halogen, $-NH_2$, $-(CH_2)_gC(=O)NR_xR_y$, - $NHC(=O)OR_6$, $-NHC(=O)NR_xR_y$, $-C(=O)OR_3$, $-NHC(=O)R_x$, $-SO_2R_3$, cyano, hydroxy, alkoxy, substituted amino, or $-C(=O)R_3$ (wherein $R_xR_y$ g, $R_6$ and $R_3$ are as defined above);
**$R_4$** can be hydrogen; alkyl, hydroxyl, halogen, or carboxy;
**$R_7$** can be hydrogen, or alkyl;
**$R_1$** can be independently hydrogen or alkyl and **$R_2$** and **$R_4$** forms an optionally substituted 4-12 membered saturated or unsaturated monocyclic or bicyclic ring system fused to ring B having 0-4 heteroatom(s) selected from the group consisting of N, O and S, wherein the substituents is one or more of oxo, alkyl, $-C(=O)OR_3$, $-SO_2R_3$, halogen, hydroxy, alkoxy, $-NH_2$ or substituted amino (wherein $R_3$ is as defined below), with the proviso that $R_2$ and $R_4$ together does not form $-CH_2-O-CH_2-O-CH_2-$;
**$X_1$** and **$X_2$** can be hydrogen, alkyl, cycloalkyl, alkaryl, alkenyl, cycloalkylalkyl, heteroaryl, heterocyclyl, heteroarylalkyl, heterocyclylalkyl, $-(CH_2)_gC(=O)NR_xR_y$ or - $(CH_2)_{g1}C(=O)OR_3$ (wherein g can be an integer from 0-3 and $g_1$ can be an integer from 1-3, and $R_x$, $R_y$ and $R_3$ are as defined below);
**$X_1$** and **$X_2$** together can optionally form a cyclic ring fused with the ring A shown in Formula I, the ring containing 3-5 carbon atoms within the ring and having 2-3 heteroatoms N, O or S;
wherein **$R_3$** can be alkyl, cycloalkyl or heterocyclyl;
wherein the halogen can be F, Cl, Br, or I; **$R_x$** and **$R_y$** each independently can be hydrogen, alkyl, $C_3$-$C_6$ alkenyl, $C_3$-$C_6$ alkynyl, carboxy, cycloalkyl, $-S(O)_mR_5$, aryl, alkaryl, heteroaryl, heterocyclyl, heteroarylalkyl, and heterocyclylalkyl; **m** can be an integer between 0-2; **$R_6$** can be alkyl, alkenyl, alkynyl, cycloalkyl, alkaryl, heteroarylalkyl or heterocyclylalkyl; wherein **$R_5$** can be hydrogen, alkyl, alkenyl, alkynyl, aryl, cycloalkyl, alkaryl, heteroaryl, heteroarylalkyl, heterocyclyl or heterocyclylalkyl.

In another aspect, provided are pharmaceutical compositions comprising one or more phosphodiesterase inhibitors of type IV ("PDE-IV"), and atleast one other active ingredients selected from muscarinic receptor antagonists (MRA), β2-agonists, p38 MAP Kinase inhibitors, and corticosteroids and one or more pharmaceutically acceptable excipients, wherein the PDE-IV inhibitor is one or more compounds having the structure of Formula Ia and Formula Ib, as described herein.

The pharmaceutical compositions of each of the above aspects can include, for example, one or more of the following illustrative compounds of Formula Ia or Formula Ib:

3-[3-(cyclopentyloxy)-4-methoxyphenyl]-1,7-dioxa-2-azaspiro[4.4]non-2-en-6-ol (Compound No. 1),

3-[3-(Cyclopentyloxy)-4-methoxyphenyl]-N-(4-fluorophenyl)-1-oxa-2,7-diazaspiro[4.4]non-2-ene-7-carboxamide (Compound No. 2),

3-[3-(Cyclopentyloxy)-4-methoxyphenyl]-7-(tetrahydrofuran-3-ylcarbonyl)-1-oxa-2,7-diazaspiro[4.4]non-2-ene (Compound No. 3),

3-[3-(cyclopentyloxy)-4-methoxyphenyl]-N,N-dimethyl-1-oxa-2,7-diazaspiro[4.4]non-2-ene-7-sulfonamide (Compound No. 4),

*N*-butyl-3-[3-(cyclopentyloxy)-4-methoxyphenyl]-1-oxa-2,7-diazaspiro[4.4]non-2-ene-7-carboxamide (Compound No. 5),

2-{3-[3-(Cyclopentyloxy)-4-methoxyphenyl]-1-oxa-2,7-diazaspiro[4.4]non-2-en-7-yl}acetamide (Compound No. 6),

Hydrochloride salt of 3-[3-(cyclopentyloxy)-4-methoxyphenyl]-8-prolyl-1-oxa-2,8-diazaspiro[4.5]dec-2-ene (Compound No. 7),

3-[3-(Cyclopentyloxy)-4-methoxyphenyl]-8-(2-morpholin-4-yl-ethyl)-1-oxa-2,8-diazaspiro[4.5]dec-2-ene (Compound No. 8),

*N*-butyl-3-[3-(cyclopentyloxy)-4-methoxyphenyl]-1-oxa-2,8-diazaspiro[4.5]dec-2-ene-8-carboxamide (Compound No. 9),

3-[3-(cyclopentyloxy)-4-methoxyphenyl]-8-(methylsulfonyl)-1-oxa-2,8-diazaspiro[4.5]dec-2-ene (Compound No. 10),

3-[3-(cyclopentyloxy)-4-methoxyphenyl]-1-oxa-2-azaspiro[4.4]non-2-ene (Compound No. 11),

3-[3,4-bis(2-morpholin-4-ylethoxy)phenyl]-1,7-dioxa-2-azaspiro[4.4]non-2-ene (Compound No. 12),

3-(3,4-diisopropoxyphenyl)-1,7-dioxa-2-azaspiro[4.4]non-2-ene (Compound No. 13),

3-[3-methoxy-4-(2-morpholin-4-ylethoxy)phenyl]-1,7-dioxa-2-azaspiro[4.4]non-2-ene (Compound No. 14),

3-[3-(cyclopentyloxy)-4-methoxyphenyl]-1,7-dioxa-2-azaspiro[4.4]non-2-en-8-one (Compound No. 15),

3-[3-(cyclopentyloxy)-4-methoxyphenyl]-1,7-dioxa-2-azaspiro[4.4]non-2-en-8-ol (Compound No. 16),

3-[3-(Cyclopentyloxy)-4-methoxyphenyl]-7-isopropyl-1-oxa-2, 7-diazaspiro [4.4] non-2-ene (Compound No. 17),

3-[3-(cyclopentyloxy)-4-methoxyphenyl]-7-(cyclopropylcarbonyl)-1-oxa-2,7-diazaspiro[4.4]non-2-ene (Compound No. 18),

N-benzyl-3-[3-(cyclopentyloxy)-4-methoxyphenyl]-1-oxa-2,7-diazaspiro[4.4]non-2-ene-7-carboxamide (Compound No. 19),

7-acetyl-3-[3-(cyclopentyloxy)-4-methoxyphenyl]-1-oxa-2,7-diazaspiro[4.4]non-2-ene (Compound No. 20),

*Tert*-butyl 3-[3-(cyclopentyloxy)-4-methoxyphenyl]-1-oxa-2,7-diazaspiro[4.5]dec-2-ene-7-carboxylate (Compound No. 21),

*N*-butyl-*N'*-{3-[3-(cyclopentyloxy)-4-methoxyphenyl]-1-oxa-2-azaspiro[4.5]dec-2-en-8-yl}urea (Compound No. 22),

*N*-{3-[3-(cyclopentyloxy)-4-methoxyphenyl]-1-oxa-2-azaspiro[4.5]dec-2-en-8-yl}-*N'*-(2-methoxyphenyl)urea (Compound No. 23),

3-[3-(cyclopentyloxy)-4-methoxyphenyl]-1-oxa-2-azaspiro[4.5]dec-2-en-8-ol (Compound No. 24),

Hydrochloride salt of 3-[3-(cyclopentyloxy)-4-methoxyphenyl]-1-oxa-2,7-diazaspiro[4.5]dec-2-ene (Compound No. 25),

3-[3-(cyclopentyloxy)-4-methoxyphenyl]-1-oxa-2-azaspiro[4.5]dec-2-en-8-one (Compound No. 26),

3-[3,4-bis(cyclopentyloxy)phenyl]-1,7-dioxa-2-azaspiro[4.4]non-2-ene (Compound No. 27),

3-[3,4-Bis(cyclopropylmethoxy)phenyl]-1,7-dioxa-2-azaspiro[4.4]non-2-ene (Compound No. 28),

3-[3-(cyclopentyloxy)-4-methoxyphenyl]-1,7-dioxa-2-azaspiro[4.4]non-2-en-4-ol (Compound No. 29),

(R)-3-[3-(cyclopentyloxy)-4-methoxyphenyl]-1,7-dioxa-2-azaspiro[4.4]non-2-ene (Compound No. 30),

3-[3-(Cyclopentyloxy)-4-methoxyphenyl]-8-(cyclopropylmethyl)-1-oxa-2,8-diazaspiro[4.5]dec-2-ene (Compound No. 31),

*N*-Benzyl-3-[3-(cyclopentyloxy)-4-methoxyphenyl]-1-oxa-2,8-diazaspiro[4.5]dec-2-ene-8-carboxamide (Compound No. 32),

3-[3,4-Bis(benzyloxy)phenyl]-1,7-dioxa-2-azaspiro[4.4]non-2-ene (Compound No. 33),

4-(1,7-Dioxa-2-azaspiro[4.4]non-2-en-3-yl)benzene-1,2-diol (Compound No. 34),

7-Amino-3-[3-(cyclopentyloxy)-4-methoxyphenyl]-1-oxa-2,7-diazaspiro[4.4]non-2-en-6-one (Compound No. 35),

Ethyl 8-benzyl-3-[3-(cyclopentyloxy)-4-methoxyphenyl]-1-oxa-2,8-diazaspiro[4.5]dec-2-ene-4-carboxylate (Compound No. 36),

3-[3-(Cyclopentyloxy)-4-methoxyphenyl]-1-oxa-2-azaspiro[4.5]dec-2-ene-4-carboxylic acid (Compound No. 37),

8-Benzyl-3-[3-(cyclopentyloxy)-4-methoxyphenyl]-1-oxa-2,8-diazaspiro[4.5]dec-2-ene (Compound No. 38),

Ethyl 3-[3-(cyclopentyloxy)-4-methoxyphenyl]-1-oxa-2-azaspiro[4.5]dec-2-ene-4-carboxylate (Compound No. 39),

3-[3-(Difluoromethoxy)-4-methoxyphenyl]-1,7-dioxa-2-azaspiro[4.4]non-2-ene (Compound No. 40),

2-(Difluoromethoxy)-5-(1,7-dioxa-2-azaspiro[4.4]non-2-en-3-yl)phenol (Compound No. 41),

3-[3-(Cyclopentyloxy)-4-methoxyphenyl]-1-oxa-2,7-diazaspiro[4.4]non-2-en-6-one (Compound No. 42),

3-[3-(Cyclopentyloxy)-4-methoxyphenyl]-3a,6a-dimethyl-3a*H*-cyclopenta[*d*]isoxazole-4,6(5*H*,6a*H*)-dione (Compound No. 43),

3-[3-(Cyclopentyloxy)-4-methoxyphenyl]-3a,4,6,6a-tetrahydrofuro[3,4-*d*]isoxazole (Compound No. 44),

3-[3-(Cyclopentyloxy)-4-methoxyphenyl]-6,6a-dihydrofuro[3,4-*d*]isoxazol-4(3a*H*)-one (Compound No. 45),

*Tert*-butyl [({3-[3-(cyclopentyloxy)-4-methoxyphenyl]-1-oxa-2-azaspiro[4.5]dec-2-en-8-yl}amino)carbonyl]carbamate (Compound No. 46),

*N*-{3-[3-(Cyclopentyloxy)-4-methoxyphenyl]-1-oxa-2-azaspiro[4.5]dec-2-en-8-yl}cyclopentanecarboxamide (Compound No. 47),

8-Acetyl-3-[3-(cyclopentyloxy)-4-methoxyphenyl]-1-oxa-2,8-diazaspiro[4.5]dec-2-ene (Compound No. 48),

8-(Cyclopentylcarbonyl)-3-[3-(cyclopentyloxy)-4-methoxyphenyl]-1-oxa-2,8-diazaspiro[4.5]dec-2-ene (Compound No. 49),

3-[3-(Cyclopentyloxy)-4-methoxyphenyl]-8-(2-piperidin-1-ylethyl)-1-oxa-2,8-diazaspiro[4.5]dec-2-ene (Compound No. 50),

3-(2,3-Dihydro-1,4-benzodioxin-6-yl)-1,7-dioxa-2-azaspiro[4.4]non-2-ene (Compound No. 51),

3-[3-(Cyclopentyloxy)-4-methoxyphenyl]-1,8-dioxa-2-azaspiro[4.5]dec-2-ene (Compound No. 52),

3-[3-(Cyclopentyloxy)-4-methoxyphenyl]-3a*H*cyclopenta[*d*]isoxazole-4,6(5*H*,6a*H*)-dione (Compound No. 53),

3-[3-(Cyclopentyloxy)-4-methoxyphenyl]-8-ethyl-1-oxa-2,8-diazaspiro[4.5]dec-2-ene (Compound No. 54),

3-[3-(Cyclopentyloxy)-4-methoxyphenyl]-8-vinyl-1-oxa-2-azaspiro[4.5]dec-2-en-8-ol (Compound No. 55),

3-[3-(Cyclopentyloxy)-4-methoxyphenyl]-3a,4,5,6,7,7a-hexahydro-1,2-benzisoxazole (Compound No. 56),

3-[3-(Cyclopentyloxy)-4-methoxyphenyl]-4,5,6,6a-tetrahydro-3a*H*-cyclopenta[*d*]isoxazole (Compound No. 57),

N-{3-[3-(Cyclopentyloxy)-4-methoxyphenyl]-1-oxa-2-azaspiro[4.5]dec-2-en-8-yl}methanesulfonamide(Compound No. 58),

3-[3-(Cyclopentyloxy)-4-methoxyphenyl]-8-methyl-1-oxa-2-azaspiro[4.5]dec-2-en-8-ol (Compound No. 59),

3-[3-(Allyloxy)-4-methoxyphenyl]-1,7-dioxa-2-azaspiro[4.4]non-2-ene (Compound No. 60),

3-[3-(2-Chloroethoxy)-4-methoxyphenyl]-1,7-dioxa-2-azaspiro[4.4]non-2-ene (Compound No. 61),

2-(Cyclopentyloxy)-4-(1,7-dioxa-2-azaspiro[4.4]non-2-en-3-yl)phenol (Compound No. 62),

3-(4-Butoxy-3-isobutoxyphenyl)-1,7-dioxa-2-azaspiro[4.4]non-2-ene (Compound No. 63),

3-(3-Isobutoxy-4-propoxyphenyl)-1,7-dioxa-2-azaspiro[4.4]non-2-ene (Compound No. 64),

3-[3-Butoxy-4-(cyclopropylmethoxy)phenyl]-1,7-dioxa-2-azaspiro[4.4]non-2-ene (Compound No. 65),

3-(3-Butoxy-4-ethoxyphenyl)-1,7-dioxa-2-azaspiro[4.4]non-2-ene (Compound No. 66),

3-[3-Butoxy-4-(cyclohexyloxy)phenyl]-1,7-dioxa-2-azaspiro[4.4]non-2-ene (Compound No. 67),

3-[3-(Cyclohexylmethoxy)-4-ethoxyphenyl]-1,7-dioxa-2-azaspiro[4.4]non-2-ene (Compound No. 68),

3-[3-(Cyclohexylmethoxy)-4-isopropoxyphenyl]-1,7-dioxa-2-azaspiro[4.4]non-2-ene (Compound No. 69),

3-[4-Butoxy-3-(cyclohexylmethoxy)phenyl]-1,7-dioxa-2-azaspiro[4.4]non-2-ene (Compound No. 70),

3-(4-Isobutoxy-3-isopropoxyphenyl)-1,7-dioxa-2-azaspiro[4.4]non-2-ene (Compound No. 71),

3-(4-Butoxy-3-isopropoxyphenyl)-1,7-dioxa-2-azaspiro[4.4]non-2-ene (Compound No. 72),

3-[4-(Cyclohexylmethoxy)-3-isopropoxyphenyl]-1,7-dioxa-2-azaspiro[4.4]non-2-ene (Compound No. 73),

3-[3-Isopropoxy-4-(2-morpholin-4-ylethoxy)phenyl]-1,7-dioxa-2-azaspiro[4.4]non-2-ene (Compound No. 74),

3-[3-(Cyclopropylmethoxy)-4-isopropoxyphenyl]-1,7-dioxa-2-azaspiro[4.4]non-2-ene (Compound No. 75),

3-[3-(Cyclopropylmethoxy)-4-(2-morpholin-4-ylethoxy)phenyl]-1,7-dioxa-2-azaspiro[4.4]non-2-ene (Compound No. 76),

3-[4-Butoxy-3-(cyclopropylmethoxy)phenyl]-1,7-dioxa-2-azaspiro[4.4]non-2-ene (Compound No. 77),

3-[3-(Cyclopropylmethoxy)-4-isopropoxyphenyl]-1,7-dioxa-2-azaspiro[4.4]non-2-ene (Compound No. 78),

3-(3-Isobutoxy-4-isopropoxyphenyl)-1,7-dioxa-2-azaspiro[4.4]non-2-ene (Compound No. 79),

3-[4-(Cyclopropylmethoxy)-3-isobutoxyphenyl]-1,7-dioxa-2-azaspiro[4.4]non-2-ene (Compound No. 80),

3-[4-(cyclohexyloxy)-3-(cyclopentyloxy)phenyl]-1,7-dioxa-2-azaspiro[4.4]non-2-ene (Compound No. 81)

3-[4-(Cyclohexylmethoxy)-3-(cyclopentyloxy)phenyl]-1,7-dioxa-2-azaspiro[4.4]non-2-ene (Compound No. 82),

3-[4-(Cyclopropylmethoxy)-3-(cyclopentyloxy)phenyl]-1,7-dioxa-2-azaspiro[4.4]non-2-ene (Compound No. 83),

3-[3-(Cyclopentyloxy)-4-isobutoxyphenyl]-1,7-dioxa-2-azaspiro[4.4]non-2-ene (Compound No. 84),

3-[3-(Cyclopentyloxy)-4-ethoxyphenyl]-1,7-dioxa-2-azaspiro[4.4]non-2-ene (Compound No. 85),

3-[3-(Cyclopropylmethoxy)-4-ethoxyphenyl]-1,7-dioxa-2-azaspiro[4.4]non-2-ene (Compound No. 86),

3-[4-(Cyclopentyloxy)-3-isobutoxyphenyl]-1,7-dioxa-2-azaspiro[4.4]non-2-ene (Compound No. 87),

3-[3-Isopropoxy-4-(2-morpholin-4-ylethoxy)phenyl]-1,7-dioxa-2-azaspiro[4.4]non-2-ene (Compound No. 88),

3-(4-Ethoxy-3-isobutoxyphenyl)-1,7-dioxa-2-azaspiro[4.4]non-2-ene (Compound No. 89),

3-[3-(Cyclopentyloxy)-4-propoxyphenyl]-1,7-dioxa-2-azaspiro[4.4]non-2-ene (Compound No. 90),

3-[4-Butoxy-3-(cyclopentyloxy)phenyl]-1,7-dioxa-2-azaspiro[4.4]non-2-ene (Compound No. 91),

3-[3-(Cyclopentyloxy)-4-isopropoxyphenyl]-1,7-dioxa-2-azaspiro[4.4]non-2-ene (Compound No. 92),

3-[3-(Cyclopentyloxy)-4-(cycloheptyloxy)phenyl]-1,7-dioxa-2-azaspiro[4.4]non-2-ene (Compound No. 93),

3-[3-(Cyclopentyloxy)-4-(2-morpholin-4-ylethoxy)phenyl]-1,7-dioxa-2-azaspiro[4.4]non-2-ene (Compound No. 94),

3-[4-(Cyclohexylmethoxy)-3-isobutoxyphenyl]-1,7-dioxa-2-azaspiro[4.4]non-2-ene (Compound No. 95),

3-[4-(Cyclohexylmethoxy)-3-(cyclopropylmethoxy)phenyl]-1,7-dioxa-2-azaspiro[4.4]non-2-ene (Compound No. 96),

3-[3-(Cyclopropylmethoxy)-4-propoxyphenyl]-1,7-dioxa-2-azaspiro[4.4]non-2-ene (Compound No. 97),

3-[4-(Cyclopentyloxy)-3-(cyclopropylmethoxy)phenyl]-1,7-dioxa-2-azaspiro[4.4]non-2-ene (Compound No. 98),

3-[4-(Cyclopropylmethoxy)-3-isopropoxyphenyl]-1,7-dioxa-2-azaspiro[4.4]non-2-ene (Compound No. 99),

3-[4-(Cyclopentyloxy)-3-isopropoxyphenyl]-1,7-dioxa-2-azaspiro[4.4]non-2-ene (Compound No. 100),

3-(3-Isopropoxy-4-propoxyphenyl)-1,7-dioxa-2-azaspiro[4.4]non-2-ene (Compound No. 101),

3-(4-Ethoxy-3-isopropoxyphenyl)-1,7-dioxa-2-azaspiro[4.4]non-2-ene (Compound No. 102),

3-[3-Butoxy-4-(2-morpholin-4-ylethoxy)phenyl]-1,7-dioxa-2-azaspiro[4.4]non-2-ene (Compound No. 103),

3-[3-Butoxy-4-(cyclopentyloxy)phenyl]-1,7-dioxa-2-azaspiro[4.4]non-2-ene (Compound No. 104),

3-(3-Butoxy-4-propoxyphenyl)-1,7-dioxa-2-azaspiro[4.4]non-2-ene (Compound No. 105),

3-(3-Butoxy-4-isopropoxyphenyl)-1,7-dioxa-2-azaspiro[4.4]non-2-ene (Compound No. 106),

3-[3-(Cyclohexylmethoxy)-4-propoxyphenyl]-1,7-dioxa-2-azaspiro[4.4]non-2-ene (Compound No. 107),

3-[3-(Cyclohexylmethoxy)-4-isobutoxyphenyl]-1,7-dioxa-2-azaspiro[4.4]non-2-ene (Compound No. 108),

3-[3-(Cyclohexylmethoxy)-4-(cyclopentyloxy)phenyl]-1,7-dioxa-2-azaspiro[4.4]non-2-ene (Compound No. 109),

3-[3-(Cyclohexylmethoxy)-4-(cyclopropylmethoxy)phenyl]-1,7-dioxa-2-azaspiro[4.4]non-2-ene (Compound No. 110),

3-[4-(Cyclohexylmethoxy)-3-propoxyphenyl]-1,7-dioxa-2-azaspiro[4.4]non-2-ene (Compound No. 111),

3-[4-(Cyclopropylmethoxy)-3-propoxyphenyl]-1,7-dioxa-2-azaspiro[4.4]non-2-ene (Compound No. 112),

3-[4-(Cyclopentyloxy)-3-propoxyphenyl]-1,7-dioxa-2-azaspiro[4.4]non-2-ene (Compound No. 113),

3-[4-(3-Isobutoxy)-3-propoxyphenyl]-1,7-dioxa-2-azaspiro[4.4]non-2-ene (Compound No. 114),

3-[3-(Cycloheptyloxy)-4-(cyclopropylmethoxy)phenyl]-1,7-dioxa-2-azaspiro[4.4]non-2-ene (Compound No. 115),

3-[3-(Cycloheptyloxy)-4-propoxyphenyl]-1,7-dioxa-2-azaspiro[4.4]non-2-ene (Compound No. 116),

3-[4-Butoxy-3-(cycloheptyloxy)phenyl]-1,7-dioxa-2-azaspiro[4.4]non-2-ene (Compound No. 117),

3-[3-(Cycloheptyloxy)-4-isopropoxyphenyl]-1,7-dioxa-2-azaspiro[4.4]non-2-ene (Compound No. 118),

3-[3-(Cycloheptyloxy)-4-(cyclopentyloxy)phenyl]-1,7-dioxa-2-azaspiro[4.4]non-2-ene (Compound No. 119),

3-(3-Ethoxy-4-propoxyphenyl)-1,7-dioxa-2-azaspiro[4.4]non-2-ene (Compound No. 120),

3-[4-(Cycloheptyloxy)-3-ethoxyphenyl]-1,7-dioxa-2-azaspiro[4.4]non-2-ene (Compound No. 121),

3-[4-(Cyclopropylmethoxy)-3-ethoxyphenyl]-1,7-dioxa-2-azaspiro[4.4]non-2-ene (Compound No. 122),

3-[4-(Cyclohexylmethoxy)-3-ethoxyphenyl]-1,7-dioxa-2-azaspiro[4.4]non-2-ene (Compound No. 123),

(S)-3-[3-(cyclopentyloxy)-4-methoxyphenyl]-1,7-dioxa-2-azaspiro[4.4]non-2-ene (Compound No. 124),

3-(3-Butoxy-4-isobutoxyphenyl)-1,7-dioxa-2-azaspiro[4.4]non-2-ene (Compound No. 125),

3-(3-Ethoxy-4-isopropoxyphenyl)-1,7-dioxa-2-azaspiro[4.4]non-2-ene (Compound No. 126),

3-[4-(Cyclopentyloxy)-3-ethoxyphenyl]-1,7-dioxa-2-azaspiro[4.4]non-2-ene (Compound No. 127),

3-(4-Butoxy-3-ethoxyphenyl)-1,7-dioxa-2-azaspiro[4.4]non-2-ene (Compound No. 128),

3-(3-Ethoxy-4-isobutoxyphenyl)-1,7-dioxa-2-azaspiro[4.4]non-2-ene (Compound No. 129),

3-[3-(Cycloheptyloxy)-4-isobutoxyphenyl]-1,7-dioxa-2-azaspiro[4.4]non-2-ene (Compound No. 130),

3-[3-(Cycloheptyloxy)-4-(cyclopentyloxy)phenyl]-1,7-dioxa-2-azaspiro[4.4]non-2-ene (Compound No. 131),

3-[3-(Cycloheptyloxy)-4-ethoxyphenyl]-1,7-dioxa-2-azaspiro[4.4]non-2-ene (Compound No. 132),

3-(4-Butoxy-3-propoxyphenyl)-1,7-dioxa-2-azaspiro[4.4]non-2-ene (Compound No. 133),

3-(4-Ethoxy-3-propoxyphenyl)-1,7-dioxa-2-azaspiro[4.4]non-2-ene (Compound No. 134),

3-[4-(Morpholin-4-ylethoxy)-3-propoxyphenyl]-1,7-dioxa-2-azaspiro[4.4]non-2-ene (Compound No. 135),

3-(4-Isopropoxy-3-propoxyphenyl)-1,7-dioxa-2-azaspiro[4.4]non-2-ene (Compound No. 136),

2-[5-(1,7-Dioxa-2-azaspiro[4.4]non-2-en-3-yl)-2-methoxyphenoxy]cyclopentanol (Compound No. 137),

*N*-{3-[3-(Cyclopentyloxy)-4-methoxyphenyl]-1-oxa-2-azaspiro[4.5]dec-2-en-8-yl}-2-fluorobenzamide (Compound No. 138),

*N*-{3-[3-(cyclopentyloxy)-4-methoxyphenyl]-1-oxa-2-azaspiro[4.5]dec-2-en-8-yl}benzamide (Compound No. 139),

3-[3-(Cyclopentyloxy)-4-methoxyphenyl]-4,5,6,6a-tetrahydro-3a*H*-pyrrolo[3,4-*d*]isoxazole (Compound No. 140),

7-(Cyclopentylcarbonyl)-3-[3-(cyclopentyloxy)-4-methoxyphenyl]-1-oxa-2,7-diazaspiro[4.5]dec-2-ene (Compound No. 141),

*Tert*-butyl 3-[3-(cyclopentyloxy)-4-methoxyphenyl]-3a,4,6,6a-tetrahydro-5*H*-pyrrolo[3,4-d]isoxazole-5-carboxylate (Compound No. 142),

3-[3-(Cyclopentyloxy)-4-methoxyphenyl]-1-oxa-2,8-diazaspiro[4.5]dec-2-ene-8-carboxamide (Compound No. 143),

*N*-Butyl-3-[3-(cyclopentyloxy)-4-methoxyphenyl]-1-oxa-2,7-diazaspiro[4.5]dec-2-ene-7-carboxamide (Compound No. 144),

3-[3-(Cyclopentyloxy)-4-methoxyphenyl]-7-(methylsulfonyl)-1-oxa-2,7-diazaspiro[4.5]dec-2-ene (Compound No. 145),

3-[4-Methoxy-3-(pyridin-3-ylmethoxy)phenyl]-1,7-dioxa-2-azaspiro[4.4]non-2-ene (Compound No. 146),

5-Acetyl-3-[3-(cyclopentyloxy)-4-methoxyphenyl]-4,5,6,6a-tetrahydro-3a*H*-pyrrolo[3,4-*d*]isoxazole (Compound No.

147),

3-[3-(Cyclopentyloxy)-4-methoxyphenyl]-5-(methylsulfonyl)-4,5,6,6a-tetrahydro-3a*H*-pyrrolo[3,4-d]isoxazole (Compound No. 148),

4-Bromo-3-[3-(cyclopentyloxy)-4-methoxyphenyl]-1,7-dioxa-2-azaspiro[4.4]non-2-ene (Compound No. 149),

3-[3-(Cyclopentyloxy)-4-methoxyphenyl]-3a,5,6,7a-tetrahydro-1,2-benzisoxazol-7(4*H*)-one (Compound No. 150),

3-[4-(Difluoromethoxy)-3-(2,3-dihydro-17-inden-2-yloxy)phenyl]-1,7-dioxa-2-azaspiro[4.4]non-2-ene (Compound No. 151),

3-[4-(Cyclopentyloxy)-3-(2,3-dihydro-1*H*-inden-2-yloxy)phenyl]-1,7-dioxa-2-azaspiro[4.4]non-2-ene (Compound No. 152),

3-[4-Butoxy-3-(2,3-dihydro-1*H*-inden-2-yloxy)phenyl]-1,7-dioxa-2-azaspiro[4.4]non-2-ene (Compound No. 153),

3-(3-{[3-(Benzyloxy)cyclopentyl]oxy}-4-methoxyphenyl)-1,7-dioxa-2-azaspiro[4.4]non-2-ene (Compound No. 154),

7-Acetyl-3-[3-(cyclopentyloxy)-4-methoxyphenyl]-1-oxa-2,7-diazaspiro[4.5]dec-2-ene (Compound No. 155),

3-[4-Methoxy-3-(pyridin-2-ylmethoxy)phenyl]-1,7-dioxa-2-azaspiro[4.4]non-2-ene (Compound No. 156),

3-[3-(2,3-Dihydro-1*H*-inden-2-yloxy)-4-ethoxyphenyl]-1,7-dioxa-2-azaspiro[4.4]non-2-ene (Compound No. 157),

3-[3-(2,3-Dihydro-1*H*-inden-2-yloxy)-4-propoxyphenyl]-1,7-dioxa-2-azaspiro[4.4]non-2-ene (Compound No. 158),

3-[4-(Cyclopropylmethoxy)-3-(2,3-dihydro-1*H*-inden-2-yloxy)phenyl]-1,7-dioxa-2-azaspiro[4.4]non-2-ene (Compound No. 159),

3-[3-(2,3-Dihydro-1*H*-inden-2-yloxy)-4-isopropoxyphenyl]-1,7-dioxa-2-azaspiro[4.4]non-2-ene (Compound No. 160),

2-(2,3-Dihydro-1*H*-inden-2-yloxy)-4-(1,7-dioxa-2-azaspiro[4.4]non-2-en-3-yl)phenol (Compound No. 161),

*N*-cyclopropyl-2-[5-(1,7-dioxa-2-azaspiro[4.4]non-2-en-3-yl)-2-methoxyphenoxy]acetamide (Compound No. 162),

Hydrochloride salt of 3-[4-methoxy-3-(piperidin-3-yloxy)phenyl]-1,7-dioxa-2-azaspiro[4.4]non-2-ene (Compound No. 163),

2-[5-(1,7-Dioxa-2-azaspiro[4.4]non-2-en-3-yl)-2-methoxyphenoxy]acetamide (Compound No. 164),

Ethyl [5-(1,7-dioxa-2-azaspiro[4.4]non-2-en-3-yl)-2-methoxyphenoxy]acetate (Compound No. 165),

[5-(1,7-Dioxa-2-azaspiro[4.4]non-2-en-3-yl)-2-methoxyphenoxy]acetonitrile (Compound No. 166),

3-{3-[(2,6-Dichloropyridin-4-yl)methoxy]-4-methoxyphenyl}-1,7-dioxa-2-azaspiro[4.4]non-2-ene (Compound No. 167),

[3-(3-Cyclopentyloxy-4-methoxy phenyl)-5-(4-carboxylic acid tert butylester-piperazin-1-yl-carbonyl)-4,5-dihydroisoxazol-5-yl)-({4-carboxylic-acid- tert butyl ester piperazine-1-yl) ethanone (Compound No. 168),

1-{1-[5-(4-Acetyl-4-phenyl-piperidine-1-carbonyl)-3-(3-cyclopentyloxy-4-methoxyphenyl)-4,5-dihydro-isoxazole-5-yl]-4-acetyl-4-phenyl-piperidin-4-yl]-ethanone (Compound No. 169),

[3-(3-Cyclopentyloxy-4-methoxy-phenyl)-5-(pyrrolidine-1-carbonyl)-4,5-dihydro-isoxazol-5-yl]-pyrrolidin-1-yl-ethanone (Compound No. 170),

[3-(3-Cyclopentyloxy-4-methoxy-phenyl)-5-(piperidine-1-carbonyl)-4,5-dihydro-isoxazol-5-yl]-piperidin-1-yl-ethanone (Compound No. 171),

3-(3-Cyclopentyloxy-4-methoxy phenyl)-5-(pyrrolidin-2-carboxylic acid methyl ester-1-carbonyl)-4,5-dihydro-isoxazol-5-yl)-[{pyrrolidine-2-carboxylic acid methyl ester-5-yl] ethanone ( Compound No. 172),

[5-[4-(4-Chlorophenyl)-4-hydroxy-piperidine-1-carbonyl]-3-(3-cyclopentyloxy-4-methoxy-phenyl)-4,5-dihydro-isoxazol-5-yl]-[4-(4-chlorophenyl)-4-hydroxy-piperidin-1-yl]-ethanone ( Compound No. 173),

[3-(3-Cyclopentyloxy-4-methoxy-phenyl)-5-(hydroxymethyl-piperidine-1-carbonyl)-4,5-dihydro-isoxazol-5-yl]-(4-hydroxymethyl-piperidin-1-yl)-ethanone (Compound No. 174),

[5-(5-Benzyl-2,5-diazabicyclo[2.2.1]heptane-2-(carbonyl)-3-(3-cyclopentyloxy-4-methoxy-phenyl]-4,5-dihydro-isoxozol-5-yl]-5-benzyl-2,5-diazabicylo-[2.2.1]hept-2-yl-ethanone ( Compound No. 175),

[3-(3-Cyclopentyloxy-4-methoxy-phenyl)-5-methyl-4,5-dihydro-isoxazol-5-yl]-piperdin-1-yl-methanone (Compound No. 176),

4-[3-(3-Cyclopentyloxy-4-methoxy-phenyl)-5-methyl-4,5-dihydro-isoxazole-5-carbonyl]-piperazine-1-carboxylic acid tert-butyl ester ( Compound No. 177),

1-[3-(3-Cyclopentyloxy-4-methoxy-phenyl)-5-methyl-4,5-dihydro-isoxazole-carbonyl]-pyrrolidin-2-carboxylic acid ( Compound No. 178),

1-[3-(3-Cyclopentyloxy-4-methoxy-phenyl)-5-methyl-4,5-dihydro-isoxazol-5-carbonyl]-pyrrolidine-2-carboxylic acid methyl ester ( Compound No. 179),

[3-(3-Cyclopentyloxy-4-methoxy-phenyl)-5-methyl-4,5-dihydro-isoxazole-5-yl]-pyrrolidin-1-yl-methanone ( Compound No. 180),

[1-4]-Bipiperidinyl-1-yl-[3-(3-cyclopentyloxy-4-methoxy-phenyl)-5-methyl-4-,5-dihydro-isoxazol-5-yl]-methanone ( Compound No. 181),

1-[3-(3-Cyclopentyloxy-4-methoxy-phenyl)-5-methyl-4,5-dihydro-isoxazole-5-carbonyl]-4-phenyl-piperidine-4-yl}-ethanone ( Compound No. 182),

[3-(3-Cyclopentyloxy-4-methoxy-phenyl)-5-methyl-4,5-dihydro-isoxazol-5-yl]-(4-methyl-piperazin-1-yl)-methanone ( Compound No. 183),

[3-(3-Cyclopentyloxy-4-methoxy-phenyl)-5-methyl-4,5-dihydro-isoxazol-5-yl]- piperazin-1-yl-methanone (Compound No. 184),

[4-(4-Chloro-phenyl)-4-hydroxy-piperidin-1-yl]-[3-(3-cyclopentyloxy-4-methoxyphenyl)-5-methyl-4,5- dihydroisoxazol-5-yl]-methanone ( Compound No. 185),

{4-[3-(3-Cyclopentyloxy-4-methoxy-phenyl)-5-methyl-4,5-dihydro-isoxazole-5-carbonyl]-[1,4]diazepan-1-yl}-[3-(3-cyclopentyloxy-4-methoxy-phenyl)-5-methyl-4,5-dihydro-isoxazol-5-yl]-methanone ( Compound No. 186),

[3-(3-Cyclopentyloxy-4-methoxy-phenyl)-5-methyl-4,5-dihydro-isoxazol-5-yl]-(4-cyclopropylmethyl-piperazin-1-yl)-methanone ( Compound No. 187),

[3-(3-Cyclopentyloxy-4-methoxy-phenyl)-5-methyl-4,5-dihydro-isoxazol-5-yl]-(4-isobutyl-1-piperazin-1-yl)-methanone ( Compound No. 188),

[3-Hydroxymethyl-piperidin-1-yl]-[3-(3-cyclopentyloxy-4-methoxy-phenyl)-5-methyl-4,5-dihydro-isoxazol-5-yl]-methanone ( Compound No. 189),

[3-(3-Cyclopentyloxy-4-methoxy-phenyl)-5-methyl-4, 5-dihydro-isoxazol-5-yl]-(4-hydroxy-piperidin-1-yl)-methanone ( Compound No. 190) ,

(4-Benzyl-piperidin-1-yl)-[3-(3-cyclopentyloxy-4-methoxy-phenyl)-5-methyl-4, 5-dihydro-isoxazol-5-yl]-methanone (Compound No. 191),

1-[3-(3-Cyclopentyloxy-4-methoxy-phenyl)-5-methyl-4, 5-dihydro-isoxazole-5-carbonyl]-piperidin-4-one (Compound No. 192),

[4-(4-Bromophenyl)-4-hydroxy-piperidin-1-yl]-[3-(3-cyclopentyloxy-4-methoxy-phenyl)-5-methyl-4, 5-dihydro-isoxazol-5-yl]-methanone (Compound No. 193),

(5-Benzyl-2, 5-diaza-bicyclo [2.2.1] hept-2-yl- [3-(3-cyclopentyloxy-4-methoxy-phenyl)-5-methyl-4, 5-dihydro-isoxazol-5-yl]-methanone (Compound No. 194),

(4-Benzyl-piperazin-1-yl)-[3-(3-cyclopentyloxy-4-methoxy-phenyl)-5-methyl-4, 5-dihydro-isoxazol-5-yl)-methanone (Compound No. 195),

1-[3-(3-Cyclopentyloxy-4-methoxy-phenyl)-5-methyl-4, 5-dihydro-isoxazole-5-carbonyl]-pyrrolidin-2-carboxylic acid methyl amide (Compound No. 196),

1-[3-(3-Cyclopentyloxy-4-methoxy-phenyl)-5-methyl-4,5-dihydro-isoxazole-5-carbonyl]-pyrrolidine-2-carboxylic acid diethyl amide (Compound No. 197),

[3-(3-Cyclopentyloxy-4-methoxy-phenyl)-5-methyl-4,5-dihydro-isoxazol-5-yl]-(2-hydroxymethyl-pyrrolidin-1-yl)-methanone (Compound No. 198),

1-[3-(3-Cyclopentyloxy-4-methoxy-phenyl)-5-methyl-4,5-dihydroisoxazole-5-carbonyl]-piperidine-2-carboxylic acid methyl ester (Compound No. 199),

[3-(3-Cyclopentyloxy-4-methoxy-phenyl)-5-methyl-4,5-dihydro-isoxozole-5-carboxyl]-pyrrolidine-2-carboxylic acid amide (Compound No. 200),

3-[3-(3-Cyclopentyloxy-4-methoxy-phenyl)-5-methyl-4,5-dihydro-isoxazole-5-carbonyl]-bicyclo[2.2.1]heptan-2-one (Compound No. 201),

3-[3-Cyclopentyloxy-4-methoxy-phenyl)-1,7-dioxa-2-aza-spiro[4.4]non-2-en-6-one (Compound No. 202),

3-[3-Cyclopentyloxy-4-methoxy-phenyl)-7-methyl-1-oxa-2,7-diaza-spiro[4.4]non-2-ene-6,9-dione (Compound No. 203),

[3-(3-Cyclopentyloxy-4-methoxy-phenyl)-5-methyl-4,5-dihydro-isoxazol-5-yl-(2-methoxymethyl-pyrrolidin-1-yl)-methanone (Compound No. 204),

3-(3-Cyclopentyloxy-4-methoxy-phenyl)-1,7-dioxa-2-aza-spiro [4.4] non-2-ene (Compound No. 205),

3-(3-Cyclopropylmethoxy-4-methoxy-phenyl)-1,7-dioxa-2-aza-spiro[4.4]non-2-ene (Compound No. 206),

3-(4-Difluoromethoxy-3-propoxy-phenyl)-1,7-dioxa-2-aza-spiro[4.4]non-2-ene (Compound No. 207),

3-(4-Difluoro-3-butoxy-phenyl)-1,7-dioxa-2-aza-spiro[4.4]non-2-ene (Compound No. 208),

3-(4-Difluoromethoxy-3-isobutoxy-phenyl)-1,7-dioxa-2-aza-spiro [4.4] non-2-ene (Compound No. 209),

3-(3-Cyclopropylmethoxy-4-difluoromethoxy-phenyl)-1,7-dioxa-2-aza-spiro[4.4]non-2-ene (Compound No. 210),

3-(3-Benzyloxy-4-difluoromethoxy-phenyl)-1,7-dioxa-2-aza-spiro [4.4] non-2-ene (Compound No. 211),

3-(4-Difluoromethoxy-3-cyclopentyloxy-phenyl)-1,7-dioxa-2-aza-spiro[4.4]non-2-ene (Compound No. 212),

3-(3,4-Bis-difluoromethoxy-phenyl)-1,7-dioxa-2-aza-spiro[4.4]non-2-ene (Compound No. 213),

3-(3-Butoxy-4-difluoromethoxy-phenyl)-1,7-dioxa-2-aza-spiro [4,4] non-2-ene (Compound No. 214),

3-[3-(Bicyclo[2.2.1]hept-2-yloxy)-4-difluoromethoxy-phenyl]-1,7-dioxo-2-aza-spiro[4.4]non-2-ene (Compound No. 215),

3-(4-Difluoromethoxy-3-methoxy-phenyl)-1,7-dioxa-2-aza-spiro[4.4]non-2-ene (Compound No. 216),

3-(4-Benzyloxy-3-methoxy-phenyl)-1,7-dioxa-2-aza-spiro[4.4]non-2-ene (Compound No. 217),

3-(3-Cycloheptyloxy-4-difluoromethoxy-phenyl)-1,7-dioxa-2-aza-spiro[4.4]non-2-ene (Compound No. 218),

4-(1,7-Dioxa-2-aza-spiro[4.4]non-2-en-3-yl)-2-methoxy-phenol (Compound No. 219),

3-[3-(indan-2-yloxy)-4-methoxy-phenyl]-1,7-dioxa-2-aza-spiro [4.4] non-2-ene (Compound No. 220),

3-(4-Ethoxy-3-methoxy-phenyl)-1,7-dioxa-2-aza-spiro[4.4]non-2-ene (Compound No. 221),

3-(3-Methoxy-4-propoxy-phenyl)-1,7-dioxa-2-aza-spiro[4.4]non-2-ene (Compound No. 222),

3-(4-Isopropoxy-3-methoxy-phenyl)-1,7-dioxa-2-aza-spiro[4.4]non-2-ene (Compound No. 223),

3-(4-Butoxy-3-methoxy-phenyl)-1,7-dioxa-2-aza-spiro[4.4]non-2-ene (Compound No. 224),

3-(4-Cyclopentyloxy-3-methoxy-phenyl)-1,7-dioxa-2-aza-spiro [4.4] non-2-ene (Compound No. 225),

3-(4-(Isobutoxy-3-methoxy-phenyl)-1,7-dioxa-2-aza-spiro[4.4]non-2-ene (Compound No. 226),

3-(4-Cyclohexyloxy-3-methoxy-phenyl)-1,7-dioxa-2-aza-spiro [4.4] non-2-ene (Compound No. 227),

3-(4-Cyclopropylmethoxy-3-methoxy-phenyl)-1,7-dioxa-2-aza-spiro[4.4]non-2-ene (Compound No. 228),

3-(3,4-Dimethoxy-phenyl)-1,7-dioxa-2-aza-spiro[4.4]non-2-ene (Compound No. 229),

3-(3-Ethoxy-4-methoxy-phenyl)-1,7-dioxa-2-aza-spiro[4.4]non-2-ene (Compound No. 230),

3-(4-Methoxy-3-propoxy-phenyl)-1,7-dioxa-2-aza-spiro[4.4]non-2-ene (Compound No. 231),

3-(3-Isopropoxy-4-methoxy-phenyl)-1,7-dioxa-2-aza-spiro[4.4]non-2-ene (Compound No. 232),

3-(3-Butoxy-4-methoxy-phenyl)-1,7-dioxa-2-aza-spiro[4.4]non-2-ene (Compound No. 233),

3-(3-Isobutoxy-4-methoxy-phenyl)-1,7-dioxa-2-aza-spiro[4.4]non-2-ene (Compound No. 234),

3-[4-Methoxy-3-(3-methyl-butoxy)-phenyl-1,7-dioxa-2-aza-spiro[4.4]non-2-ene (Compound No. 235),

3-(3-Cyclohexyloxy-4-methoxy-phenyl)-1,7-dioxa-2-aza-spiro [4.4] non-2-ene (Compound No. 236),

3-(3-Cycloheptyloxy-4-methoxy-phenyl)-1,7-dioxa-2-aza-spiro [4.4] non-2-ene (Compound No. 237),

3-[4-Methoxy-3-(2-morpholin-4-yl-ethoxy)-phenyl]-1,7-dioxa-2-aza-spiro[4.4]non-2-ene (Compound No. 238),

3-(3-Benzyloxy-4-methoxy-phenyl)-1,7-dioxa-2-aza-spiro[4.4]non-2-ene (Compound No. 239),

5-(1,7-Dioxa-2-aza-spiro[4.4]non-2-en-3-yl)-2-methoxy-phenol (Compound No. 240),

3-(3-Cyclopentyloxy-4-methoxy-phenyl)-1-oxa-2,8-diaza-spiro[4.5]dec-2-ene-8-carboxylic acid isopropyl ester

(Compound No. 241),

Hydrochloride salt of 3-(3-cyclopentyloxy-4-methoxy-phenyl)-1-oxa-2,8-diaza-spiro[4.5]dec-2-ene (Compound No. 242),

4-Chloro-N-[3-(3-cyclopentyloxy-4-methoxy-phenyl)-1-oxa-2,8-diaza-spiro[4.5]dec-2-ene-8-carbonyl]-benzene sulfonamide (Compound No. 243),

3-(3-Cyclopentyloxy-4-methoxy-phenyl)-1-oxa-2, 8-diaza-spiro [4.5] dec-2-ene-8-carboxylic acid-(2,6-difluoro-phenyl)-amide (Compound No. 244),

3-(3-Cyclopentyloxy-4-methoxy-phenyl)-1-oxa-2,8-diaza-spiro[4.5]dec-2-ene-8-carboxylic acid-(2,4-dichloro-phenyl)-amide (Compound No. 245),

[3-(3-Cyclopentyloxy-4-methoxy-phenyl)-1-oxa-2-aza-spiro[4.5]dec-2-en-8-yl]-carbamic acid isopropyl ester (Compound No. 246),

Hydrochloride salt of 3-(3-cyclopentyloxy-4-methoxy-phenyl)-1-oxa-2-aza-spiro[4.5]dec-2-en-8-ylamine ( Compound No. 247),

2-[3-(3-Cyclopentyloxy-4-methoxy-phenyl)-1-oxa-2-aza-spiro[4.5]dec-2-en-8-yl]-isoindole-1,3-dione (Compound No. 248),

7-(3-Cyclopentyloxy-4-methoxy-phenyl)-5-oxa-6-aza-spiro[3.4]oct-6-ene (Compound No. 249),

3-(3-Cyclopentyloxy-4-methoxy-phenyl)-1-oxa-2-aza-spiro[4.5]dec-2-ene (Compound No. 250),

3-(3-Cyclopentyloxy-4-methoxy-phenyl)-1-oxa-2,7-diaza-spiro[4.4]non-2-ene-7-carboxylic acid tert-butyl ester (Compound No. 251),

Hydrochloride salt of 3-(3-cyclopentyloxy-4-methoxy-phenyl)-1-oxa-2,7-diaza-spiro[4.4]non-2-ene (Compound No. 252),

3-[3-{[(3*S*)-1-Benzylpyrrolidin-3-yl]oxy}-4-(difluoromethoxy)phenyl]-1,7-dioxa-2-azaspiro[4.4]non-2-ene (Compound No. 253),

3-[2-(Difluoromethoxy)-5-(1,7-dioxa-2-azaspiro[4.4]non-2-en-3-yl)phenoxy]propan-1-ol (Compound No. 254),

[2-(Difluoromethoxy)-5-(1,7-dioxa-2-azaspiro[4.4]non-2-en-3-yl)phenoxy]acetonitrile (Compound No. 255),

4-[(5S or 5R)-1,7-dioxa-2-azaspiro[4.4]non-2-en-3-yl]-2-methoxyphenol (Compound No. 256),

4-[(5R or 5S)-1,7-Dioxa-2-azaspiro[4.4]non-2-en-3-yl]-2-methoxyphenol (Compound No. 257),

5-[(5S or 5R)-1,7-Dioxa-2-azaspiro[4.4]non-2-en-3-yl]-2-methoxyphenol (Compound No. 258),

(5S or 5R)-3-(3,4-Dimethoxyphenyl)-1,7-dioxa-2-azaspiro[4.4]non-2-ene (Compound No. 259),

(5R or 5S)-3-(3,4-Dimethoxyphenyl)-1,7-dioxa-2-azaspiro[4.4]non-2-ene (Compound No. 260),

2-(Benzyloxy)-4-(1,7-dioxa-2-azaspiro[4.4]non-2-en-3-yl)phenol (Compound No. 261),

2-[2-(Difluoromethoxy)-5-(1,7-dioxa-2-azaspiro[4.4]non-2-en-3-yl)phenoxy]ethanol (Compound No. 262),

3-[4-(Difluoromethoxy)-3-ethoxyphenyl]-1,7-dioxa-2-azaspiro[4.4]non-2-ene (Compound No. 263),

3-[3-(Cyclohexyloxy)-4-(difluoromethoxy)phenyl]-1,7-dioxa-2-azaspiro[4.4]non-2-ene (Compound No. 264),

(5R or 5S)-3-[4-(Difluoromethoxy)-3-methoxyphenyl]-1,7-dioxa-2-azaspiro[4.4]non-2-ene (Compound No. 265),

(5S or 5R)-3-[4-(Difluoromethoxy)-3-methoxyphenyl]-1,7-dioxa-2-azaspiro[4.4]non-2-ene (Compound No. 266),

Ethyl [2-(difluoromethoxy)-5-(1,7-dioxa-2-azaspiro[4.4]non-2-en-3-yl)phenoxy]acetate (Compound No. 267),

3-[4-(Difluoromethoxy)-3-(2-morpholin-4-ylethoxy)phenyl]-1,7-dioxa-2-azaspiro[4.4]non-2-ene (Compound No. 268),

2-(Difluoromethoxy)-5-(1,7-dioxa-2-azaspiro[4.4]non-2-en-3-yl)phenyl cyclohexanecarboxylate (Compound No. 269),

5-[2-(Difluoromethoxy)-5-(1,7-dioxa-2-azaspiro[4.4]non-2-en-3-yl)phenoxy]pentanoic acid (Compound No. 270),

3-[3-(2,2,2-Trifluoroethoxy)-4-(difluoromethoxy)phenyl]-1,7-dioxa-2-azaspiro[4.4]non-2-ene (Compound No. 271),

3-[3-(Cyclopentylmethoxy)-4-(difluoromethoxy)phenyl]-1,7-dioxa-2-azaspiro[4.4]non-2-ene (Compound No. 272),

*N*-cyclopropyl-2-[2-(difluoromethoxy)-5-(1,7-dioxa-2-azaspiro[4.4]non-2-en-3-yl)phenoxy]acetamide (Compound No. 273),

2-[2-(Difluoromethoxy)-5-(1,7-dioxa-2-azaspiro[4.4]non-2-en-3-yl)phenoxy]acetamide (Compound No. 274),

2-[2-(Difluoromethoxy)-5-(1,7-dioxa-2-azaspiro[4.4]non-2-en-3-yl)phenoxy]-*N*-methylacetamide (Compound No. 275),

3-[3-(Cyclopentyloxy)-4-(2,2,2-trifluoroethoxy)phenyl]-1,7-dioxa-2-azaspiro[4.4]non-2-ene (Compound No. 276),

2-(Difluoromethoxy)-5-(1,7-dioxa-2-azaspiro[4.4]non-2-en-3-yl)phenyl cyclopropanecarboxylate (Compound No. 277),

2-(Difluoromethoxy)-5-(1,7-dioxa-2-azaspiro[4.4]non-2-en-3-yl)phenyl morpholine-4-carboxylate (Compound No. 278),

2-(Difluoromethoxy)-5-(1,7-dioxa-2-azaspiro[4.4]non-2-en-3-yl)phenyl benzoate (Compound No. 279),

5-[2-(Difluoromethoxy)-5-(1,7-dioxa-2-azaspiro[4.4]non-2-en-3-yl)phenoxy] pentanamide (Compound No. 280),

3-[3-Propoxy-4-(2,2,2-trifluoroethoxy)phenyl]-1,7-dioxa-2-azaspiro[4.4]non-2-ene (Compound No. 281),

3-[3-Isopropoxy-4-(2,2,2-trifluoroethoxy)phenyl]-1,7-dioxa-2-azaspiro[4.4]non-2-ene (Compound No. 282),

3-[3-(Cyclopropylmethoxy)-4-(2,2,2-trifluoroethoxy)phenyl]-1,7-dioxa-2-azaspiro[4.4]non-2-ene (Compound No. 283),

3-[3-(2,3-Dihydro-1*H*-inden-2-yloxy)-4-(2,2,2-trifluoroethoxy)phenyl]-1,7-dioxa-2-azaspiro[4.4]non-2-ene (Compound No. 284),

5-(1,7-Dioxa-2-azaspiro[4.4]non-2-en-3-yl)-2-(2,2,2-trifluoroethoxy)phenol (Compound No. 285),

3-[3-Methoxy-4-(2,2,2-trifluoroethoxy)phenyl]-1,7-dioxa-2-azaspiro[4.4]non-2-ene (Compound No. 286),

3-[3-Ethoxy-4-(2,2,2-trifluoroethoxy)phenyl]-1,7-dioxa-2-azaspiro[4.4]non-2-ene (Compound No. 287),

3-[3-Butoxy-4-(2,2,2-trifluoroethoxy)phenyl]-1,7-dioxa-2-azaspiro[4.4]non-2-ene (Compound No. 288),

3-[3-(Cyclohexylmethoxy)-4-(2,2,2-trifluoroethoxy)phenyl]-1,7-dioxa-2-azaspiro[4.4]non-2-ene (Compound No. 289),

3-{[2-(Difluoromethoxy)-5-(1,7-dioxa-2-azaspiro[4.4]non-2-en-3-yl)phenoxy]methyl} benzonitrile (Compound No. 290),

2-{2-[2-(difluoromethoxy)-5-(1,7-dioxa-2-azaspiro[4.4]non-2-en-3-yl)phenoxy]ethyl}-1*H*-isoindole-1,3(2*H*)-dione (Compound No. 291),

3-[3-(Cyclohexyloxy)-4-(2,2,2-trifluoroethoxy)phenyl]-1,7-dioxa-2-azaspiro[4.4]non-2-ene (Compound No. 292),

Ethyl [5-(1,7-dioxa-2-azaspiro[4.4]non-2-en-3-yl)-2-(2,2,2-trifluoroethoxy) phenoxy]acetate (Compound No. 293),

3-[3-(Cyclohexylmethoxy)-4-(difluoromethoxy)phenyl]-1,7-dioxa-2-azaspiro[4.4]non-2-ene (Compound No. 294),

Tert-butyl [2-(difluoromethoxy)-5-(1,7-dioxa-2-azaspiro[4.4]non-2-en-3-yl)phenoxy]acetate (Compound No. 295),

N-cyclopropyl-2-[5-(1,7-dioxa-2-azaspiro[4.4]non-2-en-3-yl)-2-(2,2,2-trifluoroethoxy) phenoxy]acetamide (Compound No. 296),

2-(Cyclopentyloxy)-4-[(5R or 5S)-1,7-dioxa-2-azaspiro[4.4]non-2-en-3-yl]phenol (Compound No. 297),

2-(Cyclopentyloxy)-4-[(5S or 5R)-1,7-dioxa-2-azaspiro[4.4]non-2-en-3-yl]phenol (Compound No. 298),

N-benzyl-2-[5-(1,7-dioxa-2-azaspiro[4.4]non-2-en-3-yl)-2-(2,2,2-trifluoroethoxy) phenoxy]acetamide (Compound No. 299),

N-Cyclopentyl-2-[5-(1,7-dioxa-2-azaspiro[4.4]non-2-en-3-yl)-2-(2,2,2-trifluoroethoxy) phenoxy]acetamide (Compound No. 300),

Tert-butyl 4-[2-(difluoromethoxy)-5-(1,7-dioxa-2-azaspiro[4.4]non-2-en-3-yl)phenoxy] piperidine-1-carboxylate (Compound No. 301),

Hydrochloride salt of 3-[4-(difluoromethoxy)-3-(piperidin-4-yloxy)phenyl]-1,7-dioxa-2-azaspiro[4.4]non-2-ene (Compound No. 302),

3-{3-[(1-Acetylpiperidin-4-yl)oxy]-4-(difluoromethoxy)phenyl}-1,7-dioxa-2-azaspiro [4.4]non-2-ene (Compound No. 303),

Tert-butyl (3S)-3-[2-(difluoromethoxy)-5-(1,7-dioxa-2-azaspiro[4.4]non-2-en-3-yl)phenoxy]pyrrolidine-1-carboxylate (Compound No. 304),

Tert-butyl (3R)-3-[2-(difluoromethoxy)-5-(1,7-dioxa-2-azaspiro[4.4]non-2-en-3-yl)phenoxy]pyrrolidine-1-carboxylate (Compound No. 305),

Tert-butyl 3-[2-(difluoromethoxy)-5-(1,7-dioxa-2-azaspiro[4.4]non-2-en-3-yl)phenoxy]piperidine-1-carboxylate (Compound No. 306),

Tert-butyl (2S)-2-{[2-(difluoromethoxy)-5-(1,7-dioxa-2-azaspiro[4.4]non-2-en-3-yl)phenoxy]methyl}pyrrolidine-1-carboxylate (Compound No. 307),

(5R or 5S)-3-[3-(cyclopentyloxy)-4-(difluoromethoxy)phenyl]-1,7-dioxa-2-azaspiro[4.4]non-2-ene (Compound No. 308),

(5S or 5R)-3-(3-isopropoxy-4-methoxyphenyl)-1,7-dioxa-2-azaspiro[4.4]non-2-ene (Compound No. 309),

(5S or 5R)-3-[3-(Cyclopropylmethoxy)-4-methoxyphenyl]-1,7-dioxa-2-azaspiro[4.4]non-2-ene (Compound No. 310),

2-(Cyclopropylmethoxy)-4-[(5S or 5R)-1,7-dioxa-2-azaspiro[4.4]non-2-en-3-yl]phenol (Compound No. 311),

4-[(5S or 5R)-1,7-Dioxa-2-azaspiro[4.4]non-2-en-3-yl]-2-isopropoxyphenol (Compound No. 312),

(5S or 5R)-3-[3-(cyclopentyloxy)-4-(difluoromethoxy)phenyl]-1,7-dioxa-2-azaspiro[4.4]non-2-ene (Compound No. 313),

(5S or 5R)-3-[3-(Cyclopropylmethoxy)-4-(difluoromethoxy)phenyl]-1,7-dioxa-2-azaspiro[4.4]non-2-ene (Compound No. 314),

(5S or 5R)-3-[4-(difluoromethoxy)-3-isopropoxyphenyl]-1,7-dioxa-2-azaspiro[4.4]non-2-ene (Compound No. 315),

(5R or 5S)-3-[4-(difluoromethoxy)-3-isopropoxyphenyl]-1,7-dioxa-2-azaspiro[4.4]non-2-ene (Compound No. 316),

2-(Cyclopropylmethoxy)-4-[(5R or 5S)-1,7-dioxa-2-azaspiro[4.4]non-2-en-3-yl]phenol (Compound No. 317),

4-[(5R or 5S)-1,7-Dioxa-2-azaspiro[4.4]non-2-en-3-yl]-2-isopropoxyphenol (Compound No. 318),

(5R or 5S)-3-[3-(Cyclopropylmethoxy)-4-(difluoromethoxy)phenyl]-1,7-dioxa-2-azaspiro[4.4]non-2-ene (Compound No. 319),

(5R or 5S)-3-[4-(difluoromethoxy)-3-isopropoxyphenyl]-1,7-dioxa-2-azaspiro[4.4]non-2-ene (Compound No. 320),

Hydrochloride salt of 3-{4-(difluoromethoxy)-3-[(3S)-pyrrolidin-3-yloxy]phenyl}-1,7-dioxa-2-azaspiro[4.4]non-2-ene (Compound No. 321),

Hydrochloride salt of 3-{4-(difluoromethoxy)-3-[(2S)-pyrrolidin-2-ylmethoxy]phenyl}-1,7-dioxa-2-azaspiro[4.4]non-2-ene (Compound No. 322),

Hydrochloride salt of 3-{4-(difluoromethoxy)-3-[(2*R*)-pyrrolidin-2-ylmethoxy]phenyl}-1,7-dioxa-2-azaspiro[4.4]non-2-ene (Compound No. 323),

3-[4-(Difluoromethoxy)-3-{[(2*R*)-1-propionylpyrrolidin-2-yl]methoxy}phenyl]-1,7-dioxa-2-azaspiro[4.4]non-2-ene (Compound No. 324),

3-[3-{[(2*S*)-1-acetylpyrrolidin-2-yl]methoxy}-4-(difluoromethoxy)phenyl]-1,7-dioxa-2-azaspiro[4.4]non-2-ene (Compound No. 325),

3-[3-[(3S)-1-benzoylpyrrolidin-3-yl]oxy}-4-(difluoromethoxy)phenyl]-1,7-dioxa-2-azaspiro[4.4]non-2-ene (Compound No. 326),

3-[4-(Difluoromethoxy)-3-{[(3S)-1-propionylpyrrolidin-3-yl]oxy}phenyl]-1,7-dioxa-2-azaspiro[4.4]non-2-ene (Compound No. 327),

(5S or 5R)-3-[3-(Benzyloxy)-4-(difluoromethoxy)phenyl]-1,7-dioxa-2-azaspiro[4.4]non-2-ene (Compound No. 328),

2-(Benzyloxy)-4-[(5S or 5R)-1,7-dioxa-2-azaspiro[4.4]non-2-en-3-yl]phenol (Compound No. 329),

(5S or 5R)-3-[3-(Benzyloxy)-4-methoxyphenyl]-1,7-dioxa-2-azaspiro[4.4]non-2-ene (Compound No. 330),

3-{4-(Difluoromethoxy)-3-[(1-propionylpiperidin-4-yl)oxy]phenyl}-1,7-dioxa-2-azaspiro[4.4]non-2-ene (Compound No. 331),

3-[4-(Difluoromethoxy)-3-{[1-(4-fluorobenzoyl)piperidin-4-yl]oxy}phenyl]-1,7-dioxa-2-azaspiro[4.4]non-2-ene (Compound No. 332),

3-[3-{[1-(Cyclopropylcarbonyl)piperidin-4-yl]oxy}-4-(difluoromethoxy)phenyl]-1,7-dioxa-2-azaspiro[4.4]non-2-ene (Compound No. 333),

3-[3-{[1-(Cyclopentylcarbonyl)piperidin-4-yl]oxy}-4-(difluoromethoxy)phenyl]-1,7-dioxa-2-azaspiro[4.4]non-2-ene (Compound No. 334),

3-[4-(Difluoromethoxy)-3-({1-[(trifluoromethyl)sulfonyl]piperidin-4-yl}oxy)phenyl]-1,7-dioxa-2-azaspiro[4.4]non-2-ene (Compound No. 335),

3-{3-[(1-Acetylpiperidin-3-yl)oxy]-4-(difluoromethoxy)phenyl}-1,7-dioxa-2-azaspiro[4.4]non-2-ene (Compound No. 336),

3-{4-(Difluoromethoxy)-3-[(1-propionylpiperidin-3-yl)oxy]phenyl}-1,7-dioxa-2-azaspiro[4.4]non-2-ene (Compound No. 337),

3-[4-(Difluoromethoxy)-3-{[1-(4-fluorobenzoyl)piperidin-3-yl]oxy}phenyl]-1,7-dioxa-2-azaspiro[4.4]non-2-ene (Compound No. 338),

3-[3-{[1-(Cyclopropylcarbonyl)piperidin-3-yl]oxy}-4-(difluoromethoxy)phenyl]-1,7-dioxa-2-azaspiro[4.4]non-2-ene (Compound No. 339),

3-[3-{[1-(Cyclopentylcarbonyl)piperidin-3-yl]oxy}-4-(difluoromethoxy)phenyl]-1,7-dioxa-2-azaspiro[4.4]non-2-ene (Compound No. 340),

3-[4-(Difluoromethoxy)-3-{[1-(ethylsulfonyl)piperidin-3-yl]oxy}phenyl]-1,7-dioxa-2-azaspiro[4.4]non-2-ene (Compound No. 341),

3-[3-(Benzyloxy)-4-(2,2,2-trifluoroethoxy)phenyl]-1,7-dioxa-2-azaspiro[4.4]non-2-ene (Compound No. 342),

2-(Difluoromethoxy)-5-[(5*S or 5R*)-1,7-dioxa-2-azaspiro[4.4]non-2-en-3-yl]phenol (Compound No. 343), or

5-[(5R or 5S)-1,7-Dioxa-2-azaspiro[4.4]non-2-en-3-yl]-2-methoxyphenol (Compound No. 344).

Herein are provided pharmaceutical dosage forms comprising a therapeutically effective amount of one or more compounds of Formula Ia and Formula Ib and one or more pharmaceutically acceptable excipients.

In another aspect, herein are provided pharmaceutical dosage forms comprising a therapeutically effective amount of one or more compounds of Formula Ia or Formula Ib, a therapeutically effective amount of one or more muscarinic receptor antagonists (MRA), and one or more pharmaceutically acceptable excipients. The pharmaceutical dosage form may also include a therapeutically effective amount of one or more corticosteroids, one or more β2-agonists, one or more p38 MAP kinase inhibitors, one or more anticholinergics, one or more antiallergics, one or more PAF antagonists, one or more leukotriene antagonists, one or more EGFR kinase inhibitors, or one or more additional PDE-IV inhibitors or combinations thereof.

In another aspect, herein are provided pharmaceutical dosage forms comprising a therapeutically effective amount of one or more compounds of Formula Ia or Formula Ib, a therapeutically effective amount of one or more β2-agonists, and one or more pharmaceutically acceptable excipients. The pharmaceutical dosage form may also include a therapeutically effective amount of one or more corticosteroids, one or more muscarinic receptor antagonists, one or more p38 MAP kinase inhibitors, one or more anticholinergics, one or more antiallergics, one or more PAF antagonists, one or more leukotriene antagonists, one or more EGFR kinase inhibitors, or one or more additional PDE-IV inhibitors or combinations thereof.

In yet another aspect, herein are provided pharmaceutical dosage forms comprising a therapeutically effective amount of one or more compounds of Formula Ia or Formula Ib, a therapeutically effective amount of one or more p38 MAP kinase inhibitors, and one or more pharmaceutically acceptable excipients. The pharmaceutical dosage form may also include a therapeutically effective amount of one or more β2-agonists, one or more muscarinic receptor antagonists, one or more corticosteroids, one or more anticholinergics, one or more antiallergics, one or more PAF antagonists, one or more leukotriene antagonists, one or more EGFR kinase inhibitors, or one or more additional PDE-IV inhibitors or combinations thereof.

In still another aspect, herein are provided pharmaceutical dosage forms comprising a therapeutically effective amount of one or more compounds of Formula Ia or Formula Ib, a therapeutically effective amount of one or more corticosteroids, and one or more pharmaceutically acceptable excipients. The pharmaceutical dosage form may also include a therapeutically effective amount of one or more β2-agonists, one or more muscarinic receptor antagonists, one or more p38 MAP kinase inhibitors, one or more anticholinergics, one or more antiallergics, one or more PAF antagonists, one or more leukotriene antagonists, one or more EGFR kinase inhibitors, or one or more additional PDE-IV inhibitors or combinations thereof.

*tert*-Butyl 4-{[6-(2-methylphenyl)-7-oxo-8-(tetrahydro-2*H*-pyran-4-yl)-7,8-dihydropyrido[2,3-*d*]pyrimidin-2-yl]amino}piperidine-1-carboxylate (Compound No. 1a), Hydrochloride salt of 6-(2-methylphenyl)-2-(piperidin-4-ylamino)-8-(tetrahydro-2H-pyran-4-yl)pyrido[2,3-*d*]pyrimidin-7(8H)-one (Compound No. 2a),

2-[(1-Acetylpiperidin-4-yl)amino]-6-(2-methylphenyl)-8-(tetrahydro-2H-pyran-4-yl)pyrido[2,3-*d*]pyrimidin-7(8H)-one (Compound No. 3a),

6-(2-Methylphenyl)-2-{[1-(methylsulfonyl)piperidin-4-yl]amino}-8-(tetrahydro-2H-pyran-4-yl)pyrido[2,3-*d*]pyrimidin-7(8H)-one (Compound No. 4a),

2-[(1-Benzoylpiperidin-4-yl)amino]-6-(2-methylphenyl)-8-(tetrahydro-2H-pyran-4-yl)pyrido[2,3-*d*]pyrimidin-7(8H)-one (Compound No. 5a),

*N*-Isopropyl-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 6a),

*N*-(4-Fluorophenyl)-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 7a),

2-{[1-(Ethylsulfonyl)piperidin-4-yl]amino}-6-(2-methylphenyl)-8-(tetrahydro-2H-pyran-4-yl)pyrido[2,3-*d*]pyrimidin-7(8H)-one (Compound No. 8a),

2-[(1-Benzyl-piperidin-4-yl)amino]-6-(2-methyl-phenyl)-8-(tetrahydro-2H-pyran-4-yl)pyrido[2,3-*d*]pyrimidin-7(8H)-one (Compound No. 9a),

*N*-(4-Fluorophenyl)-4-{[6-(2-methylphenyl)-7-oxo-8-(tetrahydro-2*H*-pyran-4-yl)-7,8-dihydropyrido[2,3-*d*]pyrimidin-2-yl]amino}piperidine-1-carbothioamide (Compound No. 10a),

Methylphenyl)-7-oxo-8-(tetrahydro-2*H*-pyran-4-yl)-7,8-dihydropyrido[2,3-*d*]pyrimidin-2-yl]amino}-*N*-[4-(trifluoromethyl)phenyl]piperidine-1-carboxamide (Compound No. 11a),

6-(2-Methylphenyl)-2-[(1-methylpiperidin-4-yl)amino]-8-(tetrahydro-2H-pyran-4-yl)pyrido[2,3-*d*]pyrimidin-7(8H)-one (Compound No. 12a),

6-(2-Methylphenyl)-2-[(4-methylpiperazin-1-yl)amino]-8-(tetrahydro-2*H*-pyran-4-yl)pyrido[2,3-*d*]pyrimidin-7(8*H*)-one (Compound No. 13a),

2-{[1-(Isopropylsulfonyl)piperidin-4-yl]amino}-6-(2-methylphenyl)-8-(tetrahydro-2H-pyran-4-yl)pyrido[2,3-*d*]pyrimidin-7(8H)-one (Compound No. 14a),

*tert*-Butyl 4-{[6-(2-chlorophenyl)-7-oxo-8-(tetrahydro-2*H*-pyran-4-yl)-7,8-dihydropyrido[2,3-*d*]pyrimidin-2-yl]amino}pipendine-1-carboxylate (Compound No. 15a),

6-(2-Methylphenyl)-2-(piperidin-1-ylamino)-8-(tetrahydro-2*H*-pyran-4-yl)pyrido[2,3-*d*]pyrimidin-7(8*H*)-one (Compound No. 16a),

2-(Cyclobutylamino)-6-(2-methylphenyl)-8-(tetrahydro-2H-pyran-4-yl)pyrido[2,3-*d*]pyrimidin-7(8H)-one (Compound No. 17a)

4-{[6-(2-Methylphenyl)-7-oxo-8-(tetrahydro-2H-pyran-4-yl)-7,8-dihydropyrido[2,3-*d*]pyrimidin-2-yl]amino}-*N*-propylpiperidine-1-carboxamide (Compound No. 18a),

*N*-[(1S)-1,2-Dimethylpropyl]-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 19a),

*N*-Cyclohexyl-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 20a),

2-{[1-(4-Fluorobenzoyl)piperidin-4-yl]amino}-6-(2-methylphenyl)-8-(tetrahydro-2H-pyran-4-yl)pyrido[2,3-*d*]pyrimidin-7(8H)-one (Compound No. 21a),

*N*-(Cyclopentylmethyl)-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 22a),

4-{[6-(2-Methylphenyl)-7-oxo-8-(tetrahydro-2H-pyran-4-yl)-7,8-dihydropyrido[2,3-*d*]pyrimidin-2-yl]amino}-*N*-(1,1,3,3-tetramethylbutyl)piperidine-1-carboxamide (Compound No. 23a),

4-{[6-(2-Methylphenyl)-7-oxo-8-(tetrahydro-2H-pyran-4-yl)-7,8-dihydropyrido[2,3-*d*]pyrimidin-2-yl]amino}-*N*-octylpiperidine-1-carboxamide (Compound No. 24a),

*N*-Cyclopentyl-4-[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 25a),

*N*-Isopropyl-4-[6-(2-methylphenyl)-7-oxo-8-(tetrahydro-2H-pyran-4-yl)-7,8-dihydropyrido[2,3-*d*]pyrimidin-2-yl]amino}piperidine-1-carbothioamide (Compound No. 26a),

4-{[6-(2-Methylphenyl)-7-oxo-8-(tetrahydro-2H-pyran-4-yl)-7,8-dihydropyrido[2,3-*d*]pyrimidin-2-yl]amino}-*N*-octylpiperidine-1-carbothioamide (Compound No. 27a),

*N*-*tert*-Butyl-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carbothioamide (Compound No. 28a),

6-(2-Methylphenyl)-2-[(1-pyrimidin-2-ylpiperidin-4-yl)amino]-8-(tetrahydro-2H-pyran-4-yl)pyrido[2,3-*d*]pyrimidin-7(8H)-one (Compound No. 29a),

*N*-Cyclopropyl-4-[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 30a),

*N*-[(1R)-1-Cyclohexylethyl]-4-{[6-(2-methylphenyl)-7-oxo-8-(tetrahydro-2H-pyran-4-yl)-7,8-dihydropyrido[2,3-*d*]pyrimidin-2-yl]amino}piperidine-1-carboxamide (Compound No. 31a),
2-{[1-(Cyclopentylcarbonyl)piperidin-4-yl]amino}-6-(2-methylphenyl)-8-(tetrahydro-2H-pyran-4-yl)pyrido[2,3-*d*]pyrimidin-7(8H)-one (Compound No. 32a),
6-(2-Methylphenyl)-2-{[1-(pyrrolidin-1-ylcarbonyl)piperidin-4-yl]amino}-8-(tetrahydro-2H-pyran-4-yl)pyrido[2,3-*d*]pyrimidin-7(8H)-one (Compound No. 33a),
6-(2-Methylphenyl)-8-(tetrahydro-2H-pyran-4-yl)-2-(tetrahydro-2H-pyran-4-ylamino)pyrido[2,3-*d*]pyrimidin-7(8H)-one (Compound No. 34a),
6-(2-Methylphenyl)-2-[(1,2,2,6,6-pentamethylpiperidin-4-yl)amino]-8-(tetrahydro-2H-pyran-4-yl)pyrido[2,3-*d*]pyrimidin-7(8H)-one (Compound No. 35a),
*tert*-Butyl 4-{[6-(2-methylphenyl)-7-oxo-8-(tetrahydrofuran-3-yl)-7,8-dihydropyrido[2,3-*d*]pyrimidin-2-yl]amino}piperidine-1-carboxylate (Compound No. 36a),
*N*-Isopropyl-4-{[6-(2-methylphenyl)-7-oxo-8-(tetrahydrofuran-3-yl)-7,8-dihydropyrido[2,3-*d*]pyrimidin-2-yl]amino}piperidine-1-carboxamide (Compound No. 37a),
6-(2-Methylphenyl)-7-oxo-2-{[1-(pyrrolidin-1-ylcarbonyl)piperidin-4-yl]amino}-1-(tetrahydrofuran-3-yl)-7,8-dihydropyrido[2,3-*d*]pyrimidin-1-ium (Compound No. 38a),
2-{[1-(Ethylsulfonyl)piperidin-4-yl]amino}-6-(2-methylphenyl)-7-oxo-1-(tetrahydrofuran-3-yl)-7,8-dihydropyrido[2,3-*d*]pyrimidin-1-ium (Compound No. 39a),
6-(2- Methylphenyl)- 2- { [1-(methylsulfonyl) piperidin- 4- yl] amino}- 8-(tetrahydrofuran- 3- yl) pyrido [2,3-*d*] pyrimidin- 7 (8H)-one (Compound No. 40a),
*N*-Cyclopropyl-4-[6-(2-methylphenyl)-7-oxo-8-(tetrahydrofuran-3-yl)-7,8-dihydropyrido[2,3-*d*]pyrimidin-2-yl]amino}piperidine-1-carboxamide (Compound No. 41a),
6-(2-Methylphenyl)-2-{[(3S)-1-(methylsulfonyl)pyrrolidin-3-yl]amino}-8-(tetrahydrofuran-3-yl)pyrido[2,3-*d*]pyrimidin-7 (8H)-one (Compound No. 42a),
*tert*-Butyl 4-({6-(2-methylphenyl)-7-oxo-8-[(3S)-tetrahydrofuran-3-yl]-7,8-dihydropyrido[2,3-*d*]pyrimidin-2-yl}amino)piperidine-1-carboxylate (Compound No. 43a),
6-(2-Methylphenyl)-2-{[1-(methylsulfonyl)piperidin-4-yl]amino}-8-[(3S)-tetrahydrofuran-3-yl]pyrido[2,3-*d*]pyrimidin-7 (8H)-one (Compound No. 44a),
*N*-Isopropyl-4-({6-(2-methylphenyl)-7-oxo-8-[(3S)-tetrahydrofuran-3-yl]-7,8-dihydropyrido[2,3-*d*]pyrimidin-2-yl}amino) piperidine-1-carboxamide (Compound No. 45a),
6-(2-Methylphenyl)-2-{[1-(methylsulfonyl)piperidin-4-yl]amino}-8-[(3R)-tetrahydrofuran-3-yl]pyrido[2,3-*d*]pyrimidin-7 (8H)-one (Compound No. 46a),
*N*-Isopropyl-4-({6-(2-methylphenyl)-7-oxo-8-[(3R)-tetrahydrofuran-3-yl]-7,8-dihydropyrido[2,3-*d*]pyrimidin-2-yl}amino) piperidine-1-carboxamide (Compound No. 47a),
*tert*-Butyl 4-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxylate(Compound No. 48a),
Hydrochloride salt of 6-(2-methylphenyl)-2-(piperidin-4-ylamino)-8-[(3R)-tetrahydrofuran-3-yl]pyrido[2,3-*d*]pyrimidin-7 (8H)-one (Compound No. 49a),
Hydrochloride salt 6-(2-methylphenyl)-2-(piperidin-4-ylamino)-8-[(3S)-tetrahydrofuran-3-yl]pyrido[2,3-*d*]pyrimidin-7 (8H)-one (Compound No. 50a),
2-{[1-(Ethylsulfonyl)piperidin-4-yl]amino}-6-(2-methylphenyl)-8-[(3S)-tetrahydrofuran-3-yl]pyrido[2,3-*d*]pyrimidin-7 (8H)-one (Compound No. 51a),
*N*-Cyclopropyl-4-({6-(2-methylphenyl)-7-oxo-8-[(3S)-tetrahydrofuran-3-yl]-7,8-dihydropyrido[2,3-*d*]pyrimidin-2-yl}amino)piperidine-1-carboxamide (Compound No. 52a),
*N*-Cyclopropyl-4-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxamide (Compound No. 53a),
2-[(1-Benzylpiperidin-4-yl)amino]-6-(2-methylphenyl)-8-[(3R)-tetrahydrofuran-3-yl]pyrido[2,3-*d*]pyrimidin-7(8H)-one (Compound No. 54a),
2-(Cyclobutylamino)-6-(2-methylphenyl)-8-[(3R)-tetrahydrofuran-3-yl]pyrido[2,3-*d*]pyrimidin-7(8H)-one (Compound No. 55a),
2-(Cyclopropylamino)-6-(2-methylphenyl)-8-[(3R)-tetrahydrofuran-3-yl]pyrido[2,3-*d*]pyrimidin-7(8H)-one (Compound No. 56a),
2-{[1-(Ethylsulfonyl)piperidin-4-yl]amino}-6-(2-methylphenyl)-8-[(3R)-tetrahydrofuran-3-yl]pyrido[2,3-*d*]pyrimidin-7 (8H)-one (Compound No.57a),
2-[(1-Acetylpiperidin-4-yl)amino]-6-(2-methylphenyl)-8-[(3R)-tetrahydrofuran-3-yl]pyrido[2,3-*d*]pyrimidin-7(8H)-one (Compound No. 58a),
2-{[1-(4-Fluorobenzoyl)piperidin-4-yl]amino}-6-(2-methylphenyl)-8-[(3R)-tetrahydrofuran-3-yl]pyrido[2,3-*d*]pyrimidin-7 (8H)-one (Compound No. 59a),

2-{[1-(Cyclopropylcarbonyl)piperidin-4-yl]amino}-6-(2-methylphenyl)-8-[(3R)-tetrahydrofuran-3-yl]pyrido[2,3-*d*]pyrimidin-7(8H)-one (Compound No. 60a),
2-{[1-(Cyclopropylcarbonyl)piperidin-4-yl]amino}-6-(2-methylphenyl)-8-[(3S)-tetrahydrofuran-3-yl]pyrido[2,3-*d*]pyrimidin-7(8H)-one (Compound No. 61a),
2-{[1-(Cyclopentylcarbonyl)piperidin-4-yl]amino}-6-(2-methylphenyl)-8-[(3S)-tetrahydrofuran-3-yl]pyrido[2,3-*d*]pyrimidin-7(8H)-one (Compound No. 62a),
2-{[1-(Cyclopentylcarbonyl)piperidin-4-yl]amino}-6-(2-methylphenyl)-8-[(3R)-tetrahydrofuran-3-yl]pyrido[2,3-*d*]pyrimidin-7(8H)-one (Compound No. 63a),
2-[(1-Benzoylpiperidin-4-yl)amino]-6-(2-methylphenyl)-8-[(3S)-tetrahydrofuran-3-yl]pyrido[2,3-*d*]pyrimidin-7(8H)-one (Compound No. 64a),
2-[(1-Benzoylpiperidin-4-yl)amino]-6-(2-methylphenyl)-8-[(3R)-tetrahydrofuran-3-yl]pyrido[2,3-*d*]pyrimidin-7(8H)-one (Compound No. 65a),
2-{[1-(4-Fluorobenzoyl)piperidin-4-yl]amino}-6-(2-methylphenyl)-8-[(3S)-tetrahydrofuran-3-yl]pyrido[2,3-*d*]pyrimidin-7(8H)-one (Compound No. 66a),
N-(4-Fluorophenyl)-4-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxamide (Compound No. 67a),
2-[(1-Acetylpiperidin-4-yl)amino]-6-(2-methylphenyl)-8-[(3S)-tetrahydrofuran-3-yl]pyrido[2,3-*d*]pyrimidin-7(8H)-one (Compound No. 68a),
*N*-(4-Fluorophenyl)-4-({6-(2-methylphenyl)-7-oxo-8-[(3S)-tetrahydrofuran-3-yl]-7,8-dihydropyrido[2,3-d]pyrimidin-2-yl}amino)piperidine-1-carboxamide (Compound No. 69a),
*tert*-Butyl (3S)-3-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxylate (Compound No. 70a),
(3S)-*N*-Isopropyl-3-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxamide (Compound No. 71a)
*tert*-Butyl (3S)-3-({6-(2-methylphenyl)-7-oxo-8-[(3S)-tetrahydrofuran-3-yl]-7,8-dihydropyrido[2,3-*d*]pyrimidin-2-yl}amino)piperidine-1-carboxylate (Compound No. 72a),
*tert*-Butyl (3R)-3-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxylate (Compound No. 73a),
(3R)-*N*-Isopropyl-3-({6-(2-methylphenyl)-7-oxo-8-[(3R)-tetrahydrofuran-3-yl]-7,8-dihydropyrido[2,3-*d*]pyrimidin-2-yl}amino)piperidine-1-carboxamide (Compound No. 74a),
(3S)-*N*-Isopropyl-3-({6-(2-methylphenyl)-7-oxo-8-[(3S)-tetrahydrofuran-3-yl]-7,8-dihydropyrido[2,3-*d*]pyrimidin-2-yl}amino)piperidine-1-carboxamide (Compound No. 75a),
6-(2-Methylphenyl)-2-{[(3S)-1-(methylsulfonyl)piperidin-3-yl]amino}-8-[(3S)-tetrahydrofuran-3-yl]pyrido[2,3-*d*]pyrimidin-7(8H)-one (Compound No. 76a),
6-(2-Methylphenyl)-2-{[1-(methylsulfbnyl)piperidin-4-yl]amino}-8-[(3S)-1-(methylsulfonyl)pyrrolidin-3-yl]pyrido[2,3-*d*]pyrimidin-7(8H)-one (Compound No. 77a),
6-(2-Methylphenyl)-2-{[1-(methylsulfbnyl)piperidin-4-yl]amino}-8-[(3S)-1-(methylsulfonyl)pyrrolidin-3-yl]pyrido[2,3-*d*]pyrimidin-7(8H)-one (Compound No. 78a),
*tert*-Butyl 4-({6-(2-methylphenyl)-7-oxo-8-[(3S)-pyrrolidin-3-yl]-7,8-dihydropyrido[2,3-*d*]pyrimidin-2-yl}amino)piperidine-1-carboxylate (Compound No. 79a)
4-({8-[(3S)-1-Acetylpyrrolidin-3-yl]-6-(2-methylphenyl)-7-oxo-7,8-dihydropyrido[2,3-*d*]pyrimidin-2-yl}amino)-*N*-isopropylpiperidine-1-carboxamide (Compound No. 80a),
Hydrochloride salt of 8-[(3S)-1-acetylpyrrolidin-3-yl]-6-(2-methylphenyl)-2-(piperidin-4-ylamino)pyrido[2,3-*d*]pyrimidin-7(8H)-one (Compound No. 81a),
*tert*-Butyl (3S)-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxylate (Compound No. 82a),
(3S)-*N*-Isopropyl-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 83a),
(3S)-*N*-Isopropyl-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carbothioamide (Compound No. 84a),
6-(2-Methylphenyl)-2-{[(3S)-1-(methylsulfonyl)pyrrolidin-3-yl]amino}-8-(tetrahydro-2H-pyran-4-yl)pyrido[2,3-*d*]pyrimidin-7(8H)-one (Compound No. 85a),
2-{[(3S)-1-(Ethylsulfonyl)pyrrolidin-3-yl]amino}-6-(2-methylphenyl)-8-(tetrahydro-2H-pyran-4-yl)pyrido[2,3-*d*]pyrimidin-7(8H)-one (Compound No. 86a),
2-{[(3S)-1-Acetylpyrrolidin-3-yl]amino}-6-(2-methylphenyl)-8-(tetrahydro-2H-pyran-4-yl)pyrido[2,3-*d*]pyrimidin-7(8H)-one (Compound No. 87a),
(3S)-*N*-Cyclopropyl-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 88a),

(3S)-*N*-Butyl-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 89a),
(3S)-*N*-Cyclopentyl-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 90a),
(3S)-*N*-[(1S)-1,2-Dimethylpropyl]-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 91a),
2-{[(3R)-1-Benzylpyrrolidin-3-yl]amino}-6-(2-methylphenyl)-8-(tetrahydro-2H-pyran-4-yl)pyrido[2,3-*d*]pyrimidin-7(8H)-one (Compound No. 92a),
2-{[(3S)-1-Benzylpyrrolidin-3-yl]amino}-6-(2-methylphenyl)-8-(tetrahydro-2H-pyran-4-yl)pyrido[2,3-*d*]pyrimidin-7(8H)-one (Compound No. 93a),
(3S)-*N*-Cyclohexyl-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 94a),
(3S)-3-{[6-(2-Methylphenyl)-7-oxo-8-(tetrahydro-2H-pyran-4-yl)-7,8-dihydropyrido[2,3-*d*]pyrimidin-2-yl]amino}-*N*-octylpyrrolidine-1-carboxamide (Compound No. 95a),
2-{[(3S)-1-(Cyclopropylcarbonyl)pyrrolidin-3-yl]amino}-6-(2-methylphenyl)-8-(tetrahydro-2H-pyran-4-yl)pyrido[2,3-*d*]pyrimidin-7(8H)-one (Compound No. 96a),
2-{[(3S)-1-(Cyclopentylcarbonyl)pyrrolidin-3-yl]amino}-6-(2-methylphenyl)-8-(tetrahydro-2H-pyran-4-yl)pyrido[2,3-*d*]pyrimidin-7(8H)-one (Compound No. 97a),
6-(2-Methylphenyl)-2-{[(3S)-1-pyrimidin-2-yl-pyrrolidin-3-yl]amino}-8-(tetrahydro-2H-pyran-4-yl)pyrido[2,3-*d*]pyrimidin-7(8H)-one (Compound No. 98a)
(3S)-*N*-[(1R)-1-Cyclohexylethyl]-3-{[6-(2-methylphenyl)-7-oxo-8-(tetrahydro-2H-pyran-4-yl)-7,8-dihydropyrido[2,3-*d*]pyrimidin-2-yl]amino}pyrrolidine-1-carboxamide (Compound No. 99a),
6-(2-Methylphenyl)-2-{[(3S)-1-(pyrrolidin-1-ylcarbonyl)pyrrolidin-3-yl]amino}-8-(tetrahydro-2H-pyran-4-yl)pyrido[2,3-*d*]pyrimidin-7(8H)-one (Compound No. 100a),
2-{[(3S)-1-(Cyclopentylacetyl)pyrrolidin-3-yl]amino}-6-(2-methylphenyl)-8-(tetrahydro-2H-pyran-4-yl)pyrido[2,3-*d*]pyrimidin-7(8H)-one (Compound No. 101a),
*tert*-Butyl (3S)-3-{[6-(2-methylphenyl)-7-oxo-8-(tetrahydrofuran-3-yl)-7,8-dihydropyrido[2,3-*d*]pyrimidin-2-yl]amino}pyrrolidine-l-carboxylate (Compound No. 102a),
*tert*-Butyl 4-({8-[(3S)-1-benzylpyrrolidin-3-yl]-6-(2-methylphenyl)-7-oxo-7,8-dihydropyrido[2,3-*d*]pyrimidin-2-yl}amino)piperidine-1-carboxylate (Compound No. 103a),
(3S)-*N*-Isopropyl-3-{[6-(2-methylphenyl)-7-oxo-8-(tetrahydrofuran-3-yl)-7,8-dihydropyrido[2,3-*d*]pyrimidin-2-yl]amino}pyrrolidine-1-carboxamide (Compound No. 104a),
*tert*-Butyl 4-{[8-(1-benzylpiperidin-4-yl)-6-(2-methylphenyl)-7-oxo-7,8-dihydropyrido[2,3-*d*]pyrimidin-2-yl]amino}piperidine-1-carboxylate (Compound No. 105a),
*tert*-Butyl 4-{[6-(2-methylphenyl)-8-(1methylpiperidin-4-yl)-7-oxo-7,8-dihydropyrido[2,3-*d*]pyrimidin-2-yl]amino}piperidine-1carboxylate (Compound No. 106a)
8-(1-Benzylpiperidin-4-yl)-6-(2-methylphenyl)-2-[(1-methylpiperidin-4-yl)amino]pyrido[2,3-*d*]pyrimidin-7(8H)-one (Compound No. 107a),
8-(1-Benzylpiperidin-4-yl)-2-(cyclobutylamino)-6-(2-methylphenyl)pyrido[2,3-*d*]pyrimidin-7(8H)-one (Compound No. 108a),
*tert*-Butyl (3S)-3-{[8-(1-benzylpiperidin-4-yl)-6-(2-methylphenyl)-7-oxo-7,8-dihydropyrido[2,3-*d*]pyrimidin-2-yl]amino}pyrrolidine-1-carboxylate (Compound No. 109a),
(3S)-3-{[8-(1-Benzylpiperidin-4-yl)-6-(2-methylphenyl)-7-oxo-7,8-dihydropyrido[2,3-*d*]pyrimidin-2-yl]amino}-*N*-isopropylpyrrolidine-1-carboxamide (Compound No. 110a),
Hydrochloride salt of 8-(1-benzylpiperidin-4-yl)-6-(2-methylphenyl)-2-(piperidin-4-ylamino)pyrido[2,3-*d*]pyrimidin-7(8H)-one (Compound No. 111a),
Hydrochloride salt of 6-(2-methylphenyl)-8-(1-methylpiperidin-4-yl)-2-(piperidin-4-ylamino)pyrido[2,3-*d*]pyrimidin-7(8H)-one (Compound No. 112a),
4-{[8-(1-Benzylpiperidin-4-yl)-6-(2-methylphenyl)-7-oxo-7,8-dihydropyrido[2,3-*d*]pyrimidin-2-yl]amino}-*N*-isopropylpiperidine-1-carboxamide (Compound No. 113a),
4-{[8-(1-Benzylpiperidin-4-yl)-6-(2-methylphenyl)-7-oxo-7,8-dihydropyrido[2,3-*d*]pyrimidin-2-yl]amino}-*N*-cyclohexylpiperidine-1-carboxamide (Compound No. 114a),
4-{[8-(1-Benzylpiperidin-4-yl)-6-(2-methylphenyl)-7-oxo-7,8-dihydropyrido[2,3-*d*]pyrimidin-2-yl]amino}-*N*-cyclopentylpiperidine-1-carboxamide (Compound No. 115a),
4-{[8-(1-Benzylpiperidin-4-yl)-6-(2-methylphenyl)-7-oxo-7,8-dihydropyrido[2,3-*d*]pyrimidin-2-yl]amino}-*N*-isobutylpiperidine-1-carboxamide (Compound No. 116a),
4-{[8-(1-Benzylpiperidin-4-yl)-6-(2-methylphenyl)-7-oxo-7,8-dihydropyrido[2,3-*d*]pyrimidin-2-yl]amino}-*N*-[(1R)-1-cyclohexylethyl]piperidine-1-carboxamide (Compound No. 117a),

*N*-Isopropyl-4-{[6-(2-methylphenyl)-8-(1-methylpiperidin-4-yl)-7-oxo-7,8-dihydropyrido[2,3-*d*]pyrimidin-2-yl]amino}piperidine-1-carboxamide (Compound No. 118a),
*N*-Cyclopropyl-4-{[6-(2-methylphenyl)-8-(1-methylpiperidin-4-yl)-7-oxo-7,8-dihydropyrido[2,3-*d*]pyrimidin-2-yl]amino} piperidine-1-carboxamide (Compound No. 119a),
8-(1-Benzylpiperidin-4-yl)-2-{[1-(cyclopentylacetyl)piperidin-4-yl]amino}-6-(2-methylphenyl)pyrido[2,3-*d*]pyrimidin-7 (8H)-one (Compound No. 120a),
8-(1-Benzylpiperidin-4-yl)-2-{[1-(cyclopropylcarbonyl)piperidin-4-yl]amino}-6-(2-methylphenyl)pyrido[2,3-*d*]pyrimidin-7 (8H)-one (Compound No. 121a),
8-(1- Benzylpiperidin- 4- yl)- 6-(2- methylphenyl)- 2- { [1-(phenylsulfonyl) piperidin- 4- yl] amino} pyrido [2,3-*d*] pyrimidin- 7 (8H)-one (Compound No. 122a),
8-(1- Benzylpiperidin- 4- yl)- 6-(2- methylphenyl)- 2- { [1-(methylsulfonyl) piperidin- 4- yl] amino} pyrido [2,3-*d*] pyrimidin- 7 (8H)-one (Compound No. 123a),
8-(1- Benzylpiperidin- 4- yl)- 2- { [1-(ethylsulfonyl) piperidin- 4- yl] amino}- 6-(2- methylphenyl) pyrido [2,3-*d*] pyrimidin- 7 (8H)-one (Compound no. 124a),
6-(2- Methylphenyl)- 8-(1- methylpiperidin- 4- yl)- 2- { [1-(methylsulfonyl) piperidin- 4- yl] amino} pyrido [2,3-*d*] pyrimidin- 7 (8H)-one (Compound No. 125a),
2- { [1-(Ethylsulfbnyl) piperidin- 4- yl] amino}- 6-(2- methylphenyl)- 8-(1- methylpiperidin- 4- yl) pyrido [2,3-*d*] pyrimidin- 7 (8H)-one (Compound No. 126a),
2-{[1-(Isopropylsulfonyl)piperidin-4-yl]amino}-6-(2-methylphenyl)-8-(1-methylpiperidin-4-yl)pyrido[2,3-*d*]pyrimidin-7 (8H)-one (Compound No. 127a),
6-(2-Methylphenyl)-8-(1-methylpiperidin-4-yl)-2-{[1-(pyrrolidin-1-ylcarbonyl)piperidin-4-yl]amino}pyrido[2,3-*d*]pyrimidin-7(8H)-one (Compound No. 128a),
2-[(1-Benzoylpiperidin-4-yl)amino]-6-(2-methylphenyl)-8-(1-methylpiperidin-4-yl)pyrido[2,3-*d*] pyrimidin-7(8H)-one (Compound No. 129a),
2-[(1-Benzoylpiperidin-4-yl)amino]-6-(2-methylphenyl)-8-(1-methylpiperidin-4-yl)pyrido[2,3-*d*] pyrimidin-7(8H)-one (Compound No. 130a),
*tert*-Butyl 4-{[6-(2-methylphenyl)-7-oxo-8-piperidin-4-yl-7,8-dihydropyrido[2,3-*d*]pyrimidin-2-yl]amino}piperidine-1-carboxylate (Compound No. 131 a),
6-(2-Methylphenyl)-8-piperidin-4-yl-2-(piperidin-4-ylamino)pyrido[2,3-*d*]pyrimidin-7(8H)-one (Compound No. 132a),
*N*-Isopropyl-4-{[6-(2-methylphenyl)-7-oxo-8-piperidin-4-yl-7,8-dihydropyrido[2,3-*d*]pyrimidin-2-yl]amino}piperidine-1-carboxamide (Compound No. 133a),
*tert*-Butyl 4-{[8-{1-[(isopropylamino)carbonyl]piperidin-4-yl}-6-(2-methylphenyl)-7-oxo-7,8-dihydropyrido[2,3-*d*]pyrimidin-2-yl]amino}piperidine-1-carboxylate (Compound No. 134a),
Hydrochloride salt of *N*-isopropyl-4-[6-(2-methylphenyl)-7-oxo-2-(piperidin-4-ylamino)pyrido[2,3-*d*]pyrimidin-8(7H)-yl] piperidine-1-carboxamide (Compound No. 135a),
*N*-Isopropyl-4-[2-({1-[(isopropylamino)carbonyl]piperidin-4-yl}amino)-6-(2-methylphenyl)-7-oxopyrido[2,3-*d*]pyrimidin-8 (7H)-yl]piperidine-1-carboxamide (Compound No. 136a),
4-[2-( {1-[(Cyclopropylamino) carbonyl] piperidin- 4- yl} amino)- 6-(2- methylphenyl)- 7- oxopyrido [2,3-*d*] pyrimidin- 8 (7H)-yl]-*N*-isopropylpiperidine-1-carboxamide (Compound No. 137a),
4-[2-( {1-[(*tert*-Butylamino) carbonyl] piperidin- 4- yl} amino)- 6-(2- methylphenyl)- 7- oxopyrido [2,3-*d*] pyrimidin- 8 (7H)-yl]-*N*-isopropylpiperidine-1-carboxamide (Compound No. 138a),
4-[2-( {1-[(Cyclohexylamino) carbonyl] piperidin- 4- yl} amino)- 6-(2- methylphenyl)- 7- oxopyrido [2,3-*d*] pyrimidin- 8 (7H)-yl]-*N*-isopropylpiperidine-1-carboxamide (Compound No. 139a),
*N*-Isopropyl-4-[6-(2-methylphenyl)-2-{[1-(morpholin-4-ylcarbonyl)piperidin-4-yl]amino}-7-oxopyrido[2,3-*d*]pyrimidin-8 (7H)-yl]piperidine-1-carboxamide (Compound No. 140a),
4-[2-{[1-(4-Fluorobenzoyl)piperidin-4-yl]amino}-6-(2-methylphenyl)-7-oxopyrido[2,3-*d*]pyrimidin-8(7H)-yl]-*N*-isopropylpiperidine-1-carboxamide (Compound No. 141a),
*N*-Isopropyl-4-[6-(2-methylphenyl)-2-{[1-(methylsulfonyl)piperidin-4-yl]amino}-7-oxopyrido[2,3-*d*]pyrimidin-8(7H)-yl]piperidine-1-carboxamide (Compound No. 142a),
4-[2-{[1-(Ethylsulfonyl)piperidin-4-yl]amino}-6-(2-methylphenyl)-7-oxopyrido[2,3-*d*]pyrimidin-8(7H)-yl]-*N*-isopropylpiperidine-1-carboxamide (Compound No. 143a),
*N*-Isopropyl-4-[2-{[1-(isopropylsulfonyl)piperidin-4-yl]amino}-6-(2-methylphenyl)-7-oxopyrido[2,3-*d*]pyrimidin-8(7H)-yl] piperidine-1-carboxamide (Compound No. 144a),
4-[2-{[1-(Cyclopropylcarbonyl)piperidin-4-yl]amino}-6-(2-methylphenyl)-7-oxopyrido[2,3-*d*]pyrimidin-8(7H)-yl]-*N*-isopropylpiperidine-1-carboxamide (Compound No. 145a),
4-[2-[(1-Benzoylpiperidin-4-yl)amino]-6-(2-methylphenyl)-7-oxopyrido[2,3-*d*]pyrimidin-8(7H)-yl]-*N*-isopropylpiperidine-1-carboxamide (Compound No. 146a),
4-[2-[(1-Acetylpiperidin-4-yl)amino]-6-(2-methylphenyl)-7-oxopyrido[2,3-*d*]pyrimidin-8(7H)-yl]-*N*-isopropylpiperidine-1-carboxamide (Compound No. 147a),
*N*-Isopropyl-4-[6-(2-methylphenyl)-2-[(1-methylpiperidin-4-yl)amino]-7-oxopyrido[2,3-*d*]pyrimidin-8(7H)-yl]piperidine-1-carboxamide (Compound No. 148a),
4-[2-[(1-Benzylpiperidin-4-yl)amino]-6-(2-methylphenyl)-7-oxopyrido[2,3-*d*]pyrimidin-8(7H)-yl]-*N*-isopropylpiperidine-1-carboxamide (Compound No. 149a),
8-(1-Acetylpiperidin-4-yl)-2-[(1-acetylpiperidin-4-yl)amino]-6-(2-methylphenyl)pyrido[2,3-*d*]pyrimidin-7(8H)-one (Compound No. 150a),
4-[2-{[1-(4-Fluorobenzoyl)piperidin-4-yl]amino}-6-(2-methylphenyl)-7-oxopyrido[2,3-*d*]pyrimidin-8(7H)-yl]-*N*-isopropylpiperidine-1-carboxamide (Compound No. 151 a),
*tert*-Butyl 4-({6-(2-methylphenyl)-8-[1-(methylsulfonyl)piperidin-4-yl]-7-oxo-7,8-dihydropyrido[2,3-*d*]pyrimidin-2-yl}amino)piperidine-1-carboxylate (Compound No. 152a),
Hydrochloride salt of 6-(2-methylphenyl)-8-[1-(methylsulfonyl)piperidin-4-yl]-2-(piperidin-4-ylamino)pyrido[2,3-*d*]pyrimidin-7(8H)-one (Compound No. 153a),
*N*-Isopropyl-4-({6-(2-methylphenyl)-8-[1-(methylsulfbnyl)piperidin-4-yl]-7-oxo-7,8-dihydropyrido[2,3-*d*]pyrimidin-2-yl}amino)piperidine-1-carboxamide (Compound No. 154a),
*N*-(*tert*-Butyl)-4-({6-(2-methylphenyl)-8-[1-(methylsulfonyl)piperidin-4-yl]-7-oxo-7,8-dihydropyrido[2,3-*d*]pyrimidin-2-yl}amino)piperidine-1-carboxamide (Compound No. 155a),
*N*-Cyclohexyl-4-({6-(2-methylphenyl)-8-[1-(methylsulfonyl)piperidin-4-yl]-7-oxo-7,8-dihydropyrido[2,3-*d*]pyrimidin-2-yl}amino)piperidine-1-carboxamide (Compound No. 156a),
*N*-(4-Fluorophenyl)-4-({6-(2-methylphenyl)-8-[1-(methylsulfonyl)piperidin-4-yl]-7-oxo-7,8-dihydropyrido[2,3-*d*]pyrimidin-2-yl}amino)piperidine-1-carboxamide (Compound No. 157a),
6-(2-Methylphenyl)-8-[1-(methylsulfonyl)piperidin-4-yl]-2-{[1-(morpholin-4-ylcarbonyl)piperidin-4-yl]amino}pyrido[2,3-*d*]pyrimidin-7(8H)-one (Compound No.158a),
6-(2-Methylphenyl)-8-[1-(methylsulfonyl)piperidin-4-yl]-2-{[1-(methylsulfonyl)piperidin-4-yl]amino}pyrido[2,3-*d*]pyrimidin-7(8H)-one (Compound No. 159a),
2-{[1-(Ethylsulfonyl)piperidin-4-yl]amino}-6-(2-methylphenyl)-8-[1-(methylsulfonyl)piperidin-4-yl]pyrido[2,3-*d*]pyrimidin-7(8H)-one (Compound No. 160a),
2-{[1-(Isopropylsulfonyl)piperidin-4-yl]amino}-6-(2-methylphenyl)-8-[1-(methylsulfonyl)piperidin-4-yl]pyrido[2,3-*d*]pyrimidin-7(8H)-one (Compound No. 161a),
2-{[1-(Cyclopropylcarbonyl)piperidin-4-yl]amino}-6-(2-methylphenyl)-8-[1-(methylsulfonyl)piperidin-4-yl]pyrido[2,3-*d*]pyrimidin-7(8H)-one (Compound 162a),
2-[(1- Acetylpiperidin- 4- yl) amino]- 6-(2- methylphenyl)- 8-[1-(methylsulfonyl) piperidin- 4- yl] pyrido [2,3-*d*] pyrimidin- 7 (8H)-one (Compound No. 163a),
2-[(1- Benzoylpiperidin- 4- yl) amino]- 6-(2- methylphenyl)- 8-[1-(methylsulfonyl) piperidin- 4- yl] pyrido [2,3-*d*] pyrimidin- 7 (8H)-one (Compound No. 164a),
2-{[1-(4-Fluorobenzoyl)piperidin-4-yl]amino}-6-(2-methylphenyl)-8-[1-(methylsulfonyl)piperidin-4-yl]pyrido[2,3-*d*]pyrimidin-7(8H)-one (Compound No. 165a),
*tert*-Butyl 4-{[8-(1-acetylpiperidin-4-yl)-6-(2-methylphenyl)-7-oxo-7,8-dihydropyrido[2,3-*d*]pyrimidin-2-yl]amino}pipendine-1-carboxylate (Compound No. 166a),
8-(1-Acetylpiperidin-4-yl)-6-(2-methylphenyl)-2-(piperidin-4-ylamino)pyrido[2,3-*d*]pyrimidin-7(8H)-one (Compound No. 167a),
4-{[8-(1-Acetylpiperidin-4-yl)-6-(2-methylphenyl)-7-oxo-7,8-dihydropyrido[2,3-d]pyrimidin-2-yl]amino}-*N*-isopropylpiperidine-1-carboxamide (Compound No. 168a),
4-{[8-(1-Acetylpiperidin-4-yl)-6-(2-methylphenyl)-7-oxo-7,8-dihydropyrido[2,3-*d*]pyrimidin-2-yl]amino}-*N*-cyclopropylpiperidine-1-carboxamide (Compound No. 169a),
4-{[8-(1-Acetylpiperidin-4-yl)-6-(2-methylphenyl)-7-oxo-7,8-dihydropyrido[2,3-*d*]pyrimidin-2-yl]amino}-*N*-(*tert*-butyl)piperidine-1-carboxamide (Compound No. 170a),
4-{[8-(1-Acetylpiperidin-4-yl)-6-(2-methylphenyl)-7-oxo-7,8-dihydropyrido[2,3-*d*]pyrimidin-2-yl]amino}-*N*-cyclohexylpiperidine-1-carboxamide (Compound No. 171a),
4-{[8-(1-Acetylpiperidin-4-yl)-6-(2-methylphenyl)-7-oxo-7,8-dihydropyrido[2,3-*d*]pyrimidin-2-yl]amino}-*N*-(4-fluorophenyl)piperidine-1-carboxamide (Compound No. 172a),
8-(1-Acetylpiperidin-4-yl)-6-(2-methylphenyl)-2-{[1-(morpholin-4-ylcarbonyl)piperidin-4-yl]amino}pyrido[2,3-*d*]pyrimidin-7(8H)-one (Compound No. 173a),
8-(1- Acetylpiperidin- 4- yl)- 6-(2- methylphenyl)- 2- { [1-(methylsulfonyl) piperidin- 4- yl] amino} pyrido [2,3-*d*] pyrimidin- 7 (8H)-one (Compound No. 174a),
8-(1-Acetylpiperidin-4-yl)-2-{[1-(ethylsulfonyl)piperidin-4-yl]amino}-6-(2-methylphenyl)pyrido[2,3-*d*]pyrimidin-7(8H)-one (Compound No. 175a),
8-(1-Acetylpiperidin-4-yl)-2-{[1-(isopropylsulfonyl)piperidin-4-yl]amino}-6-(2-methylphenyl)pyrido[2,3-*d*]pyrimidin-7(8H)-one (Compound No. 176a),
8-(1-Acetylpiperidin-4-yl)-2-{[1-(cyclopropylcarbonyl)piperidin-4-yl]amino}-6-(2-methylphenyl)pyrido[2,3-*d*]pyrimidin-7(8H)-one (ompound No. 177a),
8-(1- Acetylpiperidin- 4- yl)- 2- { [1-(4- fluorobenzoyl) piperidin- 4- yl] amino}- 6-(2- methylphenyl) pyrido [2,3-*d*] pyrimidin- 7(8H)-one (Compound No. 178a),
*tert*-Butyl 4-{[8-[(3S)-1-acetylpyrrolidin-3-yl]-6-(2-methylphenyl)-7-oxo-7,8-dihydropyrido[2,3-*d*]pyrimidin-2-yl]amino}pipendine-1-carboxylate (Compound No. 179a),
Hydrochloride salt of 8-[(3S)-1-acetylpyrrolidin-3-yl]-6-(2-methylphenyl)-2-(piperidin-4-ylamino)pyrido[2,3-*d*]pyrimidin-7(8H)-one (Compound No. 180a),
8-[(3S)-1-Acetylpyrrolidin-3-yl]-6-(2-methylphenyl)-2-{[1-(methylsulfonyl)piperidin-4-yl]amino}pyrido[2,3-*d*]pyrimidin-7(8H)-one (Compound No. 181a),
2-[(1-Acetylpiperidin-4-yl)amino]-6-(2-methylphenyl)-8-(1-methylpiperidin-4-yl)pyrido[2,3-*d*]pyrimidin-7(8*H*)-one (Compound No. 182a),
Hydrochloride salt of 6-(2-methylphenyl)-8-[(3S)-1-(methylsulfonyl)pyrrolidin-3-yl]-2-(piperidin-4-ylamino)pyrido[2,3-*d*]pyrimidin-7(8H)-one (Compound No. 183a),
*tert*-Butyl 4-{[8-[(3R)-1-acetylpyrrolidin-3-yl]-6-(2-methylphenyl)-7-oxo-7,8-dihydropyrido[2,3-*d*]pyrimidin-2-yl]amino}piperidine-1-carboxylate (Compound No. 184a),
Hydrochloride salt of 6-(2-methylphenyl)-2-[(3S)-pyrrolidin-3-ylamino]-8-(tetrahydrofuran-3-yl)pyrido[2,3-*d*]pyrimidin-7(8H)-one (Compound No. 185a),
Hydrochloride salt of 6-(2-methylphenyl)-2-[(3S)-piperidin-3-ylamino]-8-[(3R)-tetrahydrofuran-3-yl]pyrido[2,3-*d*]pyrimidin-7(8H)-one (Compound No. 186a),
Hydrochloride salt of 6-(2-methylphenyl)-2-[(3S)-piperidin-3-ylamino]-8-[(3S)-tetrahydrofuran-3-yl]pyrido[2,3-*d*]pyrimidin-7(8H)-one (Compound No. 187a),
Hydrochloride salt of 6-(2-methylphenyl)-2-[(3R)-piperidin-3-ylamino]-8-[(3R)-tetrahydrofuran-3-yl]pyrido[2,3-*d*]pyrimidin-7(8H)-one (Compound No. 188a),
4-[2-[(1-{[(4- Fluorophenyl) amino] carbonyl} piperidin- 4- yl) amino]- 6-(2- methylphenyl)- 7- oxopyrido [2,3-*d*] pyrimidin- 8(7H)-yl]-*N*-isopropylpiperidine-1-carboxamide (Compound No. 189a),
4- {[8- {1-[(Isopropylamino) carbonyl] piperidin- 4- yl}- 6-(2- methylphenyl)- 7- oxo- 7,8- dihydropyrido [2,3-*d*] pyrimidin- 2- yl] amino}-*N*-morpholin-4-ylpiperidine-1-carboxamide (Compound No. 190a),
4-[2-{[1-(2,2-Dimethylpropanoyl)piperidin-4-yl]amino}-6-(2-methylphenyl)-7-oxopyrido[2,3-*d*]pyrimidin-8(7H)-yl]-*N*-isopropylpiperidine-1-carboxamide (Compound No. 191a),
8-[(3S)-1-Acetylpyrrolidin-3-yl]-2-[(1-benzylpiperidin-4-yl)amino]-6-(2-methylphenyl)pyrido[2,3-*d*]pyrimidin-7(8H)-one (Compound No. 192a),
*N*-Cyclopropyl-4-({6-(2-methylphenyl)-8-[1-(methylsulfonyl)piperidin-4-yl]-7-oxo-7,8-dihydropyrido[2,3-*d*]pyrimidin-2-yl}amino)piperidine-1-carboxamide (Compound No. 193a),
4-({6-(2-Methylphenyl)-8-[1-(methylsulfonyl)piperidin-4-yl]-7-oxo-7,8-dihydropyrido[2,3-*d*]pyrimidin-2-yl}amino)-*N*-morpholin-4-ylpiperidine-1-carboxamide (Compound No. 194a),
2-{[1-(2,2-Dimethylpropanoyl)piperidin-4-yl]amino}-6-(2-methylphenyl)-8-[1-(methylsulfonyl)piperidin-4-yl]pyrido[2,3-*d*]pyrimidin-7(8H)-one (Compound No. 195a),
6-(2- Methylphenyl)- 2-[(1- methylpiperidin- 4- yl) amino]- 8-[1-(methylsulfonyl) piperidin- 4- yl] pyrido [2,3-*d*] pyrimidin- 7(8H)-one (Compound No. 196a),
2-[(1- Benzylpiperidin- 4- yl) amino]- 6-(2- methylphenyl)- 8-[1-(methylsulfonyl) piperidin- 4- yl] pyrido [2,3-*d*] pyrimidin- 7(8H)-one (Compound No. 197a),
8-(1- Acetylpiperidin- 4- yl)- 2-[(1- benzoylpiperidin- 4- yl) amino]- 6-(2- methylphenyl) pyrido [2,3-*d*] pyrimidin- 7 (8H)-one (Compound No. 198a),
8-(1-Acetylpiperidin-4-yl)-2-{[1-(2,2-dimethylpropanoyl)piperidin-4-yl]amino}-6-(2-methylphenyl)pyrido[2,3-*d*]pyrimidin-7(8H)-one (Compound No. 199a),
8-(1-Acetylpiperidin-4-yl)-6-(2-methylphenyl)-2-[(1-methylpiperidin-4-yl)amino]pyrido[2,3-*d*]pyrimidin-7(8H)-one (Compound No. 200a),
8-(1-Acetylpiperidin-4-yl)-2-[(1-benzylpiperidin-4-yl)amino]-6-(2-methylphenyl)pyrido[2,3-*d*]pyrimidin-7(8H)-one (Compound No. 201a),
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-cyclopropylpiperidine-1-carboxamide (Compound No. 202a),
4- { [8-[(3S)- 1- Acetylpyrrolidin- 3- yl]- 6-(2- methylphenyl)- 7- oxo- 7,8- dihydropyrido [2,3-*d*] pyrimidin- 2- yl] amino}-*N*-(*tert*-butyl)piperidine-1-carboxamide (Compound No. 203a),
4- { [8-[(3S)- 1- Acetylpyrrolidin- 3- yl]- 6-(2- methylphenyl)- 7- oxo- 7,8- dihydropyrido [2,3-*d*] pyrimidin- 2- yl] amino}-*N*-cyclohexylpiperidine-1-carboxamide (Compound No. 204a),
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-(4-fluorophenyl)piperidine-1-carboxamide (Compound No. 205a),
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-morpholin-4-ylpiperidine-1-carboxamide (Compound No. 206a),
8-[(3S)-1-Acetylpyrrolidin-3-yl]-2-{[-(ethylsulfonyl)piperidin-4-yl]amino}-6-(2-methylphenyl)pyrido[2,3-*d*]pyrimidin-7(8H)-one (Compound No. 207a),
8-[(3S)-1-Acetylpyrrolidin-3-yl]-6-(2-methylphenyl)-2-{[1-(propylsulfonyl)piperidin-4-yl]amino}pyrido[2,3-*d*]pyrimidin-7(8H)-one ((Compound No. 208a),
8-[(3R)-1-Acetylpyrrolidin-3-yl]-2-{[1-(cyclopropylcarbonyl)piperidin-4-yl]amino}-6-(2-methylphenyl)pyrido[2,3-*d*]pyrimidin-7(8H)-one (Compound No. 209a),
2-[(1-Acetylpiperidin-4-yl)amino]-8-[(3R)-1-acetylpyrrolidin-3-yl]-6-(2-methylphenyl)pyrido[2,3-*d*]pyrimidin-7(8H)-one (Compound No. 210a),
8-[(3R)-1-Acetylpyrrolidin-3-yl]-2-[(1-benzoylpiperidin-4-yl)amino]-6-(2-methylphenyl)pyrido[2,3-*d*]pyrimidin-7(8H)-one (Compound No. 211a),
8-[(3R)-1-Acetylpyrrolidin-3-yl]-2-{[1-(4-fluorobenzoyl)piperidin-4-yl]amino}-6-(2-methylphenyl)pyrido[2,3-*d*]pyrimidin-7(8H)-one (Compound No. 212a), 8-[(3R)-1-Acetylpyrrolidin-3-yl]-2-{[1-(2,2-dimethylpropanoyl)piperidin-4-yl]amino}-6-(2-methylphenyl)pyrido[2,3-*d*]pyrimidin-7(8H)-one (Compound No. 213a),
8-[(3R)-1-Acetylpyrrolidin-3-yl]-6-(2-methylphenyl)-2-[(1-methylpiperidin-4-yl)amino]pyrido[2,3-*d*]pyrimidin-7(8H)-one (Compound No. 214a),
8-[(3R)-1-Acetylpyrrolidin-3-yl]-2-[(1-benzylpiperidin-4-yl)amino]-6-(2-methylphenyl)pyrido[2,3-*d*]pyrimidin-7(8H)-one (Compound No. 215a),
*tert*-Butyl 4-{[8-[(3S)-1-acetylpyrrolidin-3-yl]-6-(2-methylphenyl)-7-oxo-7,8-dihydropyrido[2,3-*d*]pyrimidin-2-yl]amino}piperidine-1-carboxylate (Compound No. 216a),
8-[(3S)-1-Acetylpyrrolidin-3-yl]-6-(2-methylphenyl)-2-[(1-methylpiperidin-4-yl)amino]pyrido[2,3-*d*]pyrimidin-7(8H)-one (Compound No. 217a),
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-isopropylpiperidine-1-carboxamide (Compound No. 218a),
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-cyclopropylpiperidine-1-carboxamide (Compound No. 219a),
4- { [8-[(3S)- 1- Acetylpyrrolidin- 3- yl]- 6-(2- methylphenyl)- 7- oxo- 7,8- dihydropyrido [2,3-*d*] pyrimidin- 2- yl] amino}-*N*-(*tert*-butyl)piperidine-1-carboxamide (Compound No. 220a),
4- { [8-[(3S)- 1- Acetylpyrrolidin- 3- yl]- 6-(2- methylphenyl)- 7- oxo- 7,8- dihydropyrido [2,3-*d*] pyrimidin- 2- yl] amino}-*N*-cyclohexylpiperidine-1-carboxamide (Compound No. 22 1 a)
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-(4-fluorophenyl)piperidine-1-carboxamide (Compound No. 222a),
4-{[8-[(3S)-1-Acetylpyrrolidin-3-yl]-6-(2-methylphenyl)-7-oxo-7,8-dihydropyrido[2,3-*d*]pyrimidin-2-yl]amino}-*N*-morpholin-4-ylpiperidine-1-carboxamide (Compound No. 223a),
8-[(3S)-1-Acetylpyrrolidin-3-yl]-6-(2-methylphenyl)-2-{[1-(methylsulfonyl)piperidin-4-yl]amino}pyrido[2,3-*d*]pyrimidin-7(8H)-one (Compound No. 224a),
8-[(3S)-1-Acetylpyrrolidin-3-yl]-2-{[-(ethylsulfonyl)piperidin-4-yl]amino}-6-(2-methylphenyl)pyrido[2,3-*d*]pyrimidin-7(8H)-one (Compound No. 225a),
8-[(3S)-1-Acetylpyrrolidin-3-yl]-6-(2-methylphenyl)-2-{[1-(propylsulfonyl)piperidin-4-yl]amino}pyrido[2,3-*d*]pyrimidin-7(8H)-one (Compound No. 226a),
8-[(3S)-1-Acetylpyrrolidin-3-yl]-2-{[-(cyclopropylcarbonyl)piperidin-4-yl]amino}-6-(2-methylphenyl)pyrido[2,3-*d*]pyrimidin-7(8H)-one (Compound No. 227a),
2-[(1-Acetylpiperidin-4-yl)amino]-8-[(3S)-1-acetylpyrrolidin-3-yl]-6-(2-methylphenyl)pyrido[2,3-*d*]pyrimidin-7(8H)-one (Compound No. 228a),
8-[(3S)-1-Acetylpyrrolidin-3-yl]-2-[(1-benzoylpiperidin-4-yl)amino]-6-(2-methylphenyl)pyrido[2,3-*d*]pyrimidin-7(8H)-one (Compound No. 229a),
8-[(3S)-1-Acetylpyrrolidin-3-yl]-2-{[-(4-fluorobenzoyl)piperidin-4-yl]amino}-6-(2-methylphenyl)pyrido[2,3-*d*]pyrimidin-7(8H)-one (Compound No. 230a),
8-[(3S)-1-Acetylpyrrolidin-3-yl]-2-{[1-(2,2-dimethylpropanoyl)piperidin-4-yl]amino}-6-(2-methylphenyl)pyrido[2,3-*d*]pyrimidin-7(8H)-one (Compound No. 231a) disclosed in our copending patent application No. 2602/DEL/2005;
and the compounds which are disclosed in United States Patent Application No. 60/598621, 60/630,517, 1098/DEL/2005 and 211/DEL/2005. The p38 MAP Kinase inhibitors can also be selected from compounds not limited to those described in WO98/47892, WO00/43384, and WO98/27098.

Examples of p38 MAP Kinase inhibitors include, but are not limited to, Vx-745, as disclosed in WO 98/27098, BIRB-796, as disclosed in WO 00/43384, RWJ-67657, as disclosed in WO 98/47892, and SB - 239063, as disclosed in WO 97/25048. Any reference to the above mentioned p38 kinase inhibitors also include any pharmacologically acceptable

acid addition salts thereof which may exist. By the physiologically or pharmacologically acceptable acid addition salts thereof which may be formed by the p38 kinase inhibitors are meant, according to the invention, pharmaceutically acceptable salts selected from among the salts of hydrochloric acid, hydrobromic acid, sulfuric acid, phosphoric acid, methanesulfonic acid, acetic acid, fumaric acid, succinic acid, lactic acid, citric acid, tartaric acid, and maleic acid.

The β2-agonists may be chosen from those described in the art or subsequently discovered. The β2-agonists may include, for example, one or more compounds described in U.S. Patent Nos. 3,705,233; 3,644,353; 3,642,896; 3,700,681; 4,579,985; 3,994,974; 3,937,838; 4,419,364; 5,126,375; 5,243,076; 4,992,474; or 4,011,258.

Suitable β2-agonists include, for example, one or more of albuterol, salbutamol, biltolterol, pirbuterol, levosalbutamol, tulobuterol, terbutaline, bambuterol, metaproterenol, fenoterol, salmeterol, carmoterol, arformoterol, formoterol, and their pharmaceutically acceptable salts or solvates thereof.

Suitable corticosteroids may be chosen from those described in the art. Suitable corticosteroids may include , for example, one or more compounds described in U.S. Patent Nos. 3,312,590; 3,983,233; 3,929,768; 3,721,687; 3,436,389; 3,506,694; 3,639,434; 3,992,534; 3,928,326; 3,980,778; 3,780,177; 3,652,554; 3,947,478; 4,076,708; 4,124,707; 4,158,055; 4,298,604; 4,335,121; 4,081,541; 4,226,862; 4,290,962; 4,587,236; 4,472,392; 4,472,393; 4,242,334; 4,014,909; 4,098,803; 4,619,921; 5,482,934; 5,837,699; 5,889,015; 5,278,156; 5,015,746; 5,976,573; 6,337,324; 6,057,307; 6,723,713; 6,127,353; or 6,180,781.

Suitable corticosteroids may include, for example, one or more of alclometasone, amcinonide, amelometasone, beclometasone, betamethasone, budesonide, ciclesonide, clobetasol, cloticasone, cyclomethasone, deflazacort, deprodone, dexbudesonide, diflorasone, difluprednate, fluticasone, flunisolide, halometasone, halopredone, hydrocortisone, hydrocortisone, methylprednisolone, mometasone, prednicarbate, prednisolone, rimexolone, tixocortol, triamcinolone, tolterodine, oxybutynin, ulobetasol, rofleponide, KSR 592, as disclosed in US Patent 4,285,937, ST-126, as disclosed in EP 1344526, dexamethasone and pharmaceutically acceptable salts, solvates thereof. Preferred corticosteroids include, for example, flunisolide, beclomethasone, triamcinolone, budesonide, fluticasone, mometasone, ciclesonide, and dexamethasone. Examples of possible salts or derivatives include: sodium salts, sulfobenzoates, phosphates, isonicotinates, acetates, propionates, dihydrogen phosphates, palmitates, pivalates, or furoates. In some cases, the corticosteroids may also occur in the form of their hydrates.

Suitable muscarinic receptor antagonists (MRA) include substances that directly or indirectly block activation of muscarinic cholinergic receptors. Examples include, but are not limited to, quaternary amines (*e.g.*, tiotropium salts, methantheline, ipratropium, propantheline), tertiary amines (*e.g.*, dicyclomine, scopolamine) and tricyclic amines (*e.g.*, telenzepine). Other suitable muscarinic receptor antagonists include benztropine (commercially available as COGENTIN from Merck), hexahydro-sila-difenidol hydrochloride (HHSID hydrochloride disclosed in Lambrecht et al., Trends in Pharmacol. Sci., 10 (Suppl):60 (1989); (+/-)-3-quinuclidinyl xanthene-9-carboxylate hemioxalate (QNX-hemioxalate; Birdsall et al., Trends in Pharmacol. Sci., 4:459 (1983); telenzepine dihydrochloride (Coruzzi et al., Arch. Int. Pharmacodyn. Ther., 302:232 (1989); and Kawashima et al., Gen. Pharmacol., 21:17 (1990)), and atropine.

Other muscarinic receptor antagonists can be selected from for example,
2-Cyclopentyl-2-hydroxy-*N*-[2-(1*H*-imidazo1-1-yl)ethyl]-2-phenylacetamide (Compound No. 1aa);
1*H*-Imidazol-1-ylmethyl cyclohexyl(hydroxy)(4-methylphenyl)acetate (Compound No. 2aa);
2-(4-Fluorophenyl)-2-hydroxy-*N*-[2-(1*H*-imidazo1-1-yl)ethyl]-2-phenylacetamide (Compound No. 3aa);
2-Cyclobutyl-2-hydroxy-*N*-[2-(1*H*-imidazol-1-yl)ethyl]-2-phenylacetamide (Compound No. 4aa);
2-Cyclopentyl-2-(4-fluorophenyl)-2-hydroxy-*N*-[2-(1*H*-imidazol-1-yl)ethyl]acetamide (Compound No. 5aa);
2-Hydroxy-*N*-[2-(1*H*-imidazo1-1-yl)ethyl]-2,2-diphenylacetamide (Compound No. 6aa);
2-Cyclohexyl-2-hydroxy-*N*-[2-(1*H*-imidazol-1-yl)ethyl]-2-phenylacetamide (Compound No. 7aa);
2-Cyclopentyl-2-hydroxy-*N*-[2-(2-isopropyl-1*H*-imidazol-1-yl)ethyl]-2-phenylacetamide (Compound No. 8aa);
2-Cyclohexyl-2-hydroxy-*N*-[2-(2-isopropyl-1*H*-imidazol-1-yl)ethyl]-2-phenylacetamide (Compound No. 9aa);
2-Hydroxy-*N*-[2-(2-isopropyl-1*H*-imidazol-1-yl)ethyl]-2-phenyl-2-pyridin-3-ylacetamide (Compound No. 10aa);
2-(4-Fluorophenyl)-2-hydroxy-*N*-[2-(2-isopropyl-1*H*-imidazol-1-yl)ethyl]-2-phenylacetamide (Compound No. 11 aa);
2-Hydroxy-*N*-[2-(2-isopropyl-1*H*-imidazol-1-yl)ethyl]-2,2-diphenylacetamide (Compound No. 12 aa);
2-(2-Methyl-1*H*-imidazol-1-yl)ethyl cyclopentyl(hydroxy)phenylacetate (Compound No. 13 aa);
2-(2-Methyl-1*H*-imidazol-1-yl) ethyl (2*R*)-cyclopentyl(hydroxy)phenylacetate (Compound No. 14 aa);
1-Cyclopentyl-1-hydroxy-1-(4-methoxyphenyl)-3-(2-methyl-1*H*-imidazol-1-yl)acetone (Compound No. 15 aa);
(2*R*)-2-cyclopentyl-2-hydroxy-*N*-[2-(2-methyl-1*H*-imidazol-1-yl)ethyl]-2-phenylacetamide (Compound No. 16 aa);
2-Hydroxy-*N*-[2-(1*H*-imidazol-1-yl)ethyl]-2-phenyl-2-pyridin-3-ylacetamide (Compound No. 17 aa);
2-Cyclopentyl-2-hydroxy-*N*-[2-(2-methyl-1*H*-imidazol-1-yl)ethyl]-2-phenylacetamide (Compound No. 18 aa);
2-Hydroxy-*N*-[2-(2-methyl-1*H*-imidazol-1-yl)ethyl]-2-phenyl-2-pyridin-3-ylacetamide (Compound No. 19 aa);
2-Cyclohexyl-2-hydroxy-*N*-[2-(2-methyl-1*H*-imidazol-1-yl)ethyl]-2-phenylacetamide (Compound No. 20 aa);
2-(4-Fluorophenyl)-2-hydroxy-*N*-[2-(2-methyl-1*H*-imidazol-1-yl)ethyl]-2-phenylacetamide (Compound No. 21 aa);
2-Cyclopentyl-2-(4-fluorophenyl)-2-hydroxy-*N*-[2-(2-methyl-1*H*-imidazol-1-yl)ethyl]acetamide (Compound No. 22 aa);
2-Cyclobutyl-2-hydroxy-*N*-[2-(2-methyl-1*H*-imidazol-1-yl)ethyl]-2-phenylacetamide (Compound No. 23 aa);

2-Hydroxy-*N*-[2-(2-methyl-1*H*-imidazol-1-yl)ethyl]-2,2-diphenylacetamide (Compound No. 24 aa);
3,3,3-Trifluoro-2-hydroxy-*N*-[2-(2-methyl-1*H*-imidazol-1-yl)ethyl]-2-(4-methylphenyl)propanamide (Compound No. 25 aa);
*N*-[2-(2-methyl-1*H*-imidazol-1-yl)ethyl]-2,2-diphenylacetamide (Compound No. 26 aa);
2-Cyclopentyl-*N*-[2-(2-methyl-1*H*-imidazol-1-yl)ethyl]-2-phenylacetamide (Compound No. 27 aa);
2-Cyclopentyl-2-hydroxy-*N*-[2-(2-methyl-1*H*-imidazol-1-yl)ethyl]-2-(4-methylphenyl)acetamide (Compound No. 28 aa);
2-Cyclohexyl-2-hydroxy-*N*-[2-(2-methyl-1*H*-imidazol-1-yl)ethyl]-2-(4-methylphenyl)acetamide (Compound No. 29 aa);
2-Hydroxy-*N*-[2-(2-methyl-1*H*-imidazol-1-yl)ethyl]-2-(4-methylphenyl)-2-phenylacetamide (Compound No. 30 aa);
2-Cyclopentyl-2-hydroxy-*N*-[3-(1*H*-imidazol-1-yl)propyl]-2-phenylacetamide (Compound No. 31 aa);
2-Cyclohexyl-2-hydroxy-*N*-[3-(1*H*-imidazol-1-yl)propyl]-2-phenylacetamide (Compound No. 32 aa);
2-Cyclopentyl-2-hydroxy-*N*-[3-(2-methyl-1*H*-imidazol-1-yl)propyl]-2-phenylacetamide (Compound No. 33 aa);
2-Cyclohexyl-2-hydroxy-*N*-[3-(2-methyl-1*H*-imidazol-1-yl)propyl]-2-phenylacetamide (Compound No. 34 aa);
(2R)-2-(3,3-Difluorocyclopentyl)-2-hydroxy-*N*-[3-(2-methyl-1*H*-imidazol-1-yl)propyl]-2-phenylacetamide (Compound No. 35 aa);
2-Cyclopentyl-2-hydroxy-*N*-methyl-*N*-[3-(2-methyl-1*H*-imidazol-1-yl)propyl]-2-phenylacetamide (Compound No. 36 aa);
2-Cyclohexyl-2-hydroxy-*N*-methyl-*N*-[3-(2-methyl-1*H*-imidazol-1-yl)propyl]-2-phenylacetamide (Compound No. 37 aa);
2-Cyclopentyl-2-hydroxy-*N*-[2-(1*H*-imidazol-1-yl)ethyl]-*N*-methyl-2-phenylacetamide (Compound No. 38 aa);
2-Cyclopentyl-2-hydroxy-*N*-[2-(1*H*-imidazo1-4-yl)ethyl]-2-phenylacetamide (Compound No. 39 aa);
*N*-[2-(1-benzyl-1*H*-imidazol-4-yl)ethyl]-2-cyclopentyl-2-hydroxy-2-(4-methylphenyl)acetamide (Compound No. 40 aa);
2-Cyclopentyl-2-hydroxy-*N*-[2-(1*H*-imidazol-4-yl)ethyl]-2-(4-methylphenyl)acetamide (Compound No. 41 aa);
2-Cyclohexyl-2-hydroxy-*N*-[2-(1*H*-imidazol-4-yl)ethyl]-2-(4-methylphenyl)acetamide (Compound No. 42 aa);
2-Hydroxy-*N*-[2-(1*H*-imidazol-4-yl)ethyl]-2,2-diphenylacetamide (Compound No. 43 aa);
*N*-[2-(1-benzyl-1*H*-imidazol-4-yl)ethyl]-2-cyclohexyl-2-hydroxy-2-(4-methylphenyl)acetamide (Compound No. 44 aa);
*N*-[2-(1-benzyl-1*H*-imidazol-4-yl)ethyl]-2-hydroxy-2,2-diphenylacetamide (Compound No. 45 aa);
1*H*-imidazol-1-ylmethyl cyclopentyl(hydroxy)phenylacetate (Compound No. 46 aa);
1*H*-imidazol-1-ylmethyl cyclohexyl(hydroxy)phenylacetate (Compound No. 47 aa);
1*H*-imidazol-1-ylmethyl (2R)-cyclopentyl(hydroxy)phenylacetate (Compound No. 48 aa);
1*H*-Imidazol-1-ylmethyl cyclopentyl(hydroxy)(4-methoxyphenyl)acetate (Compound No. 49 aa);
1-Cyclohexyl-1-hydroxy-3-(1*H*-imidazol-1-yl)-1-phenylacetone (Compound No. 50 aa);
1-Cyclohexyl-1-hydroxy-3-(2-methyl-1*H*-imidazol-1-yl)-1-phenylacetone (Compound No. 51 aa);
1-Cyclopentyl-1-hydroxy-3-(2-isopropyl-1*H*-imidazol-1-yl)-1-(4-methoxyphenyl)acetone (Compound No. 52 aa);
1-Cyclohexyl-1-hydroxy-3-(2-isopropyl-1*H*-imidazol-1-yl)-1-phenylacetone (Compound No. 53 aa);
1-Cyclohexyl-1-hydroxy-3-(2-methyl-4,5-dihydro-1*H*-imidazol-1-yl)-1-phenylacetone (Compound No. 54 aa);
1-Cyclopentyl-1-hydroxy-1-(4-methoxyphenyl)-3-(2-methyl-4,5-dihydro-1*H*-imidazol-1-yl)acetone (Compound No. 55 aa);
1-Cyclopentyl-1-hydroxy-3-(1*H*-imidazol-1-yl)-1-(4-methoxyphenyl)acetone (Compound No. 56 aa);
2-Cyclohexyl-2-hydroxy-*N*-[2-(1*H*-imidazol-1-yl)ethyl]-*N*-methyl-2-phenylacetamide (Compound No. 57 aa) ;
2-Cyclopentyl-2-hydroxy-*N*-[2-(1*H*-imidazol-1-yl)ethyl]-*N*-methyl-2-phenylacetamide (Compound No. 58 aa);
2-(2-Methyl-1*H*-imidazol-1-yl)ethyl cyclopentyl(phenyl)acetate (Compound No. 59 aa);
2-(2-Methyl-1*H*-imidazol-1-yl)ethyl cyclohexyl(hydroxy)phenylacetate (Compound No. 60 aa);
3-(2-Methyl-1*H*-imidazol-1-yl)propyl cyclopentyl(hydroxy) phenylacetate (Compound No. 61 aa);
3-(2-Methyl-1*H*-imidazol-1-yl)propyl (2*R*)-[(1*R*)-3,3-difluorocyclopentyl](hydroxy) phenylacetate (Compound No. 62 aa);
3-(2-Methyl-1*H*-imidazol-1-yl)propyl *2R-2-(1S or 1R) (3,3*-difluorocyclohexyl) (hydroxy)phenylacetate (Compound No. 63 aa);
2-(2-Isopropyl-1*H*-imidazol-1-yl)ethyl cyclohexyl(hydroxy) phenylacetate (Compound No. 64 aa);
2-(1*H*-Imidazol-1-yl)ethyl cyclopentyl(hydroxy)phenylacetate (Compound No. 65 aa);
2-(2-Methyl-1*H*-imidazol-1-yl)ethyl (2*R*)-[(1*S*)-3,3-difluorocyclopentyl] (hydroxy) phenylacetate (Compound No. 66 aa);
2-(1*H*-Imidazol-1-yl)ethyl cyclohexyl(hydroxy)phenylacetate (Compound No. 67 aa);
2-(2-Isopropyl-1*H*-imidazol-1-yl)ethyl cyclopentyl(hydroxy) phenylacetate (Compound No. 68 aa);
2-(2-Methyl-1*H*-imidazol-1-yl)ethyl (2*R*)-[(1*R*)-3,3-difluorocyclopentyl] (hydroxy) phenylacetate (Compound No. 69 aa);
2-(2-Isopropyl-1*H*-imidazol-1-yl)ethyl cycloheptyl(hydroxy) phenylacetate (Compound No. 70 aa);
2-(2-Methyl-1*H*-imidazol-1-yl)ethyl cycloheptyl(hydroxy)phenylacetate (Compound No. 71 aa);
3-Benzyl-1-(2-{[cycloheptyl(hydroxy ) phenylacetyl]oxy}ethyl)-2-isopropyl-1*H*-imidazol-3-ium bromide (Compound No. 72 aa);
3-Benzyl-1-[2-({(2*R*)-2-[(1*S*)-3,3-difluorocyclopentyl]-2-hydroxy-2-phenylacetyl}oxy)ethyl]-2-isopropyl-1*H*-imidazol-3-ium bromide (Compound No. 73 aa);
3-Benzyl-1-[2-({(2*R*)-2-[(1*R*)-3,3-difluorocyclopentyl]-2-hydroxy-2-phenylacetyl}oxy) ethyl]-2-isopropyl-1*H*-imidazol-3-ium bromide (Compound No. 74 aa);

3-(4-Bromobenzyl)-1-[2-({(2*R*)-2-[(1*R*)-3,3-difluorocyclopentyl]-2-hydroxy-2-phenylacetyl}oxy)ethyl]-2-isopropyl-1*H*-imidazol-3-ium bromide (Compound No.75 aa);

3-Benzyl-l-[2-({(2*R*)-2-[(1*R*)-3,3-difluorocyclopentyl]-2-hydroxy-2-phenylacetyl}oxy)ethyl]-2-methyl-1*H*-imidazol-3-ium bromide (Compound No.76 aa);

3-(4-Bromobenzyl)-1-[2-({(2*R*)-2-[(1*R*)-3,3-difluorocyclopentyl]-2-hydroxy-2-phenylacetyl}oxy) ethyl]-2-methyl-1*H*-imidazol-3-ium bromide (Compound No. 77 aa);

3-(4-Fluorobenzyl-1-[2-({(2*R*)-2-[(1*R*)-3,3-difluorocyclopentyl]-2-hydroxy-2-phenylacetyl}oxy) ethyl]-2-methyl-1*H*-imidazol-3-ium bromide (Compound No. 78 aa);

3-Benzyl-1-(2-{[cycloheptyl(hydroxy)phenylacetyl]oxy}ethyl)-2-methyl-1*H*-imidazol-3-ium bromide (Compound No. 79 aa);

3-(4-Bromobenzyl)-1-(2-{[cycloheptyl(hydroxy)phenylacetyl]oxy}ethyl)-2-methyl-1*H*-imidazol-3-ium bromide (Compound No. 80 aa);

1-[2-({(2*R*)-2-[(1*S*)-3,3-difluorocyclopentyl]-2-hydroxy-2-phenylacetyl} oxy)ethyl]-2-isopropyl-3-methyl-1*H*-imidazol-3-ium iodide (Compound No. 81 aa);

1-[2-({(2*R*)-2-[(1*R*)-3,3-difluorocyclopentyl]-2-hydroxy-2-phenylacetyl}oxy)ethyl]-3-(4-fluorobenzyl)-2-isopropyl-1*H*-imidazol-3-ium bromide (Compound No. 82 aa);

1-(2-{[2-Cyclohexyl-2-hydroxy-2-phenylacetyl] oxy}ethyl)-2-isopropyl-3-methyl-1*H*-imidazol-3-ium iodide (Compound No. 83 aa);

1-(2-{[Cyclopentyl (hydroxy) phenylacetyl]oxy}ethyl)-3-methyl-1*H*-imidazol-3-ium iodide (Compound No. 84 aa);

1-[2-({(2*R*)-2-[(1*S*)-3,3-difluorocyclopentyl]-2-hydroxy-2-phenylacetyl}oxy)ethyl]-2,3-dimethyl-1*H*-imidazol-3-ium iodide (Compound No. 85 aa);

1-[2-({(2*R*)-2-[(1*R*)-3,3-difluorocyclopentyl]-2-hydroxy-2-phenylacetyl}oxy)ethyl]-2,3-dimethyl-1*H*-imidazol-3-ium iodide (Compound No. 86 aa);

1-(2-{[Cyclohexyl(hydroxy) phenylacetyl]oxy}ethyl)-3-methyl-1*H*-imidazol-3-ium iodide (Compound No. 87 aa);

1-(2-{[Cyclopentyl(hydroxy) phenylacetyl]oxy}ethyl)-2-isopropyl-3-methyl-1*H*-imidazol-3-ium iodide (Compound No. 88 aa);

1-(2-{[Cyclopentyl(hydroxy) phenylacetyl]amino}ethyl)-2,3-dimethyl-1*H*-imidazol-3-ium iodide (Compound No. 89 aa);

1-(2-{[Cyclohexyl(hydroxy) phenylacetyl]amino}ethyl)-2-isopropyl-3-methyl-1*H*-imidazol-3-ium iodide (Compound No. 90 aa);

1-(2-*{[Cycloheptyl(hydroxy) phenylacetyl]oxy}ethyl)-2,3-dimethyl-1*H*-imidazol-3-ium iodide (Compound No. 91 aa) ;

1-(2-{[Cycloheptyl(hydroxy) phenylacetyl]oxy}ethyl)-2-isopropyl-3-methyl-1*H*-imidazol-3-ium iodide (Compound No. 92 aa);

1-[2-({(2*R*)-2-[(1*R*)-3,3-difluorocyclopentyl]-2-hydroxy-2-phenylacetyl}oxy)ethyl]-2-isopropyl-3-methyl-1*H*-imidazol-3-ium iodide (Compound No. 93 aa);

1-(3-{[(2*R*)-2-(3,3-difluorocyclopentyl)-2-hydroxy-2-phenylacetyl]amino}propyl)-2,3-dimethyl-1*H*-imidazol-3-ium iodide (Compound No. 94 aa);

1-(3-{[Cyclopentyl(hydroxy) phenylacetyl]amino}propyl)-2,3-dimethyl-1*H*-imidazol-3-ium iodide (Compound No. 95 aa);

1-(3-{[Cyclohexyl(hydroxy) phenylacetyl]amino}propyl)-2,3-dimethyl-1*H*-imidazol-3-ium iodide (Compound No. 96 aa);

1-(2-{[Cyclopentyl(hydroxy) phenylacetyl](methyl)amino}ethyl)-3-methyl-1*H*-imidazo1-3-ium iodide (Compound No. 97 aa);

1-(3-{[Cyclopentyl(hydroxy) phenylacetyl](methyl)amino}propyl)-2,3-dimethyl-1*H*-imidazol-3-ium iodide (Compound No. 98 aa);

1-(3-{[Cyclopentyl(hydroxy) phenylacetyl]oxy}propyl)-2,3-dimethyl-1*H*-imidazol-3-ium iodide (Compound No. 99 aa);

1-[3-({(2*R*)-2-[(1*S*)-3,3-Difluorocyclopentyl]-2-hydroxy-2-phenylacetyl}oxy)propyl]-2,3-dimethyl-1*H*-imidazol-3-ium iodide (Compound No. 100 aa);

3-Benzyl-1-[3-({(2*R*)-2-[(1*S*)-3,3-difluorocyclopentyl]-2-hydroxy-2-phenylacetyl}oxy) propyl]-2-methyl-1*H*-imidazol-3-ium bromide (Compound No. 101 aa);

3-(4-Bromobenzyl)-1-[3-({(2*R*)-2-[(1*S*)-3,3-difluorocyclopentyl]-2-hydroxy-2-phenylacetyl}oxy)propyl]-2-methyl-1*H*-imidazol-3-ium bromide (Compound No. 102 aa);

1-[3-({(2*R*)-2-[(1*R*)-3,3-difluorocyclopentyl]-2-hydroxy-2-phenylacetyl}oxy)propyl]-2,3-dimethyl-1*H*-imidazol-3-ium iodide (Compound No. 103 aa);

3-Benzyl-1-[3-({(2*R*)-2-[(1*R*)-3,3-difluorocyclopentyl]-2-hydroxy-2-phenylacetyl}oxy) propyl]-2-methyl-1*H*-imidazol-3-ium bromide (Compound No. 104 aa);

3-(4-Bromobenzyl)-1-[3-({(2*R*)-2-[(1*R*)-3,3-difluorocyclopentyl]-2-hydroxy-2-phenylacetyl}oxy)propyl]-2-methyl-1*H*-imidazol-3-ium bromide (Compound No. 105 aa);

1-[3-({(2R)-2-[(1*S or 1R*)-3,3-difluorocyclohexyl]-2-hydroxy-2-phenylacetyl}oxy)propyl]-2,3-dimethyl-1*H*-imidazol-3-ium bromide (Compound No. 106 aa);

3-Benzyl-1-[3-({(2*R*)-2-[( *1R or 1S*)-3,3-difluorocyclohexyl]-2-hydroxy-2-phenylacetyl}oxy)propyl]-2-methyl-1*H*-imidazol-3-ium bromide (Compound No. 107 aa);
3-(4-Bromobenzyl)-1-[3-({(2*R*)-2-[(*1R or 1S*)-3,3-difluorocyclohexyl]-2-hydroxy-2-phenylacetyl}oxy)propyl]-2-methyl-1*H*-imidazol-3-ium bromide (Compound No. 108 aa);
1-(2-{[Cyclopentyl(hydroxy) phenylacetyl](methyl)amino}ethyl)-2,3-dimethyl-1*H*-imidazol-3-ium iodide (Compound No. 109 aa);
3-(2-Methyl-1*H*-imidazol-1-yl)propyl (2*R*)-[(1*S*)-3,3-difluorocyclopentyl] (hydroxy)phenylacetate (Compound No. 110 aa);
1-(2-{[Cyclohexyl(hydroxy) phenyl acetyl]oxy}ethyl)-2,3-dimethyl-1*H*-imidazol-3-ium iodide (Compound No 111 aa);
2-(2-Isopropyl-1*H*-imidazol-1-yl)ethyl (2*R*)-[(1*S*)-3,3-difluorocyclopentyl] (hydroxy)phenylacetate (Compound No. 112 aa);
2-(2-Isopropyl-1*H*-imidazol-1-yl)ethyl (2*R*)-[(1*R*)-3,3-difluorocyclopentyl] (hydroxy)phenylacetate (Compound No. 113 aa);
3-(4-Bromobenzyl)-1-[2-({(2R)-2-[(1R)-3,3-difluorocyclohexyl]-2-hydroxy-2-phenylacetyl}oxy)ethyl]-2-methyl-1H-imidazol-3-ium bromide (Compound No. 114aa);
1-[2-({(2R)-2-[(1R)-3,3-Difluorocyclohexyl]-2-hydroxy-2-phenylacetyl}oxy)ethyl]-2,3-dimethyl-1H-imidazol-3-ium iodide (Compound No. 115aa);
1-(2-{[Cycloheptyl(hydroxy)phenylacetyl]oxy}ethyl)-2-methyl-3-(4-methylbenzyl)-1H-imidazol-3-ium bromide (Compound No. 116aa);
1-[2-({(2R)-2-[(1R)-3,3-Difluorocyclopentyl]-2-hydroxy-2-phenylacetyl}oxy)ethyl]-2-methyl-3-(4-methylbenzyl)-1H-imidazol-3-ium bromide (Compound No. 117aa);
1-[3-({(2R)-2-[(1R)-3,3-Difluorocyclohexyl]-2-hydroxy-2-phenylacetyl}oxy)propyl]-2,3-dimethyl-1H-imidazol-3-ium iodide (Compound No. 118aa);
1, 1-(3-{[Cycloheptyl(hydroxy)phenylacetyl]oxy}propyl)-2,3-dimethyl-1H-imidazol-3-ium iodide (Compound No. 119aa);
1-(3-{[Cycloheptyl(hydroxy)phenylacetyl]oxy}propyl)-2-methyl-3-(4-methylbenzyl)-1H-imidazol-3-ium bromide (Compound No. 120aa);
3-(4-Bromobenzyl)-1-(3-{[cycloheptyl(hydroxy)phenylacetyl]oxy}propyl)-2-methyl-1H-imidazol-3-ium bromide (Compound No. 121aa);
3-(4-Bromobenzyl)-1-[3-({(2R)-2-[(1R)-3,3-difluorocyclohexyl]-2-hydroxy-2-phenylacetyl}oxy)propyl]-2-methyl-1H-imidazol-3-ium bromide (Compound No. 122aa);
1-[2-({(2R)-2-[(1R)-3,3-Difluorocyclohexyl]-2-hydroxy-2-phenylacetyl}oxy)ethyl]-2-methyl-3-(4-methylbenzyl)-1H-imidazol-3-ium bromide (Compound No. 123aa);
1-[2-({(2R)-2-[(1R)-3,3-Difluorocyclohexyl]-2-hydroxy-2-phenylacetyl}oxy)ethyl]-3-(4-fluorobenzyl)-2-methyl-1H-imidazol-3-ium bromide (Compound No. 124aa);
3-[2-(1,3-Benzodioxol-5-yl)ethyl]-1-(3-[cycloheptyl(hydroxy)phenylacetyl] oxy}propyl)-2-methyl-1H-imidazol-3-ium bromide (Compound No. 125aa);
1-[3-({(2R)-2-[(1R)-3,3-Difluorocyclopentyl]-2-hydroxy-2-phenylacetyl}oxy)propyl]-3-(4-fluorobenzyl)-2-methyl-1H-imidazol-3-ium bromide (Compound No. 126aa);
1-(3-{[Cycloheptyl(hydroxy)phenylacetyl]oxy}propyl)-3-(4-fluorobenzyl)-2-methyl-1H-imidazol-3-ium bromide (Compound No. 127aa);
3-(4-Bromobenzyl)-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 128aa);
3-(4-Fluorobenzyl)-1-[2-({(2R)-2-[(S)-3,3-difluorocyclohexyl]-2-hydroxy-2-phenylacetyl}oxy)ethyl]-2-methyl-1H-imidazol-3-ium bromide (Compound No. 129aa);
3-Benzyl-1-[2-({(2R)-2-[(1R)-3,3-difluorocyclopentyl]-2-hydroxy-2-phenylacetyl}oxy)ethyl]-2-methyl-1H-imidazol-3-ium chloride (Compound No. 130aa);
3-Benzyl-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 131aa);
1-[2-({(2R)-2-[(1R)-3,3-Difluorocyclopentyl]-2-hydroxy-2-phenylacetyl}oxy)ethyl]-2,3-dimethyl-1H-imidazol-3-ium bromide (Compound No. 132aa);
1-[3-({(2R)-2-[(1R)-3,3-Difluorocyclopentyl]-2-hydroxy-2-phenylacetyl}oxy)propyl]-3-isopropyl-2-methyl-1H-imidazol-3-ium bromide (Compound No. 133aa);
1-[3-({(2R)-2-[(1R)-3,3-Difluorocyclopentyl]-2-hydroxy-2-phenylacetyl}oxy)propyl]-3-ethyl-2-methyl-1H-imidazol-3-ium iodide (Compound No. 134aa);
3-(Cyclopropylmethyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 135aa);
3-(Cyclohexylmethyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 136aa);
1-[3-({(2R)-2-[(1R)-3,3-Difluorocyclopentyl]-2-hydroxy-2-phenylacetyl}oxy)propyl]-2-methyl-3-[4-(trifluoromethyl)benzyl]-1H-imidazol-3-ium chloride (Compound No. 137aa);
3-(4-Chlorobenzyl)-1-[3-({(2R)-2-[(1R)-3,3-difluorocyclopentyl]-2-hydroxy-2-phenylacetyl}oxy)propyl]-2-methyl-1H-imidazol-3-ium chloride (Compound No. 138aa);
3-(3-Bromobenzyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 139aa); 1-[3-({(2R)-2-[(1R)-3,3-Difluorocyclopentyl]-2-hydroxy-2-phenylacetyl}oxy)propyl]-3-(4-fluorobenzyl)-2-methyl-1H-imidazol-3-ium chloride (Compound No. 140aa);
3-Benzyl-1-[3-({(2R)-2-[(1R)-3,3-difluorocyclopentyl]-2-hydroxy-2-phenylacetyl}oxy)propyl]-2-methyl-1H-imidazol-3-ium chloride (Compound No. 141aa);
1-[3-({(2R)-2-[(1R)-3,3-Difluorocyclopentyl]-2-hydroxy-2-phenylacetyl}oxy)propyl]-2-methyl-3-(2-phenylethyl)-1H-imidazol-3-ium bromide (Compound No. 142aa);
1-[3-({(2R)-2-[(1R)-3,3-Difluorocyclopentyl]-2-hydroxy-2-phenylacetyl}oxy)propyl]-2,3-dimethyl-1H-imidazol-3-ium bromide (Compound No. 143aa);
1-[3-({(2R)-2-[(1R)-3,3-Difluorocyclohexyl]-2-hydroxy-2-phenylacetyl}oxy)propyl]-3-(4-fluorobenzyl)-2-methyl-1H-imidazol-3-ium bromide (Compound No. 144aa);
3-Benzyl-1-{3-[2-cycloheptyl(hydroxy)phenylacetoxy]propyl}-2-methyl-1H-imidazol-3-ium chloride (Compound No. 145aa);
1-(3-{[2-Cycloheptyl(hydroxy)phenylacetyl]oxy}propyl)-2,3-dimethyl-1H-imidazol-3-ium bromide (Compound No. 146aa);
1-[4-({(2R)-2-[(1R)-3,3-Difluorocyclopentyl]-2-hydroxy-2-phenylacetyl}oxy)butyl]-2,3-dimethyl-1H-imidazol-3-ium bromide (Compound No. 147aa);
3-Benzyl-1-[4-({(2R)-2-[(1R)-3,3-difluorocyclopentyl]-2-hydroxy-2-phenylacetyl}oxy)butyl]-2-methyl-1H-imidazol-3-ium bromide (Compound No. 148aa);
3-Benzyl-1-[2-({(2R)-2-[(1R)-3,3-difluorocyclohexyl]-2-hydroxy-2-phenylacetyl}oxy)ethyl]-2-methyl-1H-imidazol-3-iumn bromide (Compound No. 149aa);
3-Benzyl-1-[3-({(2R)-2-[(1R)-3,3-difluorocyclohexyl]-2-hydroxy-2-phenylacetyl}oxy)propyl]-2-methyl-1H-imidazol-3-ium chloride (Compound No. 150aa);
1-[4-({(2R)-2-[(1R)-3,3-Difluorocyclohexyl]-2-hydroxy-2-phenylacetyl}oxy)butyl]-2,3-dimethyl-1H-imidazol-3-ium bromide (Compound No. 151aa);
3-Benzyl-1-[4-({(2R)-2-[(1R)-3,3-difluorocyclohexyl]-2-hydroxy-2-phenylacetyl}oxy)butyl]-2-methyl-1H-imidazol-3-ium bromide (Compound No. 152aa);
3-(4-*tert*-Butylbenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 153aa);
3-(Biphenyl-4-ylmethyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 154aa);
3-(2,5-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 155aa);
3-(2,4-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 156aa);
3-(3,5-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 157aa);
3-(3,4-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 158aa);
1-{3-[2-Cyclopentyl(hydroxy)phenylacetoxy]propyl}-2-methyl-3-(pyridin-4-ylmethyl)-1H-imidazol-3-ium bromide (Compound No. 159aa);
1-[3-({(2R)-2-[(1R)-3,3-Difluorocyclopentyl]-2-hydroxy-2-phenylacetyl}oxy)propyl]-3-(1-ethylpropyl)-2-methyl-1H-imidazol-3-ium bromide (Compound No. 160aa);
3-(1,3-Benzodioxol-5-ylmethyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 161aa);
1-[3-({(2R)-2-[(1R)-3,3-Difluorocyclopentyl]-2-hydroxy-2-phenylacetyl}oxy)propyl]-2-methyl-3-propyl-1H-imidazol-3-ium bromide (Compound No. 162aa);
1-[3-({(2R)-2-[(1R)-3,3-Difluorocyclopentyl]-2-hydroxy-2-phenylacetyl}oxy)propyl]-3-isobutyl-2-methyl-1H-imidazol-3-ium bromide (Compound No. 163aa);
3-Butyl-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 164aa);
1-{2-[2-Hydroxy(phenyl)-2-thienylacetoxy]ethyl}-2-methyl-3-(2-phenylethyl)-1H-imidazol-3-ium bromide (Compound No. 165aa);
3-(Cyclopropylmethyl)-1-{2-[2-hydroxy(phenyl)-2-thienylacetoxy]ethyl}-2-methyl-1H-imidazol-3-ium bromide (Compound No. 166aa);

3-Benzyl-1-{2-[2-hydroxy(phenyl)-2-thienylacetoxy]ethyl}-2-methyl-1H-imidazol-3-ium bromide (Compound No. 167aa);
1-{2-[2-Hydroxy(phenyl)-2-thienylacetoxy]ethyl}-2,3-dimethyl-1H-imidazol-3-ium bromide (Compound No. 168aa);
3-(Cyclopropylmethyl)-1-(2-{2-hydroxy[bis(3-methylphenyl)]acetoxy}ethyl)-2-methyl-1H-imidazol-3-ium bromide (Compound No. 169aa);
1-(2-{2-Hydroxy[bis(3-methylphenyl)]acetoxy}ethyl)-2,3-dimethyl-1H-imidazol-3-ium bromide (Compound No. 170aa);
3-Benzyl-1-(2-{2-hydroxy[bis(3-methylphenyl)]acetoxy}ethyl)-2-methyl-1H-imidazol-3-ium bromide (Compound No. 171aa);
1-{2-[2, 2-Bis(4-fluorophenyl)(hydroxy)acetoxy]ethyl}-2,3-dimethyl-1H-imidazol-3-ium bromide (Compound No. 172aa);
3-Benzyl-1-{2-[2,2-bis(4-fluorophenyl)(hydroxy)acetoxy]ethyl}-2-methyl-1H-imidazol-3-ium bromide (Compound No. 173aa);
1-{2-[2,2-Bis(4-fluorophenyl)(hydroxy)acetoxy]ethyl}-3-(cyclopropylmethyl)-2-methyl-1H-imidazol-3-ium bromide (Compound No. 174aa);
3-Benzyl-1-{3-[2,2-bis(4-fluorophenyl)(hydroxy)acetoxy]propyl}-2-methyl-1H-imidazol-3-ium bromide (Compound No. 175aa);
1-{3-[2,2-*bis*(4-Fluorophenyl)(hydroxy)acetoxy]propyl}-3-(cyclopropylmethyl)-2-methyl-1H-imidazol-3-ium bromide (Compound No. 176aa);
1-{3-[2,2-*bis*(4-Fluorophenyl)(hydroxy)acetoxy]propyl}-3-ethyl-2-methyl-1H-imidazol-3-ium bromide (Compound No. 177aa);
1-[3-({(2R)-2-[(1R)-3,3-Difluorocyclopentyl]-2-hydroxy-2-phenylacetyl}oxy)propyl]-2-methyl-3-(3-phenoxypropyl)-1H-imidazol-3-ium bromide (Compound No. 178aa);
1-[3-({(2R)-2-[(1R)-3,3-Difluorocyclohexyl]-2-hydroxy-2-phenylacetyl}oxy)propyl]-2-methyl-3-(3-phenoxypropyl)-1H-imidazol-3-ium bromide (Compound No. 179aa);
1-[3-({(2R)-2-[(1R)-3,3-Difluorocyclohexyl]-2-hydroxy-2-phenylacetyl}oxy)propyl]-2-methyl-3-propyl-1H-imidazol-3-ium bromide (Compound No. 180aa);
1-[3-({(2R)-2-[(1R)-3,3-Difluorocyclohexyl]-2-hydroxy-2-phenylacetyl}oxy)propyl]-3-isopropyl-2-methyl-1H-imidazol-3-ium bromide (Compound No. 181aa);
3-(Cyclopropylmethyl)-1-[3-({(2R)-2-[(1R)-3,3-difluorocyclohexyl]-2-hydroxy-2-phenylacetyl}oxy)propyl]-2-methyl-1H-imidazol-3-ium bromide (Compound No. 182aa);
3-(Cyclohexylmethyl)-1-[3-({(2R)-2-[(1R)-3,3-difluorocyclohexyl]-2-hydroxy-2-phenylacetyl}oxy)propyl]-2-methyl-1H-imidazol-3-ium bromide (Compound No. 183aa);
1-[3-({(2R)-2-[(1R)-3,3-Difluorocyclohexyl]-2-hydroxy-2-phenylacetyl}oxy)propyl]-2-methyl-3-(2-phenylethyl)-1H-imidazol-3-ium bromide (Compound No. 184aa);
1-[3-({(2R)-2-[(1R)-3,3-Difluorocyclohexyl]-2-hydroxy-2-phenylacetyl}oxy)propyl]-2,3-dimethyl-1H-imidazol-3-ium bromide (Compound No. 185aa);
1-{3-[2,2-*bis*(4-Fluorophenyl)(hydroxy)acetoxy]propyl}-2,3-dimethyl-1H-imidazol-3-ium bromide (Compound No. 186aa);
3-(4-Bromobenzyl)-1-(3-{2-hydroxy[bis(3-methylphenyl)]acetoxy}propyl)-2-methyl-1H-imidazol-3-ium bromide (Compound No. 187aa);
3-Benzyl-1-(3-{2-hydroxy[bis(3-methylphenyl)]acetoxy}propyl)-2-methyl-1H-imidazol-3-ium bromide (Compound No. 188aa);
3-(Cyclopropylmethyl)-1-(3-2-hydroxy[bis(3-methylphenyl)]acetoxy}propyl)-2-methyl-1H-imidazol-3-ium bromide (Compound No. 189aa);
3-Ethyl-1-(3-{2-hydroxy[bis(3-methylphenyl)]acetoxy}propyl)-2-methyl-1H-imidazol-3-ium bromide (Compound No. 190aa);
1-(3-{2-Hydroxy[bis(3-methylphenyl)]acetoxy}propyl)-2,3-dimethyl-1H-imidazol-3-ium bromide (Compound No. 191aa);
3-(4-Bromobenzyl)-1-{3-[2-hydroxy(phenyl)-2-thienylacetoxy]propyl}-2-methyl-1H-imidazol-3-ium bromide (Compound No. 192aa);
3-Benzyl-1-{3-[2-hydroxy(phenyl)-2-thienylacetoxy]propyl}-2-methyl-1H-imidazol-3-ium bromide (Compound No. 193aa);
3-(Cyclopropylmethyl)-1-{3-[2-hydroxy(phenyl)-2-thienylacetoxy]propyl}-2-methyl-1H-imidazol-3-ium bromide (Compound No. 194aa);
3-Ethyl-1-{3-[2-hydroxy(phenyl)-2-thienylacetoxy]propyl}-2-methyl-1H-imidazol-3-ium bromide (Compound No. 195aa);
1-{3-[2-Hydroxy(phenyl)-2-thienylacetoxy]propyl}-2,3-dimethyl-1H-imidazol-3-ium bromide (Compound No. 196aa);
1-[2-({(2R)-2-[(1R)-3,3-Difluorocyclopentyl]-2-hydroxy-2-phenylacetyl}oxy)ethyl]-2-methyl-3-(3-phenoxypropyl)-1H-imidazol-3-ium bromide (Compound No. 197aa);
3-(Cyclopropylmethyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 198aa);
3-Butyl-1-[2-({(2R)-2-[(1R)-3,3-difluorocyclopentyl]-2-hydroxy-2-phenylacetyl}oxy)ethyl]-2-methyl-1H-imidazol-3-ium

bromide (Compound No. 199aa);
1-[2-({(2R)-2-[(1R)-3,3-Difluorocyclopentyl]-2-hydroxy-2-phenylacetyl}oxy)ethyl]-2-methyl-3-propyl-1H-imidazol-3-ium bromide (Compound No. 200aa);
1-[2-({(2R)-2-[(1R)-3,3-Difluorocyclopentyl]-2-hydroxy-2-phenylacetyl}oxy)ethyl]-3-ethyl-2-methyl-1H-imidazol-3-ium bromide (Compound No. 201aa);
3-Benzyl-1-{2-[2-hydroxy(di-2-thienyl)acetoxy]ethyl}-2-methyl-1H-imidazol-3-ium bromide (Compound No. 202aa);
3-(Cyclopropylmethyl)-1-{2-[2-hydroxy(di-2-thienyl)acetoxy]ethyl}-2-methyl-1H-imidazol-3-ium bromide (Compound No. 203aa);
3-Ethyl-1-{2-[2-hydroxy(di-2-thienyl)acetoxy]ethyl}-2-methyl-1H-imidazol-3-ium bromide (Compound No. 204aa);
1-{2-[2-Hydroxy(di-2-thienyl)acetoxy]ethyl}-2,3-dimethyl-1H-imidazol-3-ium bromide (Compound No. 205aa);
1-{3-[2-Hydroxy(di-2-thienyl)acetoxy]propyl}-2-methyl-3-(2-phenylethyl)-1H-imidazol-3-ium bromide (Compound No. 206aa);
1-{3-[2-Hydroxy(di-2-thienyl)acetoxy]propyl}-2-methyl-3-(3-phenoxypropyl)-1H-imidazol-3-ium bromide (Compound No. 207aa);
3-Butyl-1-{3-[2-hydroxy(di-2-thienyl)acetoxy]propyl}-2-methyl-1H-imidazol-3-ium bromide (Compound No. 208aa);
3-Ethyl-1-{3-[2-hydroxy(di-2-thienyl)acetoxy]propyl}-2-methyl-1H-imidazol-3-ium bromide (Compound No. 209aa);
1-{3-[2-Hydroxy(di-2-thienyl)acetoxy]propyl}-2,3-dimethyl-1H-imidazol-3-ium bromide (Compound No. 210aa);
1-(3-{[(2R)-2-Hydroxy-2-phenyl-2-{(1S)-3-[(phenylsulfonyl)amino]cyclopentyl} acetyl]oxy}propyl)-2,3-dimethyl-1H-imidazol-3-ium bromide (Compound No. 211aa);
3-Benzyl-1-(3-{[(2R)-2-hydroxy-2-phenyl-2-{(1S)-3-[(phenylsulfonyl)amino] cyclopentyl}acetyl]oxy}propyl)-2-methyl-1H-imidazol-3-ium bromide (Compound No. 212aa);
3-(4-Bromobenzyl)-1-(3-{[(2R)-2-hydroxy-2-phenyl-2-{(1S)-3-[(phenylsulfonyl)amino] cyclopentyl}acetyl]oxy}propyl)-2-methyl-1H-imidazol-3-ium bromide (Compound No. 213aa);
1-[3-({(2R)-2-[(1S)-3,3-Difluorocyclopentyl]-2-hydroxy-2-phenylacetyl}oxy)propyl]-3-ethyl-2-methyl-1H-imidazol-3-ium bromide (Compound No. 214aa);
1-[3-({(2R)-2-[(1S)-3,3-Difluorocyclopentyl]-2-hydroxy-2-phenylacetyl}oxy)propyl]-2-methyl-3-propyl-1H-imidazol-3-ium bromide (Compound No. 215aa);
3-Butyl-1-[3-({(2R)-2-[(1S)-3,3-difluorocyclopentyl]-2-hydroxy-2-phenylacetyl}oxy)propyl]-2-methyl-1H-imidazol-3-ium bromide (Compound No. 216aa);
1-[3-({(2R)-2-[(1S)-3,3-Difluorocyclopentyl]-2-hydroxy-2-phenylacetyl}oxy)propyl]-2-methyl-3-pentyl-1H-imidazol-3-ium bromide (Compound No. 217aa);
1-[3-({(2R)-2-[(1S)-3,3-Difluorocyclohexyl]-2-hydroxy-2-phenylacetyl}oxy)propyl]-3-ethyl-2-methyl-1H-imidazol-3-ium bromide (Compound No. 218aa);
1-[3-({(2R)-2-[(1S)-3,3-Difluorocyclohexyl]-2-hydroxy-2-phenylacetyl}oxy)propyl]-2-methyl-3-propyl-1H-imidazol-3-ium bromide (Compound No. 219aa);
1-[3-({(2R)-2-[(1S)-3,3-Difluorocyclohexyl]-2-hydroxy-2-phenylacetyl}oxy)propyl]-3-(1-ethylpropyl)-2-methyl-1H-imidazol-3-ium bromide (Compound No. 220aa);
1-[3-({(2R)-2-[(1S)-3,3-Difluorocyclohexyl]-2-hydroxy-2-phenylacetyl}oxy)propyl]-3-isopropyl-2-methyl-1H-imidazol-3-ium bromide (Compound No. 221aa);
1-[3-({(2R)-2-[(1S)-3,3-Difluorocyclohexyl]-2-hydroxy-2-phenylacetyl}oxy)propyl]-2-methyl-3-pentyl-1H-imidazol-3-ium bromide (Compound No. 222aa);
1-[3-({(2R)-2-[(1S)-3,3-Difluorocyclopentyl]-2-hydroxy-2-phenylacetyl}oxy)propyl]-3-isobutyl-2-methyl-1H-imidazol-3-ium bromide (Compound No. 223aa);
1-[3-({(2R)-2-[(1S)-3,3-Difluorocyclopentyl]-2-hydroxy-2-phenylacetyl}oxy)propyl]-3-(1-ethylpropyl)-2-methyl-1H-imidazol-3-ium bromide (Compound No. 224aa);
1-[3-({(2R)-2-[(1S)-3,3-Difluorocyclopentyl]-2-hydroxy-2-phenylacetyl}oxy)propyl]-3-isopropyl-2-methyl-1H-imidazol-3-ium bromide (Compound No. 225 aa);
3-Butyl-1-[3-({(2R)-2-[(1S)-3,3-difluorocyclohexyl]-2-hydroxy-2-phenylacetyl}oxy)propyl]-2-methyl-1H-imidazol-3-ium bromide (Compound No. 226aa);
1-[2-({(2R)-2-[(1S)-3,3-Difluorocyclohexyl]-2-hydroxy-2-phenylacetyl}oxy)ethyl]-3-(1-ethylpropyl)-2-methyl-1H-imidazol-3-ium bromide (Compound No. 227aa);
1-[2-({(2R)-2-[(1S)-3,3-Difluorocyclohexyl]-2-hydroxy-2-phenylacetyl}oxy)ethyl]-2-methyl-3-pentyl-1H-imidazol-3-ium bromide (Compound No. 228aa);
3-Butyl-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 229aa);
1-[2-({(2R)-2-[(1S)-3,3-Difluorocyclohexyl]-2-hydroxy-2-phenylacetyl}oxy)ethyl]-2-methyl-3-propyl-1H-imidazol-3-ium bromide (Compound No. 230aa);
1-[2-({(2R)-2-[(1S)-3,3-Difluorocyclohexyl]-2-hydroxy-2-phenylacetyl}oxy)ethyl]-3-ethyl-2-methyl-1H-imidazol-3-ium

bromide (Compound No. 231aa);
1-[2-({(2R)-2-[(1S)-3,3-Difluorocyclohexyl]-2-hydroxy-2-phenylacetyl}oxy)ethyl]-2,3-dimethyl-1H-imidazol-3-ium bromide (Compound No. 232aa);
1-[2-({(2R)-2-[(1S)-3,3-Difluorocyclopentyl]-2-hydroxy-2-phenylacetyl}oxy)ethyl]-3-(1-ethylpropyl)-2-methyl-1H-imidazol-3-ium bromide (Compound No. 233aa);
1-[2-({(2R)-2-[(1S)-3,3-Difluorocyclopentyl]-2-hydroxy-2-phenylacetyl}oxy)ethyl]-3-isopropyl-2-methyl-1H-imidazol-3-ium bromide (Compound No. 234aa);
1-[2-({(2R)-2-[(1S)-3,3-Difluorocyclopentyl]-2-hydroxy-2-phenylacetyl}oxy)ethyl]-2-methyl-3-pentyl-1H-imidazol-3-ium bromide (Compound No. 235aa);
3-Butyl-1-[2-({(2R)-2-[(1S)-3,3-difluorocyclopentyl]-2-hydroxy-2-phenylacetyl}oxy)ethyl]-2-methyl-1H-imidazol-3-ium bromide (Compound No. 236aa);
1-[2-({(2R)-2-[(1S)-3,3-Difluorocyclopentyl]-2-hydroxy-2-phenylacetyl}oxy)ethyl]-2-methyl-3-propyl-1H-imidazol-3-ium bromide (Compound No. 237aa);
1-[2-({(2R)-2-[(1S)-3,3-Difluorocyclopentyl]-2-hydroxy-2-phenylacetyl}oxy)ethyl]-3-ethyl-2-methyl-1H-imidazol-3-ium bromide (Compound No. 238aa);
2-(2-Methyl-1H-imidazol-1-yl)ethyl(2R)-[(1R)-3,3-difluorocyclohexyl](hydroxy) phenylacetate (Compound No. 239aa);
3-(2-Methyl-1*H*-imidazol-1-yl)propyl (2*R*)-[(1*R*)-3,3-difluorocyclohexyl](hydroxy) phenylacetate (Compound No. 240aa);
3-(2-Methyl-1H-imidazol-1-yl)propyl cycloheptyl(hydroxy)phenylacetate (Compound No. 24 1 aa);
2-(2-Methyl-1H-imidazol-1-yl)ethyl(2R)-[(1S)-3,3-difluorocyclohexyl](hydroxy) phenylacetate (Compound No. 242aa);
4-(2-Methyl-1H-imidazol-1-yl)butyl(2R)-[(1R)-3,3-difluorocyclopentyl](hydroxy) phenylacetate (Compound No. 243aa);
4-(2-Methyl-1H-imidazol-1-yl)butyl(2R)-[(1R)-3,3-difluorocyclohexyl](hydroxy) phenylacetate (Compound No. 244aa);
2-(2-Methyl-1*H*-imidazol-1-yl)ethyl hydroxy(phenyl)-2-thienylacetate (Compound No. 245aa);
2-(2-Methyl-1*H*-imidazol-1-yl)ethyl hydroxy[bis(3-methylphenyl)]acetate (Compound No. 246aa);
2-(2-Methyl-1H-imidazol-1-yl)ethyl bis(4-fluorophenyl)(hydroxy)acetate (Compound No. 247aa);
3-(2-Methyl-1H-imidazol-1-yl)propylbis(4-fluorophenyl)(hydroxy)acetate (Compound No. 248aa);
3-(2-Methyl-1H-imidazol-1-yl)propylhydroxy[bis(3-methylphenyl)]acetate (Compound No. 249aa);
3-(2-Methyl-1*H*-imidazol-1-yl)propyl hydroxy(phenyl)2-thienylacetate(Compound No. 250aa);
2-(2-Methyl-1H-imidazol-1-yl)ethyl hydroxy(di-2-thienyl)acetate (Compound No. 251aa);
3-(2-Methyl-1H-imidazol-1-yl)propyl hydroxy(di-2-thienyl)acetate (Compound No. 252aa);
3-(2-Methyl-1H-imidazol-1-yl)propyl (2R)-hydroxy(phenyl){(1S)-3-[(phenylsulfonyl) amino] cyclopentyl}acetate (Compound No. 253aa);
3-(2-Methyl-1H-imidazol-1-yl)propyl(2R)-[(1S)-3,3-difluorocyclohexyl](hydroxy) phenylacetate (Compound No. 254aa);
3-butyl-1-[3-({(2*R*)-2-[(1*R*)-3,3-difluorocyclohexyl]-2-hydroxy-2-phenylacetyl}oxy)propyl]-2-methyl-1*H*-imidazol-3-ium bromide (Compound No. 255aa);
1-[3-({(2*R*)-2-[(1*R*)-3,3-difluorocyclohexyl]-2-hydroxy-2-phenylacetyl}oxy)propyl]-2-methyl-3-pentyl-1*H*-imidazol-3-ium bromide(Compound No. 256aa);
1-[3-({(2*R*)-2-[(1*R*)-3,3-difluorocyclohexyl]-2-hydroxy-2-phenylacetyl}oxy)propyl]-3-(1-ethylpropyl)-2-methyl-1*H*-imidazol-3-ium bromide(Compound No. 257aa);
1-[3-({(2*R*)-2-[(1*R*)-3,3-difluorocyclohexyl]-2-hydroxy-2-phenylacetyl}oxy)propyl]-2-methyl-3-(2-methylpropyl)-1*H*-imidazol-3-ium bromide(Compound No. 258aa);
1-(3-{[(3,3-difluorocyclopentyl)(hydroxy)thiophen-2-ylacetyl]oxy}propyl)-3-ethyl-2-methyl-1*H*-imidazol-3-ium bromide (Compound No. 259aa);
1-(3-{[(3,3-difluorocyclopentyl)(hydroxy)thiophen-2-ylacetyl]oxy}propyl)-2,3-dimethyl-1*H*-imidazol-3-ium bromide(Compound No. 260aa); and
3-benzyl-1-(3-{[(3,3-difluorocyclopentyl)(hydroxy)thiophen-2-ylacetyl]oxy}propyl)-2-methyl-1*H*-imidazol-3-ium bromide (Compound No. 261aa).

Suitable anticholinergics include, for example, ipratropium salts, oxitropium salts, salts of the compounds known from WO 02/32899: tropenol N-methyl-2,2-diphenylpropionate, scopine N-methyl-2,2-diphenylpropionate, scopine N-methyl-2-fluoro-2,2-diphenylacetate and tropenol N-methyl-2-fluoro-2,2-diphenylacetate; as well as salts of the compounds known from WO 02/32898: tropenol N-methyl-3,3',4,4'-tetrafluorobenzilate, scopine N-methyl-3,3',4,4'-tetrafluorobenzilate, scopine N-methyl-4,4'-dichlorobenzilate, scopine N-methyl-4,4'-difluorobenzilate, tropenol N-methyl-3,3'-difluorobenzilate, scopine N-methyl-3,3'-difluorobenzilate, and tropenol N-ethyl-4,4'-difluorobenzilate, optionally in the form of their hydrates and solvates. By salts are meant those compounds which contain, in addition to the above mentioned cations, as counterion, an anion with a single negative charge selected from among the chloride, bromide, and methanesulfonate.

Particular anticholinergics include, for example, tiotropium bromide, ipratropium bromide, oxitropium bromide, tropenol 2,2-diphenylpropionate methobromide, scopine 2,2-diphenylpropionate methobromide, scopine 2-fluoro-2,2-diphenylacetate methobromide, tropenol 2-fluoro-2,2-diphenylacetate methobromide, tropenol 3,3',4,4'-tetrafluorobenzilate methobromide, scopine 3,3',4,4'-tetrafluorobenzilate methobromide; scopine 4,4'-dichlorobenzilate methobromide, scopine 4,4'-difluorobenzilate methobromide, tropenol 3,3'-difluorobenzilate methobromide, scopine 3,3'-difluorobenzilate methobromide, and tropenol 4,4'-difluorobenzilate ethylbromide.

Suitable antiallergic agents include, for example, epinastine, cetirizine, azelastine, fexofenadine, levocabastine, loratadine, mizolastine, ketotifene, emedastine, dimetindene, clemastine, bamipine, hexachloropheniramine, pheniramine, doxylamine, chlorophenoxamine, dimenhydrinate, diphenhydramine, promethazine, ebastine, desloratadine, and meclizine. Particular antiallergic agents include, for example, epinastine, cetirizine, azelastine, fexofenadine, levocabastine, loratadine, ebastine, desloratadine, and mizolastine, epinastine. Any reference to the above-mentioned antiallergic agents also includes any pharmacologically acceptable acid addition salts thereof, which may exist.

Suitable PAF antagonists include, for example, 4-(2-chlorophenyl)-9-methyl-2-[3-(4-morpholinyl)-3-propanon-1-yl]-6H-thieno[3,2-f][1,2,4]triazolo[4,3-a][1,4]diazepine and 6-(2-chlorophenyl)-8,9-dihydro-1-methyl-8-[(4-morpholinyl)carbonyl]-4H,7H-cyclopenta[4.5]thieno[3,2-f][1,2,4]triazolo[4,3-a][1,4]diazepine.

Suitable EGFR kinase inhibitors include, for example, 4-[(3-chloro-4-fluorophenyl)amino]-7-(2-{4-[(S)-(2-oxotetrahydrofuran-5-yl)carbonyl]piperazin-1-yl}-ethoxy)-6-[(vinylcarbonyl)amino]quinazoline, 4-[(3-chloro4-fluorophenyl)amino]-7-[4-((S)-6-methyl-2-oxomorpholin-4-yl)butyloxy]-6-[(vinylcarbonyl)amino]quinazoline, 4-[(3-chloro4-fluorophenyl)amino]-7-[4-((R)-6-methyl-2-oxomorpholin-4-yl)butyloxy]-6-[(vinylcarbonyl)amino]quinazoline, 4-[(3-chloro-4-fluorophenyl)amino]-7-[2-((S)-6-methyl-2-oxomorpholin-4-yl)ethoxy]-6-[(vinylcarbonyl)amino]quinazoline, 4-[(3-chloro-4-fluorophenyl)amino]-6-[(4-{N-[2-(ethoxycarbonyl)ethyl]-N-[(ethoxycarbonyl)methyl]-amino}-1-oxo-2-buten-1-yl)amino]-7-cyclopropylmethoxyquinazoline, 4-[(R)-(1-phenylethyl)amino]-6-{[4-(morpholin-4-yl)-1-oxo-2-buten-1-yl]amino}-7-cyclopropyl-methoxyquinazo line, and 4-[(3-chloro-4-fluorophenyl)amino]-6-[3-(morpholin-4-yl)propyloxy]-7-methoxyquinazoline. Any reference to the above-mentioned EGFR kinase inhibitors also includes any pharmacologically acceptable acid addition salts thereof which may exist. By the physiologically or pharmacologically acceptable acid addition salts thereof which may be formed by the EGFR kinase inhibitors are meant, according to the invention, pharmaceutically acceptable salts selected from among the salts of hydrochloric acid, hydrobromic acid, sulfuric acid, phosphoric acid, methanesulfonic acid, acetic acid, fumaric acid, succinic acid, lactic acid, citric acid, tartaric acid, or maleic acid. The salts of the EGFR kinase inhibitors selected from among the salts of acetic acid, hydrochloric acid, hydrobromic acid, sulfuric acid, phosphoric acid, and methanesulfonic acid are preferred according to the invention.

Suitable additional PDE-IV inhibitors include, for example, enprofylline, roflumilast, ariflo, Bay-198004, CP-325,366, BY343, D-4396 (Sch-351591), V-11294A, Z-15370, and AWD-12-281. Particular PDE-IV inhibitors include enprofylline, roflumilast, ariflo, Z15370, and AWD-12-281. Any reference to the above mentioned PDE-IV inhibitors also includes any pharmacologically acceptable acid addition salts thereof which may exist. By the physiologically acceptable acid addition salts which may be formed by the abovementioned PDE-IV inhibitors are meant, according to the invention, pharmaceutically acceptable salts selected from among the salts of hydrochloric acid, hydrobromic acid, sulfuric acid, phosphoric acid, methanesulfonic acid, acetic acid, fumaric acid, succinic acid, lactic acid, citric acid, tartaric acid, or maleic acid. According to the invention, the salts selected from among the acetate, hydrochloride, hydrobromide, sulfate, phosphate, and methanesulfonate are preferred in this context.

The leukotriene antagonist can be selected from compounds not limited to those described in US 5,565,473, US 5,583,152, US 4,859,692 or US 4,780,469.

Examples of leukotriene antagonist include, but are not limited to, montelukast, zafirlukast, pranlukast and pharmaceutically acceptable salts thereof.

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like.

Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-dibenzylethylenediamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, and tromethamine.

When a compound of the present invention is basic, salts may be prepared from pharmaceutically acceptable non-toxic acids, including inorganic and organic acids, such as acetic, benzenesulfonic, benzoic, citric, ethanesulfonic, fumaric, gluconic, glutamic, hydrobromic, hydrochloric, isethionic, lactic, maleic, malic, mandelic, methanesulfonic, nitric, pantothenic, phosphoric, succinic, sulfuric, tartaric, and p-toluenesulfonic acid.

Pharmaceutical compositions of the present invention may be administered by following routes, for example, oral, topical, intravenous, intraarterial, intraperitoneal, intrathecal, intraventricular, intraurethral, intrasternal, intracranial, intramuscular, subcutaneous, intranasally, inhalation, rectally or vaginally.

Solid form preparations include powders, tablets, dispersible granules, capsules, cachets, suppositories, troches, patches, gel caps, magmas, lozenges, creams, pastes, plasters, lotions, discs, or ointments. Liquid form preparations include solutions suspensions, emulsions, syrups, elixirs, aerosols, inhalations, nasal spays or oral sprays.

The active compound can be admixed under sterile condition with pharmaceutically acceptable carrier and any needed preservatives or buffer as may be required.

Pharmaceutical compositions for use in the methods described herein may be prepared by any of the methods of pharmacy, but all methods include the step of bringing into association the active ingredient with the carrier which constitutes one or more necessary ingredients. In general, the compositions are prepared by uniformly and intimately admixing the active ingredient with pharmaceutically acceptable liquid carriers or finely divided solid carriers or both, and then, if necessary, shaping the product into the desired presentation.

Commonly used carriers include one or more of corn starch, lactose, talc, calcium phosphate, calcium sulphate, calcium stearate, magnesium stearate, steane acid, sorbitol, microcrystalline cellulose, mannitol, gelatin, natural or synthetic gums, such as carboxymethylcellulose, methylcellulose, alginate, dextran, acacia gum, karaya gum, locust bean gum. Additionally, other excipients such as diluents, binders, lubricants, disintegrants, colors and flavoring agents may be employed. For example, a tablet may be prepared by compression or molding, optionally with one or more pharmaceutically acceptable excipient. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent.

In addition to the common dosage forms set out above, the therapeutically active ingredients may also be administered by controlled release means and/or delivery devices to provide the rate-controlled release of any one or more of the components or active ingredients to optimize the desired therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

The "polymeric matrix" serves essentially to modulate drug release kinetics and to stabilize metastable drug. Due to their versatility, polymers represent election material for matrix delivery systems. Indeed polymeric matrices can be profitably used in, for example, oral delivery, implantable systems, tissue engineering, DNA/RNA release, intelligent delivery systems and polymer conjugation.

The magnitude of a prophylactic or therapeutic dose of one or more compounds described herein in the acute or chronic prevention, treatment, or management of a disorder or condition will vary with the severity of the condition to be treated and the route of administration. The dose, and perhaps the dose frequency, will also vary according to the age, body weight, and response of the individual patient. Suitable total daily dose ranges can be readily determined by those skilled in the art. In general, the total daily dose range for one or more compounds described herein, for the conditions described herein, may range from about 1 mg to about several grams administered in single or divided doses according to the particular application and the potency of the active ingredient. Suitable dosage amounts can be determined using small dosages that are less than the optimum dose. Such small dosages can be increased in small increments until the optimum effect is reached. Dosage amounts may be divided and administered as divided doses if desired.

Phosphodiesterase inhibitors of type IV of Formula Ia and Formula Ib and muscarinic receptor antagonists (MRA) can be present in compositions described herein in ratios from about 1:10 to 10:1. Phosphodiesterase inhibitors of type IV and muscarinic receptor antagonists (MRA) can also be present in compositions described herein in ratios of about 1:1, 2:1, 1:2, 1:3, 3:1, 1:5 and even 5:1.

Phosphodiesterase inhibitors of type IV of Formula Ia and Formula Ib and β2-agonists can be present in ratios from about 1:10 to 10:1. Phosphodiesterase inhibitors of type IV and β2-agonists can also be present in compositions described herein in ratios of about 1:1, 2:1, 1:2, 1:3, 3:1, 1:5 and even 5:1.

Phosphodiesterase inhibitors of type IV of Formula Ia and Formula Ib and p38 MAP Kinase inhibitors can be present in compositions described herein in ratios from about 1:10 to 10:1. Phosphodiesterase inhibitors of type IV and p38 MAP Kinase inhibitors can also be present in compositions described herein in ratios of about 1:1, 2:1, 1:2, 1:3, 3:1, 1:5 and even 5:1.

Phosphodiesterase inhibitors of type IV of Formula Ia and Formula Ib and corticosteroids can be present in compositions described herein in ratios from about 1:10 to 10:1. Phosphodiesterase inhibitors of type IV and corticosteroids can also be present in compositions described herein in ratios of about 1:1, 2:1, 1:2, 1:3, 3:1, 1:5 and even 5:1.

The present invention also provides for methods of treating or preventing autoimmune, inflammatory, or allergic disorders. Methods include administering to a mammal in need thereof a pharmaceutical dosage form comprising a therapeutically effective amount of one or more compounds described herein of Formula Ia or Formula Ib, and therapeutically effective amount of at least one other active ingredient such as one or more muscarinic receptor antagonists (MRA), β2-agonists, one or more corticosteroids, or one or more p38 MAP kinase inhibitors, and one or more pharmaceutically acceptable excipients.

The present invention also provides for methods of treating or preventing autoimmune, inflammatory, or allergic disorders. Methods include administering to a mammal in need thereof a pharmaceutical dosage form comprising a therapeutically effective amount of one or more compounds described herein, of Formula Ia or Formula Ib and therapeutically effective amount of at least one other active ingredient such as one or more anticholinergics, one or more muscarinic receptor antagonists, one or more antiallergics, one or more PAF antagonists, one or more leukotriene antagonists, one or more EGFR kinase inhibitors, or one or more additional PDE-IV inhibitors, and one or more pharmaceutically acceptable excipients.

Yet other methods include concurrent or sequential administration to a mammal in need thereof: a) a pharmaceutical dosage form comprising a therapeutically effective amount of one or more compounds described herein of Formula Ia or Formula Ib, and one or more pharmaceutically acceptable excipients; and b) one or more dosage forms comprising therapeutically effective amounts at least one other active ingredient such as one or more of corticosteriods, one or more β2-agonists, or one or more p38 MAP kinase inhibitors, and one or more pharmaceutically acceptable excipients.

Yet other methods include concurrent or sequential administration to a mammal in need thereof: a) a pharmaceutical dosage form comprising a therapeutically effective amount of one or more compounds described herein of Formula Ia or Formula Ib, and one or more pharmaceutically acceptable excipients; and b) one or more dosage forms comprising therapeutically effective amounts at least one other active ingredient such as one or more muscarinic receptor antagonists, one or more anticholinergics, one or more antiallergics, one or more PAF antagonists, one or more leukotriene antagonists, one or more EGFR kinase inhibitors or one or more additional PDE-IV inhibitors, and one or more pharmaceutically acceptable excipients.

In one embodiment, there are provided methods for treating or preventing autoimmune and/or inflammatory and/or allergic diseases or disorders comprising administering one or more compounds of pharmaceutical compositions described herein. Such autoimmune and/or inflammatory and/or allergic diseases or disorders include, for example, respiratory disorder, asthma, chronic bronchitis, chronic obstructive pulmonary disease, whooping cough, eosinophilic granuloma, psoriasis and other benign or malignant proliferative skin diseases, eczema, inflammatory bowel disease, endotoxic shock, anaphylactic shock, laminitis in horses, septic shock, ulcerative colitis, Crohn's disease, reperfusion injury of the myocardium and brain, inflammatory arthritis, perodontitis, chronic glomerulonephritis, atopic dermatitis, urticaria, adult respiratory distress syndrome, infant respiratory distress syndrome, transplant rejection, rhinitis, pruritus, diabetes insipidus, eye diseases, allergic rhinitis, allergic conjunctivitis, vernal conjunctivitis, arterial restenosis, ortherosclerosis, atherosclerosis, neurogenic inflammation, pain, cough, rheumatoid arthritis, osteoporosis, osteoarthritis, inflammation, ankylosing spondylitis, transplant rejection, graft versus host disease, hypersecretion of gastric acid, bacterial, fungal induced sepsis, viral induced sepsis, fungal induced septic shock, viral induced septic shock, inflammation-mediated chronic tissue degeneration, cytokine-mediated chronic tissue degeneration, osteoarthritis, cancer, cachexia, muscle wasting, depression memory impairment, tumor growth, cancerous invasion of normal tissues Hashimoto's thyroiditis (underactive thyroid), Graves' disease (overactive thyroid), Lupus and acquired immuno deficiency syndrome.

While the present invention has been described in terms of its specific embodiments, certain modifications and equivalents will be apparent to those skilled in the art and are included within the scope of the present invention. The examples are provided to illustrate particular aspects of the disclosure and do not limit the scope of the present invention as defined by the claims.

Biological Assay Methods:

Example 1: *In-vitro* assay to evaluate efficacy of PDE IV inhibitors in combination with p38 MAP Kinase inhibitors

*Cell based Assay for TNF-α release*:

Blood was collected in heparin or EDTA vacutainers from healthy human volunteers and Peripheral Blood Mononuclear Cells isolated using Ficoll Hypaque gradient. The cells were resuspended in serum free RPMI 1640 medium at a concentration of 2 million cells/ml.). 1 ml of this cell suspension was co-incubated with 20 μl of compound, alone or in combination, for 10 min in a flat bottom 96 well microtiter plate. Compounds were dissolved in DMSO initially and diluted in medium for a final concentration of 0.2 % DMSO. LPS (1 μg/ml, final concentration) was then added at a volume of 10 μl per well. After 30 min, 20 μl of fetal calf serum (final concentration of 10 %) was added to each well. Cultures were incubated overnight at 37 °C in an atmosphere of 5 % $CO_2$ and 95 % air. Supernatant were then removed and tested by ELISA for TNF-α release using a commercial kit (e.g. BD Biosciences). The level of TNF-α in treated wells was compared with the vehicle treated controls and inhibitory potency of compound was expressed as $IC_{50}$ values calculated from the percent inhibition values by using Graph pad prism.

$$\text{Percent inhibition} = 100 - \frac{\text{Percent TNF-}\alpha\text{ drug treated}}{\text{Percent TNF-}\alpha\text{ in vehicle treated}} \times 100$$

IC $_{50}$ of TNF-α release inhibition:

Compound No. 266 (PDE IV inhibitor) and p38 MAP Kinase inhibitors (Compound No. 44a, Compound No. 46a, Compound No. 47a and Vx-745, as disclosed in WO 98/27098) exhibited following $IC_{50}$ in inhibiting TNF-α release from human PBMCs.

**TABLE 1**

| Compound | $IC_{50}$ (nM) |
|---|---|
| Compound No. 266 | 76 |
| Compound No. 44a | 3 |
| Compound No. 46a | 2 |
| Compound No. 47a | 22 |
| Vx-745, as disclosed in WO 98/27098 | 14 |

Compound No. 266 (PDE IV inhibitor) and p38 MAP Kinase inhibitors (Compound No. 44a, Compound No. 46a, Compound No. 47a and Vx-745, as disclosed in WO 98/27098) exhibited following combination index (CI) in inhibiting TNF-α release from human PBMCs.

**TABLE 2**

| Compound | Combination Index (CI) | Indication |
|---|---|---|
| Compound No. 266 + Vx-745, as disclsoed in WO 98/27098 | 0.34 | Synergy |
| Compound No. 266 + Compound No. 44a | 0.25 | Synergy |
| Compound No. 266 + Compound No. 46a | 0.62 | Synergy |
| Compound No. 266 + Compound No. 47a | 0.16 | Synergy |

PDE 4 inhibitor Compound No. 266 inhibited the release of TNF alpha with an $IC_{50}$ value of 76 nM. It exhibited a synergistic response with p38 MAP Kinase inhibitors Compound No. 44a, Compound No. 46a, Compound No. 47a and Vx-745, as disclosed in WO 98/27098 in inhibiting the TNF alpha release in human PBMCs.

Example 2: *In-vitro* assay to evaluate efficacy of PDE IV inhibitors in combination with β-agonist - Measurement of Intracellular cAMP Elevation in U937 Cells

U937 cells are grown (human promonocytic cell line) in endotoxin-free RPMI 1640 + HEPES medium containing 10% (v/v) heat-inactivated foetal bovine serum and 1% (v/v) of an antibiotic solution (5000 IU/ml penicillin, 5000 μg/ml streptomycin). Cells are resuspended ($0.25 \times 10^6$/200 μl) in Krebs' buffer solution and are incubated at 37°C for 15 min in the presence of test compounds or vehicle (20μl). Generation of cAMP is initiatated by adding 50 μl of 10 μM prostaglandin (PGE2). The reaction is stopped after 15 min, by adding 1 N HCl (50 μl) and is placed on ice for 30 min. The sample is centrifuged (450g, 3 min), and levels of cAMP are measured in the supernatant by using cAMP enzyme-linked immunosorbent assay kit (Assay Designs). Percent inhibition is calculated by the following formula and $IC_{50}$ value is calculated with these values using Graph pad prism.

$$\text{Percent inhibition} = 100 - \frac{\text{Percent conversion in drug treated}}{\text{Percent conversion in vehicle treated}} \times 100$$

Example 3: *In-vitro* functional assays to evaluate efficacy of PDE IV inhibitors in combination with beta-agonists

***Animals and anaesthesia***

Guinea Pig is procured (400-600gm) and trachea is removed under anesthesia (sodium pentobarbital, 300 mg/kg i.p) and is immediately kept in ce-cold Krebs Henseleit buffer. Indomethacin (10μM) is present throughout the KH buffer to prevent the formation of bronchoactive prostanoids.

***Trachea experiments:***

The tissue of adherent fascia is cleaned and cut into strips of equal size (with approx. 4-5 tracheal rings in each strip). Epithilium is carefully removed by rubbing, minimizing damage to the smooth muscle. The trachea is opened along the mid-dorsal surface with the smooth muscle band intact and a series of transverse cuts is made from alternate sides so that they do not transect the preparation completely. Opposite end of the cut rings is tied with the help of a thread. The tissue is mounted in isolated tissue baths containing 10ml Krebs Henseleit buffer maintained at 37°C and bubbled with carbogen, at a basal tension of 1 gm. The buffer is changed 4-5 times for about an hour. The tissue is equilibrated for 1 hr with 1μM carbachol or 10μM histamine for stabilization. The tissue is washed for 30 minutes followed by a precontraction with histamine (10μM) or carbachol (1μM). The tension which is developed is allowed to stabilize for 15-20 minutes followed by the cumulative addition of beta-agonists prior to incubation with suboptimal dose of PDE IV inhibitor. The contractile response of tissues is recorded either on Powerlab data acquisition system or on Grass polygraph (Model 7). The relaxation as percentage is expressed of maximum carbachol response. The data is expressed as mean ± S.E. mean for n observations. The $EC_{50}$ is calculated as the concentration producing 50% of the maximum relaxation to 1μM carbachol. The percent relaxation is compared between the treated and control tissues using non-parametric unpaired t-test. A p value of $< 0.05$ is considered to be statistically significant.

Example 4: *In-vivo* assay to evaluate efficacy of PDE IV inhibitors in combination with beta-agonists

*Lipopolysaccharide (LPS) induced airway hyperreactivity (AHR) and neutrophilia:*

<u>Drug treatment:</u>

Beta-agonist (1ng/kg to 1mg/kg) and PDE4 inhibitor (1ng/kg to 1mg/kg) are instilled intratracheally under anesthesia either alone or in combination.

Method:

Male wistar rats weighing 200±20gem are used in the study. Rats should have free access to food and water. On the day of experiment, animals are exposed to lipopolysaccharide (LPS, 100μg/ml) for 40 min. One group of vehicle treated rats should be exposed to phosphate buffered saline (PBS) for 40 min. Two hours after LPS/PBS exposure, animals are placed inside a whole body plethysmograph (Buxco Electronics, USA) and are exposed to PBS or increasing acetylcholine (1, 6, 12, 24, 48 and 96 mg/ml) aerosol until Penh values (index of airway resistance) of rats attain 2 times the value (PC-100) seen with PBS alone. Respiratory parameters are recorded online using Biosystem XA software, (Buxco Electronics, USA). Penh, at any chosen dose of acetylcholine is expressed as percent of PBS response and PC100 (2 folds of PBS value) values computed using a nonlinear regression analysis. Percent inhibition is computed using the following formula.

$$\%\ \text{Inhibition} = \frac{PC100_{LPS} - PC100_{TEST}}{PC100_{LPS} - PC100_{PBS}} \times 100$$

Where,
$PC100_{LPS}$ = PC100 in vehicle treated group challenged group with LPS
$PC100_{TEST}$ = PC100 in group treated with a given dose of test compound
$PC100_{PBS}$ = PC100 in vehicle treated group challenged with PBS

Immediately after the airway hyperreactivity response is recorded, animals are eithanized and bronchoalveolar lavage (BAL) is performed. Collected lavage fluid is centrifuged at 3000 rpm for 5 min, at 4°C. Pellet is collected and resuspend in 1ml HBSS. Total leukocyte count is determined in the resuspended sample. A portion of suspension to be cytocentrifuged and stained with Leishmann's stain for differential leukocyte count. Total leukocyte and Neutrophil counts are expressed as cell count (millions cells $ml^{-1}$ of BAL). Percent inhibition I computed using the following formula.

$$\%\ \text{Inhibition} = \frac{NC_{LPS} - NC_{TEST}}{NC_{LPS} - NC_{PBS}} \times 100$$

Where,
$NC_{LPS}$ = Percentage of neutrophil in vehicle treated group challenged with LPS
$NC_{TEST}$ =Percentage of neutrophil in group treated with a given dose of test compound
$NC_{PBS}$ = Percentage of neutrophil in vehicle treated group challenged with PBS
$ED_{50}$ vales are computed from the percent inhibition data using Graph Pad Prism software (Graphpad Software Inc.,USA).

Example 5: *In-vitro* assay to evaluate efficacy of PDE IV inhibitors in combination with corticosteroids

*Cell based Assay for TNF-α release*:

Blood was collected in heparin or EDTA vacutainers from healthy human volunteers and Peripheral Blood Mononuclear Cells isolated using Ficoll Hypaque gradient. The cells were resuspended in serum free RPMI 1640 medium at a concentration of 2 million cells/ml). 1 ml of this cell suspension was co-incubated with 20 μl of compound, alone or in combination (PDE IV inhibitor and corticosteroid), for 10 min in a flat bottom 96 well microtiter plate The aforesaid compounds were dissolved in DMSO initially and diluted in medium for a final concentration of 0.2 % DMSO. LPS (1 mg/ml, final concentration) was then added at a volume of 10 μl per well. After 30 min, 20 μl of fetal calf serum (final concentration of 10 %) was added to each well. Cultures were incubated overnight at 37 °C in an atmosphere of 5 % $CO_2$ and 95 % air. Supernatant was then removed and tested by ELISA for TNF-α release using a commercial kit (e.g. BD Biosciences). The level of TNF-α in treated wells was compared with the vehicle treated controls and inhibitory potency of compound was expressed as $IC_{50}$ values calculated by from percent inhibition values using Graph pad prism. $IC_{50}$ values of test compounds were found to be in the range of lower μM to nM concentration.

$$\text{Percent inhibition} = 100 - \frac{\text{Percent TNF-}\alpha\text{ drug treated}}{\text{Percent TNF-}\alpha\text{ in vehicle treated}} \times 100$$

A synergistic effect was observed with the combination of PDE IV inhibitor with corticosteroid which can be seen from below mentioned graph.

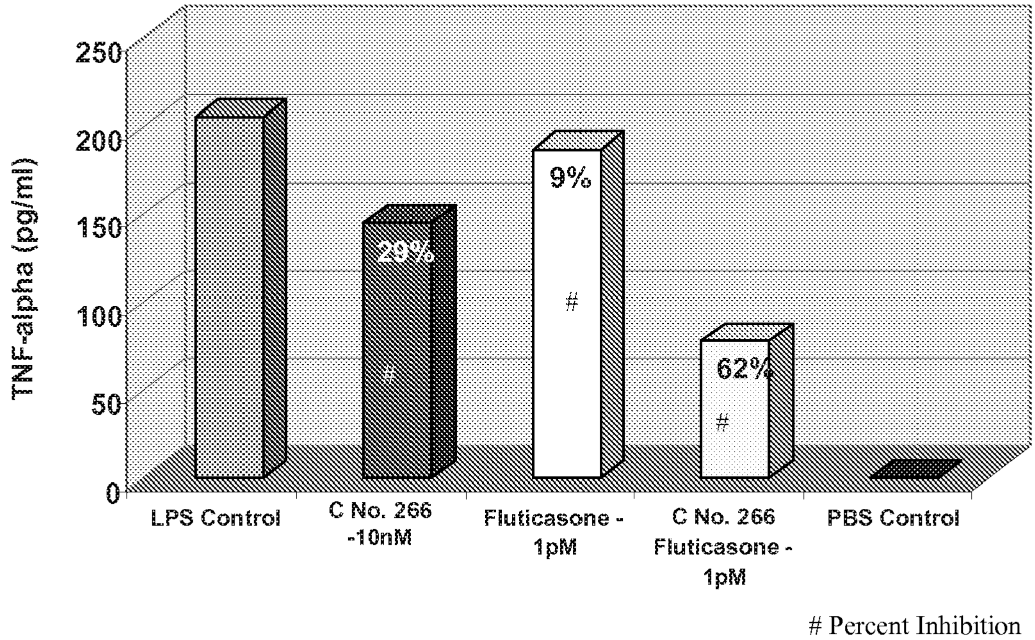


- C No. 266 refers to Compound No. 266
- Combination Index=0.21 indicating synergistic activity
- C No. 266 showed synergy with corticosteroids indicating potential to lower dose

Example 6: *In-vivo* assay to evaluate efficacy of PDE IV inhibitors in combination with corticosteroids

**LPS induced rat neutrophilia model**

Drug treatment:

PDE-4 inhibitor and corticosteroids were instilled intratracheally under anesthesia at different doses, either alone or in combination.
LPS challenge: One hour after drug instillation, (LPS 20 μg/200 μl of PBS) was instilled intratracheally. One group of vehicle treated rats were instilled with 200 μl of phosphate buffered saline (PBS) and served as negative control.
Bronchoalveolar lavage (BAL): Two hours after LPS challenge, bronchoalveolar lavage was performed; the animals were sacrificed using thiopentone sodium (150 mg/kg/i.p.). Trachea was cannulated and BAL was performed using Hank's Buffer salt solution (HBSS) (5 ml x 10 times). The bronchoalveolar lavage fluid was centrifuged at 800 g for 5 min, at 4°C and the pellet was resuspended in 1 ml HBSS. Total leukocyte count was performed in the resuspended sample by using hemocytometer. A cytocentrifuge preparation was made using the resuspended bronchoalveolar lavage fluid on a glass slide, stained with Leishmann's stain and then differential leukocyte counts was performed for computation of neutrophil. Statistical significance of each parameter in different treatment groups was determined with respective to vehicle control group using one-way analysis of variance followed by Dunnett's 't' test for multiple comparison. A p level of ≤ 0.05 was considered to be statistically significant.
Percent inhibition was computed using the following formula.

$$\% \text{ Inhibition} = \frac{Neu_{LPS} - Neu_{TEST}}{Neu_{LPS} - Neu_{PBS}} \times 100$$

Where,
$Neu_{LPS}$ = Neutrophil count in vehicle treated LPS challenged group
$Neu_{TEST}$ = Neutrophil count in group treated with a given dose of test compound
$Neu_{PBS}$ = Percentage of Neutrophil in group challenged with PBS
A synergistic effect was seen with the combination of PDE IV inhibitor with corticosteroids which is apparent from the graph given below:

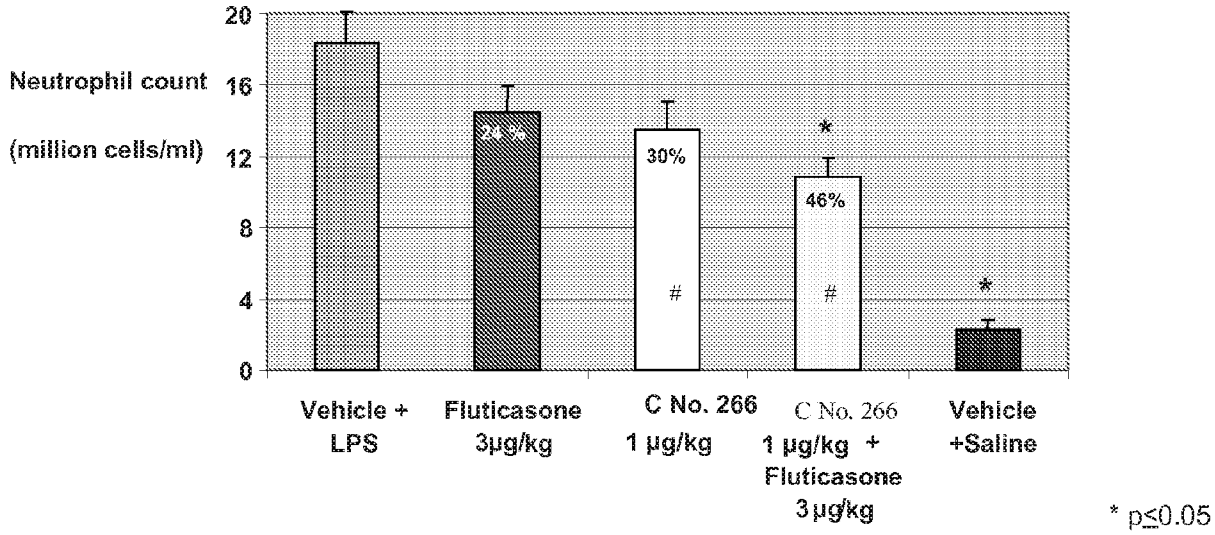


#Percent inhibition values

- C No. 266 refers to Compound No. 266.
- Combination Index = 0.58 indicating synergistic activity
- C No. 266 (PDE IV inhibitor) showed synergy with corticosteroids indicating a potential to lower dose

Example 7: *In-vivo* assay to evaluate efficacy of PDE-IV inhibitors in combination with Muscarinic Receptor Antagonists (MRA)

Drug treatment:

MRA (1ng/kg to 1mg/kg) and PDE-IV inhibitor (1ng/kg to 1mg/kg) were instilled intratracheally under anesthesia either alone or in combination.

Method:

Wistar rats weighing 200±20gm were used in the study. Rats had free access to food and water. On the day of experiment, animals were exposed to lipopolysaccharide (LPS, 100µg/ml) for 40 min. One group of vehicle treated rats was exposed to phosphate buffered saline (PBS) for 40 min. four hours after LPS/PBS exposure, animals were placed inside a whole body plethysmograph (Buxco Electronics, USA) and exposed to PBS or increasing concentration

of acetylcholine (1, 6, 12, 24, 48 and 96 mg/ml) aerosol until Penh values (index of airway resistance) of rats attained 2 times the value (PC-100) seen with PBS alone. The respiratory parameters were recorded online using Biosystem XA software, (Buxco Electronics, USA). Penh, at any chosen dose of acetylcholine was expressed as percent of PBS response and using a nonlinear regression analysis PC100 (2 folds of PBS value) values were computed. Percent inhibition was computed using the following formula.

$$\% \text{ Inhibition} = \frac{PC100_{LPS} - PC100_{TEST}}{PC100_{LPS} - PC100_{PBS}} \times 100$$

Where,
$PC100_{LPS}$ = PC100 in vehicle treated and LPS challenged group
$PC100_{TEST}$ = PC100 in group treated with a given dose of test compound
$PC100_{PBS}$ = PC100 in vehicle treated group challenged with PBS

A synergistic effect was observed with the combination of muscarinic receptor antagonist (MRA) with PDE 4 inhibitor which can be seen from below mentioned graph.

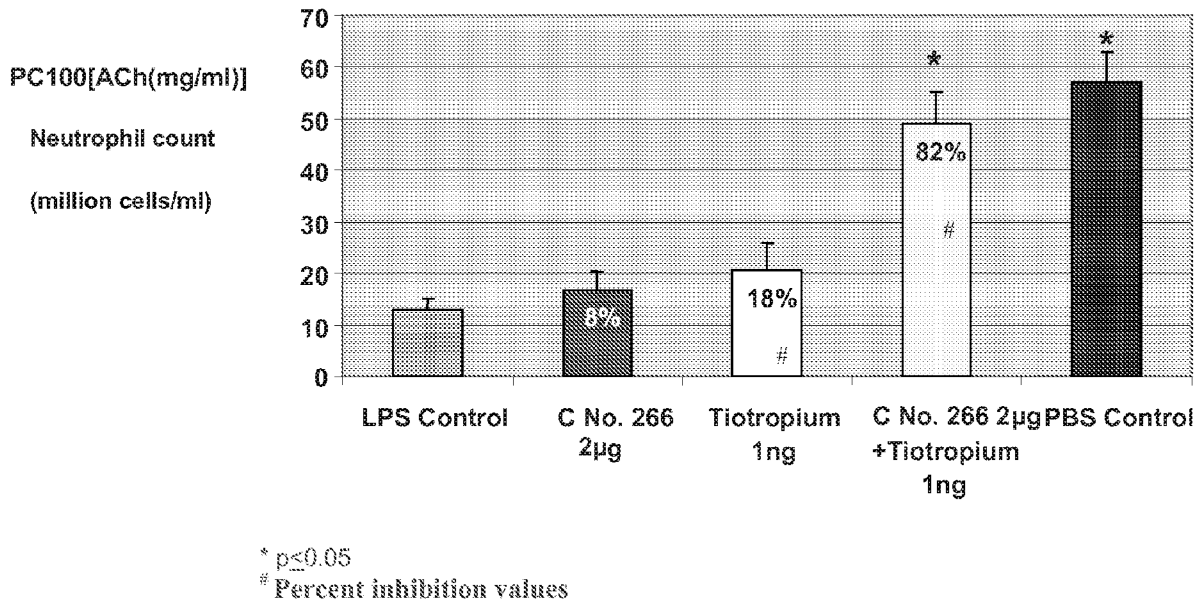


- C No. 266 refers to Compound No. 266.
- Combination index = 0.26 indicating synergistic activity
- C No. 266 (PDE IV inhibitor) showed synergy with Tiotropium (MRA) indicating a potential to lower dose

In another experiment, at the dose of 10 ng/kg, the MRA example Compound No. 104aa was not effective when administered alone, between co-administered with the PDE4 example Compound No. 266 (10 µg/kg), a synergistic effect was observed. Combining MRA Compound No. 104aa with PDE4 inhibitor, Compound No. 266 at these separately ineffective doses demonstrated a statistically significant increase in PC100 value as compared to LPS-control group. The percentage protection exhibited by the combination of both the agents was 82%. The combination index was calculated to be 0.19, indicating a synergistic effect.

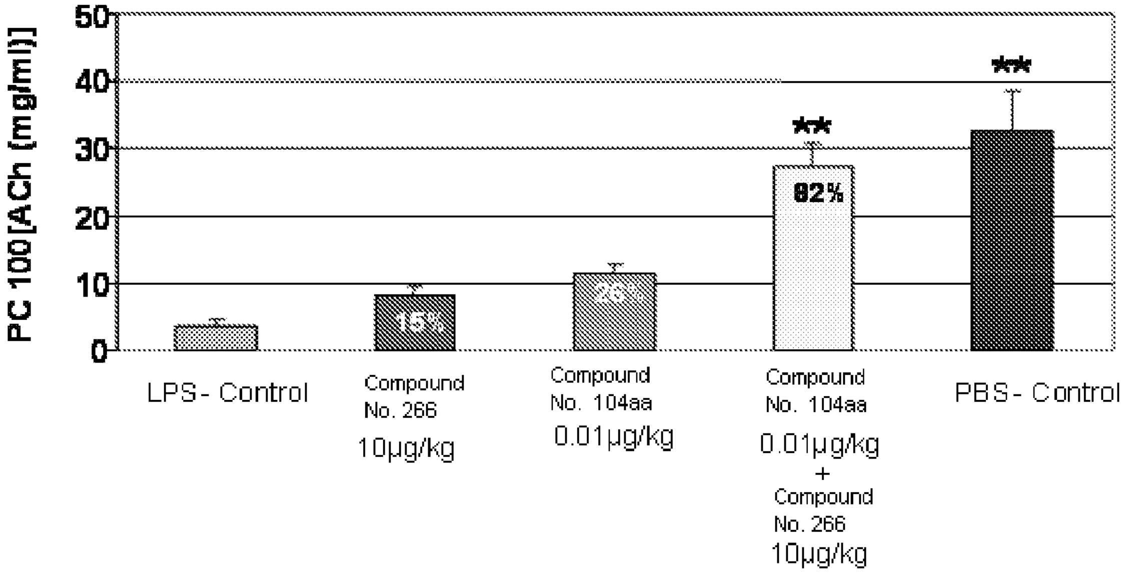


** indicates *p<0.01*

## Claims

1. A pharmaceutical composition comprising one or more phosphodiesterase inhibitors of type IV ("PDE-IV"), and at least one other active ingredients selected from muscarinic receptor antagonists (MRA), β2-agonists, p38 MAP Kinase inhibitors, and corticosteroids and one or more pharmaceutically acceptable excipients wherein the PDE-IV inhibitor is one or more compounds having the structure of Formula Ia or Formula Ib wherein

a. Formula Ia is:

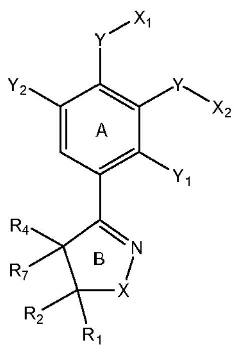

FORMULA Ia

and its pharmaceutically acceptable salts, pharmaceutically acceptable solvates, enantiomers, diastereomers or N-oxides, wherein

**When X is oxygen,**

**$R_1$** is hydrogen, alkyl, heterocyclyl, $-(CH_2)_mC(=O)R_3$, or $(CH_2)_{1-4}OR'$, (wherein m is an integer 0-2, $R_3$ is alkyl, cycloalkyl, heterocyclyl, or optionally substituted $R_p$ or Rq, wherein $R_p$ is heterocyclyl or heteroaryl

ring, wherein the rings are attached to $(CH_2)_mC(=O)$ through N, and $R_q$ is heterocyclyl or heteroaryl ring wherein the rings are attached to $-(CH_2)_mC(=O)$ through C, and wherein R' is alkyl, alkenyl, alkynyl, saturated or unsaturated cycloalkyl, aryl, heterocyclyl or heteroaryl);

**$R_2$** is $(CH_2)_mC(=O)R_3$, $-(CH_2)_{1-4}OR'$, or $C(=O)NR_xR_y$ {where m, $R_3$ and R' are as defined above, and wherein $R_x$ and $R_y$ each independently is hydrogen, alkyl, $C_3$-$C_6$ alkenyl, $C_3$-$C_6$ alkynyl, cycloalkyl, carboxy, $-S(O)_mR_5$ (wherein $R_5$ is hydrogen, alkyl, alkenyl, alkynyl, aryl, cycloalkyl, alkaryl, heteroaryl, heteroarylalkyl, heterocyclyl or heterocyclylalkyl), aryl, alkaryl, heteroaryl, heterocyclyl, heteroarylalkyl, or heterocyclylalkyl}, or **$R_1$** and **$R_2$** together form an optionally substituted cycloalkyl or heterocyclyl ring wherein the optional substituent is oxo, alkyl, alkenyl, alkynyl, halogen, nitro, $-NH_2$, $-NHC(=O)OR_6$, $-C(=O)NR_xR_y$, cyano, hydroxy, alkoxy, or substituted amino (wherein $R_6$ is alkyl, alkenyl, alkynyl, cycloalkyl, alkaryl, heteroarylalkyl or heterocyclylalkyl), with the proviso that if $R_1$ is $-(CH_2)_{1-4}OR'$, then $R_2$ is also $-(CH_2)_{1-4}OR'$, and with the proviso that if $R_1$ is $C(=O)NR_xR_y$, then $R_2$ is also $C(=O)NR_xR_y$;

**$R_4$** is hydrogen; alkyl; $-OR_5$; halogen; $-NH_2$, substituted amino; cyano; carboxy; or - $C(=O)NR_xR_y$ (wherein $R_5$, $R_x$ and $R_y$ are as defined above); or **$R_2$** and **$R_4$** forms an optionally substituted 4-12 membered saturated or unsaturated monocyclic or bicyclic ring system fused to ring B having 0-4 heteroatom(s), wherein the substituents are one or more of alkyl, halogen, hydroxy, alkoxy, $-NH_2$ or substituted amino (wherein $R_3$ and $R_x$ and $R_y$ are as defined above), with the proviso that $R_2$ and $R_4$ together does not form $-CH_2-O-CH_2-O-CH_2-$;

**$R_7$** is hydrogen, alkyl, alkenyl, alkynyl, $-OR_5$, halogen, cyano,$-NH_2$, or substituted amino;

**$X_1$** and **$X_2$** each independently is hydrogen, alkyl, alkaryl, cycloalkyl, alkaryl, alkenyl, cycloalkylalkyl, heterocyclyl, heteroaryl, heterocyclylalkyl, $-(CH_2)_gC(=O)NR_xR_y$, - $(CH_2)_{g1}C(=O)OR_3$ or heteroarylalkyl; wherein $g_1$ is an integer from 1-3 (wherein $R_x$, $R_y$, g and $R_3$ are as defined above);

**Y** is each independently an oxygen atom; a sulphur atom; or -NR (wherein R is hydrogen, acyl, aryl, or alkyl);

**$Y_1$** and **$Y_2$** each independently is hydrogen; alkyl; -OR; -SR; or -NHR (wherein R is as defined above);

wherein any of **$Y_1$** and **$X_2$** & **$X_1$** and **$Y_2$** together optionally form a ring fused with the ring A, the ring containing 3-5 carbon atoms within the ring and having 1-3 heteroatoms, and **$X_1$** and **$X_2$** can together optionally form a ring fused with ring A, the ring containing 3-5 carbon atoms within the ring and having 2-3 heteroatoms, and

**When X is $NR_{7'}$ or S (wherein $R_{7'}$ can be hydrogen, or $C_{1-6}$ alkyl)**

**$R_1$ and $R_2$** is independently alkyl, alkenyl, alkynyl, alkoxy, hydroxy, cyano, nitro, halogen, heteroaryl, heterocyclyl, heteroarylalkyl, heterocyclylalkyl, $NH_2$, substituted amino, carboxy, $-(CH_2)_mC(=O)R_3$, $-C(=O)NR_xR_y$, or $(CH_2)_{1-4}OR'$, {wherein m is an integer 0-2, $R_3$ is alkyl, cycloalkyl, heterocyclyl, or optionally substituted $R_p$ or $R_q$ (wherein $R_p$ is heterocyclyl or heteroaryl ring, wherein the rings are attached to $(CH_2)_mC(=O)$ through N, and $R_q$ is heterocyclyl or heteroaryl ring wherein the rings are attached to $-(CH_2)_mC(=O)$ through C), wherein R' is alkyl, alkenyl, alkynyl, saturated or unsaturated cycloalkyl, aryl, heterocyclyl or heteroaryl, and wherein $R_x$ and $R_y$ each independently is hydrogen, alkyl, $C_3$-$C_6$ alkenyl, $C_3$-$C_6$ alkynyl, cycloalkyl, carboxy, $-S(O)_mR_5$ (wherein $R_5$ is hydrogen, alkyl, alkenyl, alkynyl, aryl, cycloalkyl, alkaryl, heteroaryl, heteroarylalkyl, heterocyclyl or heterocyclylalkyl), aryl, alkaryl, heteroaryl, heterocyclyl, heteroarylalkyl, or heterocyclylalkyl}, or **$R_1$** and **$R_2$** together form an optionally substituted cycloalkyl or heterocyclyl ring wherein the optional substituent is oxo, alkyl, alkenyl, alkynyl, halogen, nitro, $-NH_2$, $-NHC(=O)OR_6$ (wherein $R_6$ is alkyl, alkenyl, alkynyl, cycloalkyl, alkaryl, heteroarylalkyl or heterocyclylalkyl), - $C(=O)NR_xR_y$, cyano, hydroxy, alkoxy, or substituted amino;

**$R_4$** is hydrogen; alkyl; $-OR_5$; halogen; $-NH_2$, substituted amino; cyano; carboxy; or - $C(=O)NR_xR_y$ (wherein $R_5$, $R_x$ and $R_y$ are as defined above); or **$R_2$** and **$R_4$** forms an optionally substituted 4-12 membered saturated or unsaturated monocyclic or bicyclic ring system fused to ring B having 0-4 heteroatom(s), wherein the substituents can be one or more of alkyl, halogen, hydroxy, alkoxy or substituted amino (wherein $R_3$ and $R_x$ and $R_y$ are as defined above), with the proviso that $R_2$ and $R_4$ together does not form $-CH_2-O-CH_2-O-CH_2-$;

**$R_7$** is hydrogen, alkyl, alkenyl, alkynyl, $-OR_5$, halogen, cyano,$-NH_2$, or substituted amino;

**$X_1$** and **$X_2$** each independently is alkyl, cycloalkyl, alkaryl, heteroaryl, heterocyclyl, heteroarylalkyl, or heterocyclylalkyl;

**Y** is each independently an oxygen atom; a sulphur atom; or -NR (wherein R is hydrogen, acyl, aryl, or alkyl);

**$Y_1$** and **$Y_2$** each independently is hydrogen, alkyl, -OR, -SR, or -NHR (wherein R is as defined above);

wherein any of **$Y_1$** and **$X_2$** & **$X_1$** and **$Y_2$** together optionally form a ring fused with the ring A, the ring containing 3-5 carbon atoms within the ring and having 1-3 heteroatoms such as N, O and S;

**$X_1$** and **$X_2$** can together optionally form a cyclic ring fused with the ring A, the ring containing 3-5 carbon atoms within the ring and having 2-3 heteroatoms,

and

b. Formula Ib is:

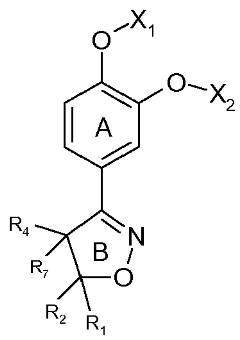

Formula Ib

and its pharmaceutically acceptable salts, pharmaceutically acceptable solvates, enantiomers, diastereomers or N-oxides, wherein

**$R_1$** and **$R_2$** together forms an optionally substituted cycloalkyl or heterocyclyl ring

wherein one or more optional substituent are oxo, alkyl, alkaryl, alkenyl, alkynes, heterocyclylalkyl, cycloalkylalkyl, $-SO_2NR_xR_y$, halogen, $-NH_2$, $-(CH_2)_gC(=O)NR_xR_y$, - $NHC(=O)OR_6$, $-NHC(=O)NR_xR_y$, $-C(=O)OR_3$, $-NHC(=O)R_x$, $-SO_2R_3$, cyano, hydroxy, alkoxy, substituted amino, or $-C(=O)R_3$ (wherein $R_xR_y$ g, $R_6$ and $R_3$ are as defined above);

**$R_4$** is hydrogen; alkyl, hydroxyl, halogen, or carboxy;

**$R_7$** is hydrogen, or alkyl;

**$R_1$** is independently hydrogen or alkyl and **$R_2$** and **$R_4$** forms an optionally substituted 4-12 membered saturated or unsaturated monocyclic or bicyclic ring system fused to ring B having 0-4 heteroatom(s), wherein the substituents is one or more of oxo, alkyl, $-C(=O)OR_3$, $-SO_2R_3$, halogen, hydroxy, alkoxy, $-NH_2$ or substituted amino (wherein $R_3$ is as defined below), with the proviso that $R_2$ and $R_4$ together does not form $-CH_2-O-CH_2-O-CH_2-$;

**$X_1$** and **$X_2$** are hydrogen, alkyl, cycloalkyl, alkaryl, alkenyl, cycloalkylalkyl, heteroaryl, heterocyclyl, heteroarylalkyl, heterocyclylalkyl, $-(CH_2)_gC(=O)NR_xR_y$ or - $(CH_2)_{g1}C(=O)OR_3$ (wherein g can be an integer from 0-3 and $g_1$ can be an integer from 1-3, and $R_x$, $R_y$ and $R_3$ are as defined below);

**$X_1$** and **$X_2$** together can optionally form a cyclic ring fused with the ring A shown in Formula I, the ring containing 3-5 carbon atoms within the ring and having 2-3 heteroatoms N, O or S;

wherein **$R_3$** is alkyl, cycloalkyl or heterocyclyl;

wherein the halogen can be F, Cl, Br, or I; **$R_x$** and **$R_y$** each independently can be hydrogen, alkyl, $C_3$-$C_6$ alkenyl, $C_3$-$C_6$ alkynyl, carboxy, cycloalkyl, $-S(O)_mR_5$, aryl, alkaryl, heteroaryl, heterocyclyl, heteroarylalkyl, and heterocyclylalkyl; m can be an integer between 0-2; **$R_6$** can be alkyl, alkenyl, alkynyl, cycloalkyl, alkaryl, heteroarylalkyl or heterocyclylalkyl; and

wherein **$R_5$** is hydrogen, alkyl, alkenyl, alkynyl, aryl, cycloalkyl, alkaryl, heteroaryl, heteroarylalkyl, heterocyclyl or heterocyclylalkyl.

**2.** The pharmaceutical composition of claim 1, wherein the one or more compounds of Formula Ia or Formula Ib are selected from:

3-[3-(cyclopentyloxy)-4-methoxyphenyl]-1,7-dioxa-2-azaspiro[4.4]non-2-en-6-ol (Compound No. 1),
3-[3-(Cyclopentyloxy)-4-methoxyphenyl]-*N*-(4-fluorophenyl)-1-oxa-2,7-diazaspiro[4.4]non-2-ene-7-carboxamide (Compound No. 2),
3-[3-(Cyclopentyloxy)-4-methoxyphenyl]-7-(tetrahydrofuran-3-ylcarbonyl)-1-oxa-2,7-diazaspiro[4.4]non-2-ene (Compound No. 3),
3-[3-(cyclopentyloxy)-4-methoxyphenyl]-*N*, *N*-dimethyl-1-oxa-2,7-diazaspiro [4.4] non-2-ene-7-sulfonamide (Compound No. 4),
*N*-butyl-3-[3-(cyclopentyloxy)-4-methoxyphenyl]-1-oxa-2,7-diazaspiro[4.4]non-2-ene-7-carboxamide (Compound No. 5),
2- {3-[3-(Cyclopentyloxy)-4-methoxyphenyl]-1-oxa-2,7-diazaspiro[4.4]non-2-en-7-yl}acetamide (Compound No. 6),
Hydrochloride salt of 3-[3-(cyclopentyloxy)-4-methoxyphenyl]-8-prolyl-1-oxa-2,8-diazaspiro[4.5]dec-2-ene (Compound No. 7),
3-[3-(Cyclopentyloxy)-4-methoxyphenyl]-8-(2-morpholin-4-yl-ethyl)-1-oxa-2,8-diazaspiro[4.5]dec-2-ene (Compound No. 8),
*N*-butyl-3-[3-(cyclopentyloxy)-4-methoxyphenyl]-1-oxa-2,8-diazaspiro[4.5]dec-2-ene-8-carboxamide (Compound No. 9),
3-[3-(cyclopentyloxy)-4-methoxyphenyl]-8-(methylsulfonyl)-1-oxa-2,8-diazaspiro[4.5]dec-2-ene (Compound No. 10),
3-[3-(cyclopentyloxy)-4-methoxyphenyl]-1-oxa-2-azaspiro[4.4]non-2-ene (Compound No. 11),
3-[3,4-bis(2-morpholin-4-ylethoxy)phenyl]-1,7-dioxa-2-azaspiro[4.4]non-2-ene (Compound No. 12),
3-(3,4-diisopropoxyphenyl)-1,7-dioxa-2-azaspiro[4.4]non-2-ene (Compound No. 13),
3-[3-methoxy-4-(2-morpholin-4-ylethoxy)phenyl]-1,7-dioxa-2-azaspiro[4.4]non-2-ene (Compound No. 14),
3-[3-(cyclopentyloxy)-4-methoxyphenyl]-1,7-dioxa-2-azaspiro[4.4]non-2-en-8-one (Compound No. 15),
3-[3-(cyclopentyloxy)-4-methoxyphenyl]-1,7-dioxa-2-azaspiro[4.4]non-2-en-8-ol (Compound No. 16),
3-[3-(Cyclopentyloxy)-4-methoxyphenyl]-7-isopropyl-1-oxa-2, 7-diazaspiro [4.4] non-2-ene (Compound No. 17),
3-[3-(cyclopentyloxy)-4-methoxyphenyl]-7-(cyclopropylcarbonyl)-1-oxa-2,7-diazaspiro[4.4]non-2-ene (Compound No. 18),
*N*-benzyl-3-[3-(cyclopentyloxy)-4-methoxyphenyl]-1-oxa-2,7-diazaspiro[4.4]non-2-ene-7-carboxamide (Compound No. 19),
7-acetyl-3-[3-(cyclopentyloxy)-4-methoxyphenyl]-1-oxa-2,7-diazaspiro[4.4]non-2-ene (Compound No. 20),
*Tert*-butyl 3-[3-(cyclopentyloxy)-4-methoxyphenyl]-1-oxa-2,7-diazaspiro[4.5]dec-2-ene-7-carboxylate (Compound No. 21),
*N*-butyl-*N'*-{3-[3-(cyclopentyloxy)-4-methoxyphenyl]-1-oxa-2-azaspiro[4.5]dec-2-en-8-yl}urea (Compound No. 22),
*N*-{3-[3-(cyclopentyloxy)- 4- methoxyphenyl]- 1- oxa- 2- azaspiro [4.5] dec- 2- en- 8- yl}-N-(2- methoxyphenyl) urea (Compound No. 23),
3-[3-(cyclopentyloxy)-4-methoxyphenyl]-1-oxa-2-azaspiro[4.5]dec-2-en-8-ol (Compound No. 24),
Hydrochloride salt of 3-[3-(cyclopentyloxy)-4-methoxyphenyl]-1-oxa-2,7-diazaspiro[4.5]dec-2-ene (Compound No. 25),
3-[3-(cyclopentyloxy)-4-methoxyphenyl]-1-oxa-2-azaspiro[4.5]dec-2-en-8-one (Compound No. 26),
3-[3,4-bis(cyclopentyloxy)phenyl]-1,7-dioxa-2-azaspiro[4.4]non-2-ene (Compound No. 27),
3-[3,4-Bis(cyclopropylmethoxy)phenyl]-1,7-dioxa-2-azaspiro[4.4]non-2-ene (Compound No. 28),
3-[3-(cyclopentyloxy)-4-methoxyphenyl]-1,7-dioxa-2-azaspiro[4.4]non-2-en-4-ol (Compound No. 29),
(R)-3-[3-(cyclopentyloxy)-4-methoxyphenyl]-1,7-dioxa-2-azaspiro[4.4]non-2-ene (Compound No. 30),
3-[3-(Cyclopentyloxy)-4-methoxyphenyl]-8-(cyclopropylmethyl)-1-oxa-2,8-diazaspiro[4.5]dec-2-ene (Compound No. 31),
*N*-Benzyl-3-[3-(cyclopentyloxy)-4-methoxyphenyl]-1-oxa-2,8-diazaspiro[4.5]dec-2-ene-8-carboxamide (Compound No. 32),
3-[3,4-Bis(benzyloxy)phenyl]-1,7-dioxa-2-azaspiro[4.4]non-2-ene (Compound No. 33),
4-(1,7-Dioxa-2-azaspiro[4.4]non-2-en-3-yl)benzene-1,2-diol (Compound No. 34),
7-Amino-3-[3-(cyclopentyloxy)-4-methoxyphenyl]-1-oxa-2,7-diazaspiro[4.4]non-2-en-6-one (Compound No. 35),
Ethyl 8-benzyl-3-[3-(cyclopentyloxy)-4-methoxyphenyl]-1-oxa-2,8-diazaspiro[4.5]dec-2-ene-4-carboxylate (Compound No. 36),
3-[3-(Cyclopentyloxy)-4-methoxyphenyl]-1-oxa-2-azaspiro[4.5]dec-2-ene-4-carboxylic acid (Compound No. 37),
8-Benzyl-3-[3-(cyclopentyloxy)-4-methoxyphenyl]-1-oxa-2,8-diazaspiro[4.5]dec-2-ene (Compound No. 38),
Ethyl 3-[3-(cyclopentyloxy)-4-methoxyphenyl]-1-oxa-2-azaspiro[4.5]dec-2-ene-4-carboxylate (Compound No. 39),
3-[3-(Difluoromethoxy)-4-methoxyphenyl]-1,7-dioxa-2-azaspiro[4.4]non-2-ene (Compound No. 40),
2-(Difluoromethoxy)-5-(1,7-dioxa-2-azaspiro[4.4]non-2-en-3-yl)phenol (Compound No. 41),
3-[3-(Cyclopentyloxy)-4-methoxyphenyl]-1-oxa-2,7-diazaspiro[4.4]non-2-en-6-one (Compound No. 42),
3-[3-(Cyclopentyloxy)- 4- methoxyphenyl]- 3a, 6a- dimethyl- 3a*H*-cyclopenta [*d*] isoxazole- 4,6 (5*H*, 6a*H*)-dione (Compound No. 43),
3-[3-(Cyclopentyloxy)-4-methoxyphenyl]-3a,4,6,6a-tetrahydrofuro[3,4-*d*]isoxazole (Compound No. 44),
3-[3-(Cyclopentyloxy)-4-methoxyphenyl]-6,6a-dihydrofuro[3,4-*d*]isoxazol-4(3a*H*)-one (Compound No. 45),
*Tert*-butyl [({3-[3-(cyclopentyloxy)-4-methoxyphenyl]-1-oxa-2-azaspiro[4.5]dec-2-en-8-yl}amino)carbonyl]carbamate (Compound No. 46),
*N*-{3-[3-(Cyclopentyloxy)- 4- methoxyphenyl]- 1- oxa- 2- azaspiro [4.5] dec- 2- en- 8- yl} cyclopentanecarboxamide (Compound No. 47),
8-Acetyl-3-[3-(cyclopentyloxy)-4-methoxyphenyl]-1-oxa-2,8-diazaspiro[4.5]dec-2-ene (Compound No. 48),

8-(Cyclopentylcarbonyl)-3-[3-(cyclopentyloxy)-4-methoxyphenyl]-1-oxa-2,8-diazaspiro[4.5]dec-2-ene (Compound No. 49),
3-[3-(Cyclopentyloxy)-4-methoxyphenyl]-8-(2-piperidin-1-ylethyl)-1-oxa-2,8-diazaspiro[4.5]dec-2-ene (Compound No. 50),
3-(2,3-Dihydro-1,4-benzodioxin-6-yl)-1,7-dioxa-2-azaspiro[4.4]non-2-ene (Compound No. 51),
3-[3-(Cyclopentyloxy)-4-methoxyphenyl]-1,8-dioxa-2-azaspiro[4.5]dec-2-ene (Compound No. 52),
3-[3-(Cyclopentyloxy)-4-methoxyphenyl]-3a*H*-cyclopenta[*d*]isoxazole-4,6(5*H*,6a*H*)-dione (Compound No. 53),
3-[3-(Cyclopentyloxy)-4-methoxyphenyl]-8-ethyl-1-oxa-2,8-diazaspiro[4.5]dec-2-ene (Compound No. 54),
3-[3-(Cyclopentyloxy)-4-methoxyphenyl]-8-vinyl-1-oxa-2-azaspiro[4.5]dec-2-en-8-ol (Compound No. 55),
3-[3-(Cyclopentyloxy)-4-methoxyphenyl]-3a,4,5,6,7,7a-hexahydro-1,2-benzisoxazole (Compound No. 56),
3-[3-(Cyclopentyloxy)-4-methoxyphenyl]-4,5,6,6a-tetrahydro-3a*H*-cyclopenta[*d*]isoxazole (Compound No. 57),
*N*-{3-[3-(Cyclopentyloxy)-4-methoxyphenyl]-1-oxa-2-azaspiro[4.5]dec-2-en-8-yl}methanesulfonamide (Compound No. 58),
3-[3-(Cyclopentyloxy)-4-methoxyphenyl]-8-methyl-1-oxa-2-azaspiro[4.5]dec-2-en-8-ol (Compound No. 59),
3-[3-(Allyloxy)-4-methoxyphenyl]-1,7-dioxa-2-azaspiro[4.4]non-2-ene (Compound No. 60),
3-[3-(2-Chloroethoxy)-4-methoxyphenyl]-1,7-dioxa-2-azaspiro[4.4]non-2-ene (Compound No. 61),
2-(Cyclopentyloxy)-4-(1,7-dioxa-2-azaspiro[4.4]non-2-en-3-yl)phenol (Compound No. 62),
3-(4-Butoxy-3-isobutoxyphenyl)-1,7-dioxa-2-azaspiro[4.4]non-2-ene (Compound No. 63),
3-(3-Isobutoxy-4-propoxyphenyl)-1,7-dioxa-2-azaspiro[4.4]non-2-ene (Compound No. 64),
3-[3-Butoxy-4-(cyclopropylmethoxy)phenyl]-1,7-dioxa-2-azaspiro[4.4]non-2-ene (Compound No. 65),
3-(3-Butoxy-4-ethoxyphenyl)-1,7-dioxa-2-azaspiro[4.4]non-2-ene (Compound No. 66),
3-[3-Butoxy-4-(cyclohexyloxy)phenyl]-1,7-dioxa-2-azaspiro[4.4]non-2-ene (Compound No. 67),
3-[3-(Cyclohexylmethoxy)-4-ethoxyphenyl]-1,7-dioxa-2-azaspiro[4.4]non-2-ene (Compound No. 68),
3-[3-(Cyclohexylmethoxy)-4-isopropoxyphenyl]-1,7-dioxa-2-azaspiro[4.4]non-2-ene (Compound No. 69),
3-[4-Butoxy-3-(cyclohexylmethoxy)phenyl]-1,7-dioxa-2-azaspiro[4.4]non-2-ene (Compound No. 70),
3-(4-Isobutoxy-3-isopropoxyphenyl)-1,7-dioxa-2-azaspiro[4.4]non-2-ene (Compound No. 71),
3-(4-Butoxy-3-isopropoxyphenyl)-1,7-dioxa-2-azaspiro[4.4]non-2-ene (Compound No. 72),
3-[4-(Cyclohexylmethoxy)-3-isopropoxyphenyl]-1,7-dioxa-2-azaspiro[4.4]non-2-ene (Compound No. 73),
3-[3-Isopropoxy-4-(2-morpholin-4-ylethoxy)phenyl]-1,7-dioxa-2-azaspiro[4.4]non-2-ene (Compound No. 74),
3-[3-(Cyclopropylmethoxy)-4-isopropoxyphenyl]-1,7-dioxa-2-azaspiro[4.4]non-2-ene (Compound No. 75),
3-[3-(Cyclopropylmethoxy)-4-(2-morpholin-4-ylethoxy)phenyl]-1,7-dioxa-2-azaspiro[4.4]non-2-ene (Compound No. 76),
3-[4-Butoxy-3-(cyclopropylmethoxy)phenyl]-1,7-dioxa-2-azaspiro[4.4]non-2-ene (Compound No. 77),
3-[3-(Cyclopropylmethoxy)-4-isopropoxyphenyl]-1,7-dioxa-2-azaspiro[4.4]non-2-ene (Compound No. 78),
3-(3-Isobutoxy-4-isopropoxyphenyl)-1,7-dioxa-2-azaspiro[4.4]non-2-ene (Compound No. 79),
3-[4-(Cyclopropylmethoxy)-3-isobutoxyphenyl]-1,7-dioxa-2-azaspiro[4.4]non-2-ene (Compound No. 80),
3-[4-(cyclohexyloxy)-3-(cyclopentyloxy)phenyl]-1,7-dioxa-2-azaspiro[4.4]non-2-ene (Compound No. 81),
3-[4-(Cyclohexylmethoxy)-3-(cyclopentyloxy)phenyl]-1,7-dioxa-2-azaspiro[4.4]non-2-ene (Compound No. 82),
3-[4-(Cyclopropylmethoxy)-3-(cyclopentyloxy)phenyl]-1,7-dioxa-2-azaspiro[4.4]non-2-ene (Compound No. 83),
3-[3-(Cyclopentyloxy)-4-isobutoxyphenyl]-1,7-dioxa-2-azaspiro[4.4]non-2-ene (Compound No. 84),
3-[3-(Cyclopentyloxy)-4-ethoxyphenyl]-1,7-dioxa-2-azaspiro[4.4]non-2-ene (Compound No. 85),
3-[3-(Cyclopropylmethoxy)-4-ethoxyphenyl]-1,7-dioxa-2-azaspiro[4.4]non-2-ene (Compound No. 86),
3-[4-(Cyclopentyloxy)-3-isobutoxyphenyl]-1,7-dioxa-2-azaspiro[4.4]non-2-ene (Compound No. 87),
3-[3-Isopropoxy-4-(2-morpholin-4-ylethoxy)phenyl]-1,7-dioxa-2-azaspiro[4.4]non-2-ene (Compound No. 88),
3-(4-Ethoxy-3-isobutoxyphenyl)-1,7-dioxa-2-azaspiro[4.4]non-2-ene (Compound No. 89),
3-[3-(Cyclopentyloxy)-4-propoxyphenyl]-1,7-dioxa-2-azaspiro[4.4]non-2-ene (Compound No. 90),
3-[4-Butoxy-3-(cyclopentyloxy)phenyl]-1,7-dioxa-2-azaspiro[4.4]non-2-ene (Compound No. 91),
3-[3-(Cyclopentyloxy)-4-isopropoxyphenyl]-1,7-dioxa-2-azaspiro[4.4]non-2-ene (Compound No. 92),
3-[3-(Cyclopentyloxy)-4-(cycloheptyloxy)phenyl]-1,7-dioxa-2-azaspiro[4.4]non-2-ene (Compound No. 93),
3-[3-(Cyclopentyloxy)-4-(2-morpholin-4-ylethoxy)phenyl]-1,7-dioxa-2-azaspiro[4.4]non-2-ene (Compound No. 94),
3-[4-(Cyclohexylmethoxy)-3-isobutoxyphenyl]-1,7-dioxa-2-azaspiro[4.4]non-2-ene (Compound No. 95),
3-[4-(Cyclohexylmethoxy)-3-(cyclopropylmethoxy)phenyl]-1,7-dioxa-2-azaspiro[4.4]non-2-ene (Compound No. 96),
3-[3-(Cyclopropylmethoxy)-4-propoxyphenyl]-1,7-dioxa-2-azaspiro[4.4]non-2-ene (Compound No. 97),
3-[4-(Cyclopentyloxy)-3-(cyclopropylmethoxy)phenyl]-1,7-dioxa-2-azaspiro[4.4]non-2-ene (Compound No. 98),
3-[4-(Cyclopropylmethoxy)-3-isopropoxyphenyl]-1,7-dioxa-2-azaspiro[4.4]non-2-ene (Compound No. 99),
3-[4-(Cyclopentyloxy)-3-isopropoxyphenyl]-1,7-dioxa-2-azaspiro[4.4]non-2-ene (Compound No. 100),

3-(3-Isopropoxy-4-propoxyphenyl)-1,7-dioxa-2-azaspiro[4.4]non-2-ene (Compound No. 101),
3-(4-Ethoxy-3-isopropoxyphenyl)-1,7-dioxa-2-azaspiro[4.4]non-2-ene (Compound No. 102),
3-[3-Butoxy-4-(2-morpholin-4-ylethoxy)phenyl]-1,7-dioxa-2-azaspiro[4.4]non-2-ene (Compound No. 103),
3-[3-Butoxy-4-(cyclopentyloxy)phenyl]-1,7-dioxa-2-azaspiro[4.4]non-2-ene (Compound No. 104),
3-(3-Butoxy-4-propoxyphenyl)-1,7-dioxa-2-azaspiro[4.4]non-2-ene (Compound No. 105),
3-(3-Butoxy-4-isopropoxyphenyl)-1,7-dioxa-2-azaspiro[4.4]non-2-ene (Compound No. 106),
3-[3-(Cyclohexylmethoxy)-4-propoxyphenyl]-1,7-dioxa-2-azaspiro[4.4]non-2-ene (Compound No. 107),
3-[3-(Cyclohexylmethoxy)-4-isobutoxyphenyl]-1,7-dioxa-2-azaspiro[4.4]non-2-ene (Compound No. 108),
3-[3-(Cyclohexylmethoxy)-4-(cyclopentyloxy)phenyl]-1,7-dioxa-2-azaspiro[4.4]non-2-ene (Compound No. 109),
3-[3-(Cyclohexylmethoxy)-4-(cyclopropylmethoxy)phenyl]-1,7-dioxa-2-azaspiro[4.4]non-2-ene (Compound No. 110),
3-[4-(Cyclohexylmethoxy)-3-propoxyphenyl]-1,7-dioxa-2-azaspiro[4.4]non-2-ene (Compound No. 111),
3-[4-(Cyclopropylmethoxy)-3-propoxyphenyl]-1,7-dioxa-2-azaspiro[4.4]non-2-ene (Compound No. 112),
3-[4-(Cyclopentyloxy)-3-propoxyphenyl]-1,7-dioxa-2-azaspiro[4.4]non-2-ene (Compound No. 113),
3-[4-(3-Isobutoxy)-3-propoxyphenyl]-1,7-dioxa-2-azaspiro[4.4]non-2-ene (Compound No. 114),
3-[3-(Cycloheptyloxy)-4-(cyclopropylmethoxy)phenyl]-1,7-dioxa-2-azaspiro[4.4]non-2-ene (Compound No. 115),
3-[3-(Cycloheptyloxy)-4-propoxyphenyl]-1,7-dioxa-2-azaspiro[4.4]non-2-ene (Compound No. 116),
3-[4-Butoxy-3-(cycloheptyloxy)phenyl]-1,7-dioxa-2-azaspiro[4.4]non-2-ene (Compound No. 117),
3-[3-(Cycloheptyloxy)-4-isopropoxyphenyl]-1,7-dioxa-2-azaspiro[4.4]non-2-ene (Compound No. 118),
3-[3-(Cycloheptyloxy)-4-(cyclopentyloxy)phenyl]-1,7-dioxa-2-azaspiro[4.4]non-2-ene (Compound No. 119),
3-(3-Ethoxy-4-propoxyphenyl)-1,7-dioxa-2-azaspiro[4.4]non-2-ene (Compound No. 120),
3-[4-(Cycloheptyloxy)-3-ethoxyphenyl]-1,7-dioxa-2-azaspiro[4.4]non-2-ene (Compound No. 121),
3-[4-(Cyclopropylmethoxy)-3-ethoxyphenyl]-1,7-dioxa-2-azaspiro[4.4]non-2-ene (Compound No. 122),
3-[4-(Cyclohexylmethoxy)-3-ethoxyphenyl]-1,7-dioxa-2-azaspiro[4.4]non-2-ene (Compound No. 123),
(S)-3-[3-(cyclopentyloxy)-4-methoxyphenyl]-1,7-dioxa-2-azaspiro[4.4]non-2-ene (Compound No. 124),
3-(3-Butoxy-4-isobutoxyphenyl)-1,7-dioxa-2-azaspiro[4.4]non-2-ene (Compound No. 125),
3-(3-Ethoxy-4-isopropoxyphenyl)-1,7-dioxa-2-azaspiro[4.4]non-2-ene (Compound No. 126),
3-[4-(Cyclopentyloxy)-3-ethoxyphenyl]-1,7-dioxa-2-azaspiro[4.4]non-2-ene (Compound No. 127),
3-(4-Butoxy-3-ethoxyphenyl)-1,7-dioxa-2-azaspiro[4.4]non-2-ene (Compound No. 128),
3-(3-Ethoxy-4-isobutoxyphenyl)-1,7-dioxa-2-azaspiro[4.4]non-2-ene (Compound No. 129),
3-[3-(Cycloheptyloxy)-4-isobutoxyphenyl]-1,7-dioxa-2-azaspiro[4.4]non-2-ene (Compound No. 130),
3-[3-(Cycloheptyloxy)-4-(cyclopentyloxy)phenyl]-1,7-dioxa-2-azaspiro[4.4]non-2-ene (Compound No. 131),
3-[3-(Cycloheptyloxy)-4-ethoxyphenyl]-1,7-dioxa-2-azaspiro[4.4]non-2-ene (Compound No. 132),
3-(4-Butoxy-3-propoxyphenyl)-1,7-dioxa-2-azaspiro[4.4]non-2-ene (Compound No. 133),
3-(4-Ethoxy-3-propoxyphenyl)-1,7-dioxa-2-azaspiro[4.4]non-2-ene (Compound No. 134),
3-[4-(Morpholin-4-ylethoxy)-3-propoxyphenyl]-1,7-dioxa-2-azaspiro[4.4]non-2-ene (Compound No. 135),
3-(4-Isopropoxy-3-propoxyphenyl)-1,7-dioxa-2-azaspiro[4.4]non-2-ene (Compound No. 136),
2-[5-(1,7-Dioxa-2-azaspiro[4.4]non-2-en-3-yl)-2-methoxyphenoxy]cyclopentanol (Compound No. 137),
*N*-{3-[3-(Cyclopentyloxy)-4-methoxyphenyl]-1-oxa-2-azaspiro[4.5]dec-2-en-8-yl}-2-fluorobenzamide (Compound No. 138),
*N*-{3-[3-(cyclopentyloxy)-4-methoxyphenyl]-1-oxa-2-azaspiro[4.5]dec-2-en-8-yl}benzamide (Compound No. 139),
3-[3-(Cyclopentyloxy)-4-methoxyphenyl]-4,5,6,6a-tetrahydro-3a*H*-pyrrolo[3,4-*d*]isoxazole (Compound No. 140),
7-(Cyclopentylcarbonyl)-3-[3-(cyclopentyloxy)-4-methoxyphenyl]-1-oxa-2,7-diazaspiro[4.5]dec-2-ene (Compound No. 141),
*Tert*-butyl 3-[3-(cyclopentyloxy)-4-methoxyphenyl]-3a,4,6,6a-tetrahydro-5*H*-pyrrolo[3,4-*d*]isoxazole-5-carboxylate (Compound No. 142),
3-[3-(Cyclopentyloxy)-4-methoxyphenyl]-1-oxa-2,8-diazaspiro[4.5]dec-2-ene-8-carboxamide (Compound No. 143),
*N-B*utyl-3-[3-(cyclopentyloxy)-4-methoxyphenyl]-1-oxa-2,7-diazaspiro[4.5]dec-2-ene-7-carboxamide (Compound No. 144),
3-[3-(Cyclopentyloxy)-4-methoxyphenyl]-7-(methylsulfonyl)-1-oxa-2,7-diazaspiro[4.5]dec-2-ene (Compound No. 145),
3-[4-Methoxy-3-(pyridin-3-ylmethoxy)phenyl]-1,7-dioxa-2-azaspiro[4.4]non-2-ene (Compound No. 146),
5-Acetyl-3-[3-(cyclopentyloxy)-4-methoxyphenyl]-4,5,6,6a-tetrahydro-3a*H*-pyrrolo[3,4-*d*]isoxazole (Compound

No. 147),
3-[3-(Cyclopentyloxy)-4-methoxyphenyl]-5-(methylsulfonyl)-4,5,6,6a-tetrahydro-3a*H*-pyrrolo[3,4-*d*]isoxazole (Compound No. 148),
4-Bromo-3-[3-(cyclopentyloxy)-4-methoxyphenyl]-1,7-dioxa-2-azaspiro[4.4]non-2-ene (Compound No. 149),
3-[3-(Cyclopentyloxy)-4-methoxyphenyl]-3a,5,6,7a-tetrahydro-1,2-benzisoxazol-7(4*H*)-one (Compound No. 150),
3-[4-(Difluoromethoxy)-3-(2,3-dihydro-1*H*-inden-2-yloxy)phenyl]-1,7-dioxa-2-azaspiro[4.4]non-2-ene (Compound No. 151),
3-[4-(Cyclopentyloxy)-3-(2,3-dihydro-1H-inden-2-yloxy)phenyl]-1,7-dioxa-2-azaspiro[4.4]non-2-ene (Compound No. 152),
3-[4-Butoxy-3-(2,3-dihydro-1*H*-inden-2-yloxy)phenyl]-1,7-dioxa-2-azaspiro[4.4]non-2-ene (Compound No. 153),
3-(3-{[3-(Benzyloxy)cyclopentyl]oxy}-4-methoxyphenyl)-1,7-dioxa-2-azaspiro[4.4]non-2-ene (Compound No. 154),
7-Acetyl-3-[3-(cyclopentyloxy)-4-methoxyphenyl]-1-oxa-2,7-diazaspiro[4.5]dec-2-ene (Compound No. 155),
3-[4-Methoxy-3-(pyridin-2-ylmethoxy)phenyl]-1,7-dioxa-2-azaspiro[4.4]non-2-ene (Compound No. 156),
3-[3-(2,3-Dihydro-1*H*-inden-2-yloxy)-4-ethoxyphenyl]-1,7-dioxa-2-azaspiro[4.4]non-2-ene (Compound No. 157),
3-[3-(2,3-Dihydro-1*H*-inden-2-yloxy)-4-propoxyphenyl]-1,7-dioxa-2-azaspiro[4.4]non-2-ene (Compound No. 158),
3-[4-(Cyclopropylmethoxy)-3-(2,3-dihydro-1*H*-inden-2-yloxy) phenyl]-1,7-dioxa-2-azaspiro [4.4] non-2-ene (Compound No. 159),
3-[3-(2,3-Dihydro-1*H*-inden-2-yloxy)-4-isopropoxyphenyl]-1,7-dioxa-2-azaspiro[4.4]non-2-ene (Compound No. 160),
2-(2,3-Dihydro-1*H*-inden-2-yloxy)-4-(1,7-dioxa-2-azaspiro[4.4]non-2-en-3-yl)phenol (Compound No. 161),
*N*-cyclopropyl-2-[5-(1,7-dioxa-2-azaspiro[4.4]non-2-en-3-yl)-2-methoxyphenoxy] acetamide (Compound No. 162),
Hydrochloride salt of 3-[4-methoxy-3-(piperidin-3-yloxy)phenyl]-1,7-dioxa-2-azaspiro[4.4]non-2-ene (Compound No. 163),
2-[5-(1,7-Dioxa-2-azaspiro[4.4]non-2-en-3-yl)-2-methoxyphenoxy]acetamide (Compound No. 164),
Ethyl [5-(1,7-dioxa-2-azaspiro[4.4]non-2-en-3-yl)-2-methoxyphenoxy]acetate (Compound No. 165),
[5-(1,7-Dioxa-2-azaspiro[4.4]non-2-en-3-yl)-2-methoxyphenoxy]acetonitrile (Compound No. 166),
3-{3-[(2,6-Dichloropyridin-4-yl)methoxy]-4-methoxyphenyl}-1,7-dioxa-2-azaspiro[4.4]non-2-ene (Compound No. 167),
[3-(3-Cyclopentyloxy-4-methoxy phenyl)-5-(4-carboxylic acid tert butylester-piperazin-1-yl-carbonyl)-4,5-dihydroisoxazol-5-yl)-({4-carboxylic-acid- tert butyl ester piperazine-1-yl) ethanone (Compound No. 168),
1-{1-[5-(4-Acetyl-4-phenyl-piperidine-1-carbonyl)-3-(3-cyclopentyloxy-4-methoxyphenyl)-4,5-dihydro-isoxazole-5-yl]-4-acetyl-4-phenyl-piperidin-4-yl]-ethanone (Compound No. 169),
[3-(3-Cyclopentyloxy-4-methoxy-phenyl)-5-(pyrrolidine-1-carbonyl)-4,5-dihydro-isoxazol-5-yl]-pyrrolidin-1-yl-ethanone (Compound No. 170),
[3-(3-Cyclopentyloxy-4-methoxy-phenyl)-5-(piperidine-1-carbonyl)-4,5-dihydro-isoxazol-5-yl]-piperidin-1-yl-ethanone (Compound No. 171),
3-(3-Cyclopentyloxy-4-methoxy phenyl)-5-(pyrrolidin-2-carboxylic acid methyl ester-1-carbonyl)-4,5-dihydro-isoxazol-5-yl)-[{pyrrolidine-2-carboxylic acid methyl ester-5-yl] ethanone ( Compound No. 172),
[5-[4-(4-Chlorophenyl)-4-hydroxy-piperidine-1-carbonyl]-3-(3-cyclopentyloxy-4-methoxy-phenyl)-4,5-dihydro-isoxazol-5-yl]-[4-(4-chlorophenyl)-4-hydroxy-piperidin-1-yl]-ethanone (Compound No. 173),
[3-(3-Cyclopentyloxy-4-methoxy-phenyl)-5-(hydroxymethyl-piperidine-1-carbonyl)-4,5-dihydro-isoxazol-5-yl]-(4-hydroxymethyl-piperidin-1-yl)-ethanone (Compound No. 174),
[5-(5-Benzyl-2,5-diazabicyclo[2.2.1]heptane-2-(carbonyl)-3-(3-cyclopentyloxy-4-methoxy-phenyl]-4,5-dihydro-isoxozol-5-yl]-5-benzyl-2,5-diazabicylo-[2.2.1]hept-2-yl-ethanone ( Compound No. 175),
[3-(3-Cyclopentyloxy-4-methoxy-phenyl)-5-methyl-4,5-dihydro-isoxazol-5-yl]-piperdin-1-yl-methanone (Compound No. 176),
4-[3-(3-Cyclopentyloxy-4-methoxy-phenyl)-5-methyl-4,5-dihydro-isoxazole-5-carbonyl]-piperazine-1-carboxylic acid tert-butyl ester ( Compound No. 177),
1-[3-(3-Cyclopentyloxy-4-methoxy-phenyl)-5-methyl-4,5-dihydro-isoxazole-carbonyl]-pyrrolidin-2-carboxylic acid ( Compound No. 178),
1-[3-(3-Cyclopentyloxy-4-methoxy-phenyl)-5-methyl-4,5-dihydro-isoxazol-5-carbonyl]-pyrrolidine-2-carboxylic acid methyl ester ( Compound No. 179),

[3-(3- Cyclopentyloxy- 4- methoxy- phenyl)- 5- methyl- 4,5- dihydro- isoxazole- 5- yl]-pyrrolidin- 1- yl- methanone ( Compound No. 180),

[1-4]-Bipiperidinyl- 1- yl-[3-(3- cyclopentyloxy- 4- methoxy- phenyl)- 5- methyl- 4-, 5- dihydro- isoxazol- 5- yl]-methanone ( Compound No. 181),

1-[3-(3-Cyclopentyloxy-4-methoxy-phenyl)-5-methyl-4,5-dihydro-isoxazole-5-carbonyl]-4-phenyl-piperidine-4-yl}-ethanone ( Compound No. 182),

[3-(3-Cyclopentyloxy-4-methoxy-phenyl)-5-methyl-4,5-dihydro-isoxazol-5-yl]-(4-methyl-piperazin-1-yl)-methanone ( Compound No. 183),

[3-(3-Cyclopentyloxy-4-methoxy-phenyl)-5-methyl-4,5-dihydro-isoxazol-5-yl]- piperazin-1-yl-methanone (Compound No. 184),

[4-(4-Chloro-phenyl)-4-hydroxy-piperidin-1-yl]-[3-(3-cyclopentyloxy-4-methoxyphenyl)-5-methyl-4,5- dihydroisoxazol-5-yl]-methanone ( Compound No. 185),

{4-[3-(3- Cyclopentyloxy- 4- methoxy- phenyl)- 5- methyl- 4,5- dihydro- isoxazole- 5- carbonyl]-[1,4] diazepan- 1-yl}-[3-(3-cyclopentyloxy-4-methoxy-phenyl)-5-methyl-4,5-dihydro-isoxazol-5-yl]-methanone ( Compound No. 186),

[3-(3-Cyclopentyloxy-4-methoxy-phenyl)-5-methyl-4,5-dihydro-isoxazol-5-yl]-(4-cyclopropylmethyl-piperazin-1-yl)-methanone ( Compound No. 187),

[3-(3- Cyclopentyloxy- 4- methoxy- phenyl)- 5- methyl- 4,5- dihydro- isoxazol- 5- yl]-(4- isobutyl- 1- piperazin- 1-yl)-methanone ( Compound No. 188),

[3- Hydroxymethyl- piperidin- 1- yl]-[3-(3- cyclopentyloxy- 4- methoxy- phenyl)- 5- methyl- 4,5- dihydro- isoxazol- 5-yl]-methanone ( Compound No. 189),

[3-(3-Cyclopentyloxy-4-methoxy-phenyl)-5-methyl-4, 5-dihydro-isoxazol-5-yl]-(4-hydroxy-piperidin-1-yl)-methanone ( Compound No. 190),

(4-Benzyl-piperidin-1-yl)-[3-(3-cyclopentyloxy-4-methoxy-phenyl)-5-methyl-4, 5-dihydro-isoxazol-5-yl]-methanone (Compound No. 191),

1-[3-(3-Cyclopentyloxy-4-methoxy-phenyl)-5-methyl-4, 5-dihydro-isoxazole-5-carbonyl]-piperidin-4-one (Compound No. 192),

[4-(4-Bromophenyl)-4-hydroxy-piperidin-1-yl]-[3-(3-cyclopentyloxy-4-methoxy-phenyl)-5-methyl-4, 5-dihydro-isoxazol-5-yl]-methanone (Compound No. 193),

(5-Benzyl-2, 5-diaza-bicyclo [2.2.1] hept-2-yl- [3-(3-cyclopentyloxy-4-methoxy-phenyl)-5-methyl-4, 5-dihydro-isoxazol-5-yl]-methanone (Compound No. 194),

(4-Benzyl-piperazin-1-yl)-[3-(3-cyclopentyloxy-4-methoxy-phenyl)-5-methyl-4, 5-dihydro-isoxazol-5-yl)-methanone (Compound No. 195),

1-[3-(3-Cyclopentyloxy-4-methoxy-phenyl)-5-methyl-4, 5-dihydro-isoxazole-5-carbonyl]-pyrrolidin-2-carboxylic acid methyl amide (Compound No. 196),

1-[3-(3-Cyclopentyloxy-4-methoxy-phenyl)-5-methyl-4,5-dihydro-isoxazole-5-carbonyl]-pyrrolidine-2-carboxylic acid diethyl amide (Compound No. 197),

[3-(3- Cyclopentyloxy- 4- methoxy- phenyl)- 5- methyl- 4,5- dihydro- isoxazol- 5- yl]-(2- hydroxymethyl- pyrrolidin- 1-yl)-methanone (Compound No. 198),

1-[3-(3-Cyclopentyloxy-4-methoxy-phenyl)-5-methyl-4,5-dihydroisoxazole-5-carbonyl]-piperidine-2-carboxylic acid methyl ester (Compound No. 199),

[3-(3-Cyclopentyloxy-4-methoxy-phenyl)-5-methyl-4,5-dihydro-isoxozole-5-carboxyl]-pyrrolidine-2-carboxylic acid amide (Compound No. 200),

3-[3-(3-Cyclopentyloxy-4-methoxy-phenyl)-5-methyl-4,5-dihydro-isoxazole-5-carbonyl]-bicyclo[2.2.1]heptan-2-one (Compound No. 201),

3-[3-Cyclopentyloxy-4-methoxy-phenyl)-1,7-dioxa-2-aza-spiro[4.4]non-2-en-6-one (Compound No. 202),

3-[3-Cyclopentyloxy-4-methoxy-phenyl)-7-methyl-1-oxa-2,7-diaza-spiro[4.4]non-2-ene-6,9-dione (Compound No. 203),

[3-(3- Cyclopentyloxy- 4- methoxy- phenyl)- 5- methyl- 4,5- dihydro- isoxazol- 5- yl-(2- methoxymethyl- pyrrolidin- 1-yl)-methanone (Compound No. 204),

3-(3-Cyclopentyloxy-4-methoxy-phenyl)-1,7-dioxa-2-aza-spiro [4.4] non-2-ene (Compound No. 205),

3-(3-Cyclopropylmethoxy-4-methoxy-phenyl)-1,7-dioxa-2-aza-spiro[4.4]non-2-ene (Compound No. 206),

3-(4-Difluoromethoxy-3-propoxy-phenyl)-1,7-dioxa-2-aza-spiro[4.4]non-2-ene (Compound No. 207),

3-(4-Difluoro-3-butoxy-phenyl)-1,7-dioxa-2-aza-spiro[4.4]non-2-ene (Compound No. 208),

3-(4-Difluoromethoxy-3-isobutoxy-phenyl)-1,7-dioxa-2-aza-spiro [4.4] non-2-ene (Compound No. 209),

3-(3-Cyclopropylmethoxy-4-difluoromethoxy-phenyl)-1,7-dioxa-2-aza-spiro[4.4]non-2-ene (Compound No. 210),

3-(3-Benzyloxy-4-difluoromethoxy-phenyl)-1,7-dioxa-2-aza-spiro [4.4] non-2-ene (Compound No. 211),

3-(4-Difluoromethoxy-3-cyclopentyloxy-phenyl)-1,7-dioxa-2-aza-spiro[4.4]non-2-ene (Compound No. 212),
3-(3,4-Bis-difluoromethoxy-phenyl)-1,7-dioxa-2-aza-spiro[4.4]non-2-ene (Compound No. 213),
3-(3-Butoxy-4-difluoromethoxy-phenyl)-1,7-dioxa-2-aza-spiro [4,4] non-2-ene (Compound No. 214),
3-[3-(Bicyclo[2.2.1]hept-2-yloxy)-4-difluoromethoxy-phenyl]-1,7-dioxo-2-aza-spiro[4.4]non-2-ene (Compound No. 215),
3-(4-Difluoromethoxy-3-methoxy-phenyl)-1,7-dioxa-2-aza-spiro[4.4]non-2-ene (Compound No. 216),
3-(4-Benzyloxy-3-methoxy-phenyl)-1,7-dioxa-2-aza-spiro[4.4]non-2-ene (Compound No. 217),
3-(3-Cycloheptyloxy-4-difluoromethoxy-phenyl)-1,7-dioxa-2-aza-spiro[4.4]non-2-ene (Compound No. 218),
4-(1,7-Dioxa-2-aza-spiro[4.4]non-2-en-3-yl)-2-methoxy-phenol (Compound No. 219),
3-[3-(indan-2-yloxy)-4-methoxy-phenyl]-1,7-dioxa-2-aza-spiro [4.4] non-2-ene (Compound No. 220),
3-(4-Ethoxy-3-methoxy-phenyl)-1,7-dioxa-2-aza-spiro[4.4]non-2-ene (Compound No. 221),
3-(3-Methoxy-4-propoxy-phenyl)-1,7-dioxa-2-aza-spiro[4.4]non-2-ene (Compound No. 222),
3-(4-Isopropoxy-3-methoxy-phenyl)-1,7-dioxa-2-aza-spiro[4.4]non-2-ene (Compound No. 223),
3-(4-Butoxy-3-methoxy-phenyl)-1,7-dioxa-2-aza-spiro[4.4]non-2-ene (Compound No. 224),
3-(4-Cyclopentyloxy-3-methoxy-phenyl)-1,7-dioxa-2-aza-spiro [4.4] non-2-ene (Compound No. 225),
3-(4-(Isobutoxy-3-methoxy-phenyl)-1,7-dioxa-2-aza-spiro[4.4]non-2-ene (Compound No. 226),
3-(4-Cyclohexyloxy-3-methoxy-phenyl)-1,7-dioxa-2-aza-spiro [4.4] non-2-ene (Compound No. 227),
3-(4-Cyclopropylmethoxy-3-methoxy-phenyl)-1,7-dioxa-2-aza-spiro[4.4]non-2-ene (Compound No. 228),
3-(3,4-Dimethoxy-phenyl)-1,7-dioxa-2-aza-spiro[4.4]non-2-ene (Compound No. 229),
3-(3-Ethoxy-4-methoxy-phenyl)-1,7-dioxa-2-aza-spiro[4.4]non-2-ene (Compound No. 230),
3-(4-Methoxy-3-propoxy-phenyl)-1,7-dioxa-2-aza-spiro[4.4]non-2-ene (Compound No. 231),
3-(3-Isopropoxy-4-methoxy-phenyl)-1,7-dioxa-2-aza-spiro[4.4]non-2-ene (Compound No. 232),
3-(3-Butoxy-4-methoxy-phenyl)-1,7-dioxa-2-aza-spiro[4.4]non-2-ene (Compound No. 233),
3-(3-Isobutoxy-4-methoxy-phenyl)-1,7-dioxa-2-aza-spiro[4.4]non-2-ene (Compound No. 234),
3-[4-Methoxy-3-(3-methyl-butoxy)-phenyl-1,7-dioxa-2-aza-spiro[4.4]non-2-ene (Compound No. 235),
3-(3-Cyclohexyloxy-4-methoxy-phenyl)-1,7-dioxa-2-aza-spiro [4.4] non-2-ene (Compound No. 236),
3-(3-Cycloheptyloxy-4-methoxy-phenyl)-1,7-dioxa-2-aza-spiro [4.4] non-2-ene (Compound No. 237),
3-[4-Methoxy-3-(2-morpholin-4-yl-ethoxy)-phenyl]-1,7-dioxa-2-aza-spiro[4.4]non-2-ene (Compound No. 238),
3-(3-Benzyloxy-4-methoxy-phenyl)-1,7-dioxa-2-aza-spiro[4.4]non-2-ene (Compound No. 239),
5-(1,7-Dioxa-2-aza-spiro[4.4]non-2-en-3-yl)-2-methoxy-phenol (Compound No. 240),
3-(3-Cyclopentyloxy-4-methoxy-phenyl)-1-oxa-2,8-diaza-spiro[4.5]dec-2-ene-8-carboxylic acid isopropyl ester (Compound No. 241),
Hydrochloride salt of 3-(3-cyclopentyloxy-4-methoxy-phenyl)-1-oxa-2,8-diaza-spiro[4.5]dec-2-ene (Compound No. 242),
4-Chloro-N-[3-(3-cyclopentyloxy-4-methoxy-phenyl)-1-oxa-2,8-diaza-spiro[4.5]dec-2-ene-8-carbonyl]-benzene sulfonamide (Compound No. 243),
3-(3-Cyclopentyloxy-4-methoxy-phenyl)-1-oxa-2, 8-diaza-spiro [4.5] dec-2-ene-8-carboxylic acid-(2,6-difluoro-phenyl)-amide (Compound No. 244),
3-(3-Cyclopentyloxy-4-methoxy-phenyl)-1-oxa-2,8-diaza-spiro[4.5]dec-2-ene-8-carboxylic acid-(2,4-dichloro-phenyl)-amide (Compound No. 245),
[3-(3-Cyclopentyloxy-4-methoxy-phenyl)-1-oxa-2-aza-spiro[4.5]dec-2-en-8-yl]-carbamic acid isopropyl ester (Compound No. 246),
Hydrochloride salt of 3-(3-cyclopentyloxy-4-methoxy-phenyl)-1-oxa-2-aza-spiro[4.5]dec-2-en-8-ylamine ( Compound No. 247),
2-[3-(3-Cyclopentyloxy-4-methoxy-phenyl)-1-oxa-2-aza-spiro[4.5]dec-2-en-8-yl]-isoindole-1,3-dione (Compound No. 248),
7-(3-Cyclopentyloxy-4-methoxy-phenyl)-5-oxa-6-aza-spiro[3.4]oct-6-ene (Compound No. 249),
3-(3-Cyclopentyloxy-4-methoxy-phenyl)-1-oxa-2-aza-spiro[4.5]dec-2-ene (Compound No. 250),
3-(3-Cyclopentyloxy-4-methoxy-phenyl)-1-oxa-2,7-diaza-spiro[4.4]non-2-ene-7-carboxylic acid tert-butyl ester (Compound No. 251),
Hydrochloride salt of 3-(3-cyclopentyloxy-4-methoxy-phenyl)-1-oxa-2,7-diaza-spiro[4.4]non-2-ene (Compound No. 252),
3-[3-{[(3*S*)-1-Benzylpyrrolidin-3-yl]oxy}-4-(difluoromethoxy)phenyl]-1,7-dioxa-2-azaspiro[4.4]non-2-ene (Compound No. 253),
3-[2-(Difluoromethoxy)-5-(1,7-dioxa-2-azaspiro[4.4]non-2-en-3-yl)phenoxy]propan-1-ol (Compound No. 254),
[2-(Difluoromethoxy)-5-(1,7-dioxa-2-azaspiro[4.4]non-2-en-3-yl)phenoxy]acetonitrile (Compound No. 255),
4-[(5S or 5R)-1,7-dioxa-2-azaspiro[4.4]non-2-en-3-yl]-2-methoxyphenol (Compound No. 256),
4-[(5R or 5S)-1,7-Dioxa-2-azaspiro[4.4]non-2-en-3-yl]-2-methoxyphenol (Compound No. 257),

5-[(5S or 5R)-1,7-Dioxa-2-azaspiro[4.4]non-2-en-3-yl]-2-methoxyphenol (Compound No. 258),
(5S or 5R)-3-(3,4-Dimethoxyphenyl)-1,7-dioxa-2-azaspiro[4.4]non-2-ene (Compound No. 259),
(5R or 5S)-3-(3,4-Dimethoxyphenyl)-1,7-dioxa-2-azaspiro[4.4]non-2-ene (Compound No. 260),
2-(Benzyloxy)-4-(1,7-dioxa-2-azaspiro[4.4]non-2-en-3-yl)phenol (Compound No. 261),
2-[2-(Difluoromethoxy)-5-(1,7-dioxa-2-azaspiro[4.4]non-2-en-3-yl)phenoxy]ethanol (Compound No. 262),
3-[4-(Difluoromethoxy)-3-ethoxyphenyl]-1,7-dioxa-2-azaspiro[4.4]non-2-ene (Compound No. 263),
3-[3-(Cyclohexyloxy)-4-(difluoromethoxy)phenyl]-1,7-dioxa-2-azaspiro[4.4]non-2-ene (Compound No. 264),
(5R or 5S)-3-[4-(Difluoromethoxy)-3-methoxyphenyl]-1,7-dioxa-2-azaspiro[4.4]non-2-ene (Compound No. 265),
(5S or 5R)-3-[4-(Difluoromethoxy)-3-methoxyphenyl]-1,7-dioxa-2-azaspiro[4.4]non-2-ene (Compound No. 266),
Ethyl [2-(difluoromethoxy)-5-(1,7-dioxa-2-azaspiro[4.4]non-2-en-3-yl)phenoxy]acetate (Compound No. 267),
3-[4-(Difluoromethoxy)-3-(2-morpholin-4-ylethoxy)phenyl]-1,7-dioxa-2-azaspiro[4.4]non-2-ene (Compound No. 268),
2-(Difluoromethoxy)-5-(1,7-dioxa-2-azaspiro[4.4]non-2-en-3-yl)phenyl cyclohexanecarboxylate (Compound No. 269),
5-[2-(Difluoromethoxy)-5-(1,7-dioxa-2-azaspiro[4.4]non-2-en-3-yl)phenoxy]pentanoic acid (Compound No. 270),
3-[3-(2,2,2-Trifluoroethoxy)-4-(difluoromethoxy)phenyl]-1,7-dioxa-2-azaspiro[4.4]non-2-ene (Compound No. 271),
3-[3-(Cyclopentylmethoxy)-4-(difluoromethoxy)phenyl]-1,7-dioxa-2-azaspiro[4.4]non-2-ene (Compound No. 272),
*N*-cyclopropyl-2-[2-(difluoromethoxy)-5-(1,7-dioxa-2-azaspiro[4.4]non-2-en-3-yl)phenoxy]acetamide (Compound No. 273),
2-[2-(Difluoromethoxy)-5-(1,7-dioxa-2-azaspiro[4.4]non-2-en-3-yl)phenoxy]acetamide (Compound No. 274),
2-[2-(Difluoromethoxy)-5-(1,7-dioxa-2-azaspiro[4.4]non-2-en-3-yl)phenoxy]-N methylacetamide (Compound No. 275),
3-[3-(Cyclopentyloxy)-4-(2,2,2-trifluoroethoxy)phenyl]-1,7-dioxa-2-azaspiro[4.4]non-2-ene (Compound No. 276),
2-(Difluoromethoxy)-5-(1,7-dioxa-2-azaspiro[4.4]non-2-en-3-yl)phenyl cyclopropanecarboxylate (Compound No. 277),
2-(Difluoromethoxy)-5-(1,7-dioxa-2-azaspiro[4.4]non-2-en-3-yl)phenyl morpholine-4-carboxylate (Compound No. 278),
2-(Difluoromethoxy)-5-(1,7-dioxa-2-azaspiro[4.4]non-2-en-3-yl)phenyl benzoate (Compound No. 279),
5-[2-(Difluoromethoxy)-5-(1,7-dioxa-2-azaspiro[4.4]non-2-en-3-yl)phenoxy] pentanamide (Compound No. 280),
3-[3-Propoxy-4-(2,2,2-trifluoroethoxy)phenyl]-1,7-dioxa-2-azaspiro[4.4]non-2-ene (Compound No. 281),
3-[3-Isopropoxy-4-(2,2,2-trifluoroethoxy)phenyl]-1,7-dioxa-2-azaspiro[4.4]non-2-ene (Compound No. 282),
3-[3-(Cyclopropylmethoxy)-4-(2,2,2-trifluoroethoxy)phenyl]-1,7-dioxa-2-azaspiro[4.4]non-2-ene (Compound No. 283),
3-[3-(2,3- Dihydro- 1*H*-inden- 2- yloxy)- 4-(2,2,2- trifluoroethoxy) phenyl]- 1,7- dioxa- 2- azaspiro [4.4] non- 2- ene (Compound No. 284),
5-(1,7-Dioxa-2-azaspiro[4.4]non-2-en-3-yl)-2-(2,2,2-trifluoroethoxy)phenol (Compound No. 285),
3-[3-Methoxy-4-(2,2,2-trifluoroethoxy)phenyl]-1,7-dioxa-2-azaspiro[4.4]non-2-ene (Compound No. 286),
3-[3-Ethoxy-4-(2,2,2-trifluoroethoxy)phenyl]-1,7-dioxa-2-azaspiro[4.4]non-2-ene (Compound No. 287),
3-[3-Butoxy-4-(2,2,2-trifluoroethoxy)phenyl]-1,7-dioxa-2-azaspiro[4.4]non-2-ene (Compound No. 288),
3-[3-(Cyclohexylmethoxy)-4-(2,2,2-trifluoroethoxy)phenyl]-1,7-dioxa-2-azaspiro[4.4]non-2-ene (Compound No. 289),
3-{[2-(Difluoromethoxy)-5-(1,7-dioxa-2-azaspiro[4.4]non-2-en-3-yl)phenoxy]methyl} benzonitrile (Compound No. 290),
2-{2-[2-(difluoromethoxy)-5-(1,7-dioxa-2-azaspiro[4.4]non-2-en-3-yl)phenoxy]ethyl}-1*H*-isoindole-1,3(2*H*)-dione (Compound No. 291),
3-[3-(Cyclohexyloxy)-4-(2,2,2-trifluoroethoxy)phenyl]-1,7-dioxa-2-azaspiro[4.4]non-2-ene (Compound No. 292),
Ethyl [5-(1,7-dioxa-2-azaspiro[4.4]non-2-en-3-yl)-2-(2,2,2-trifluoroethoxy) phenoxy]acetate (Compound No. 293),
3-[3-(Cyclohexylmethoxy)-4-(difluoromethoxy)phenyl]-1,7-dioxa-2-azaspiro[4.4]non-2-ene (Compound No. 294),

Tert-butyl [2-(difluoromethoxy)-5-(1,7-dioxa-2-azaspiro[4.4]non-2-en-3-yl)phenoxy]acetate (Compound No. 295),
N-cyclopropyl-2-[5-(1,7-dioxa-2-azaspiro[4.4]non-2-en-3-yl)-2-(2,2,2-trifluoroethoxy) phenoxy]acetamide (Compound No. 296),
2-(Cyclopentyloxy)-4-[(5R or 5S)-1,7-dioxa-2-azaspiro[4.4]non-2-en-3-yl]phenol (Compound No. 297),
2-(Cyclopentyloxy)-4-[(5S or 5R)-1,7-dioxa-2-azaspiro[4.4]non-2-en-3-yl]phenol (Compound No. 298),
N-benzyl-2-[5-(1,7-dioxa-2-azaspiro[4.4]non-2-en-3-yl)-2-(2,2,2-trifluoroethoxy) phenoxy]acetamide (Compound No. 299),
N-Cyclopentyl-2-[5-(1,7-dioxa-2-azaspiro[4.4]non-2-en-3-yl)-2-(2,2,2-trifluoroethoxy) phenoxy]acetamide (Compound No. 300),
*T*ert-butyl 4-[2-(difluoromethoxy)-5-(1,7-dioxa-2-azaspiro[4.4]non-2-en-3-yl)phenoxy] piperidine-1-carboxylate (Compound No. 301),
Hydrochloride salt of 3-[4-(difluoromethoxy)-3-(piperidin-4-yloxy)phenyl]-1,7-dioxa-2-azaspiro[4.4]non-2-ene (Compound No. 302),
3-{3-[(1-Acetylpiperidin-4-yl)oxy]-4-(difluoromethoxy)phenyl}-1,7-dioxa-2-azaspiro [4.4]non-2-ene (Compound No. 303),
*T*ert-butyl (3S)-3-[2-(difluoromethoxy)-5-(1,7-dioxa-2-azaspiro[4.4]non-2-en-3-yl)phenoxy]pyrrolidine-1-carboxylate (Compound No. 304),
*T*ert-butyl (3R)-3-[2-(difluoromethoxy)-5-(1,7-dioxa-2-azaspiro[4.4]non-2-en-3-yl)phenoxy]pyrrolidine-1-carboxylate (Compound No. 305),
Tert-butyl 3-[2-(difluoromethoxy)-5-(1,7-dioxa-2-azaspiro[4.4]non-2-en-3-yl)phenoxy]piperidine-1-carboxylate (Compound No. 306),
*T*ert-butyl (2S)-2- {[2-(difluoromethoxy)-5-(1,7-dioxa-2-azaspiro[4.4]non-2-en-3-yl)phenoxy]methyl}pyrrolidine-1-carboxylate (Compound No. 307),
(5R or 5S)-3-[3-(cyclopentyloxy)-4-(difluoromethoxy)phenyl]-1,7-dioxa-2-azaspiro[4.4]non-2-ene (Compound No. 308),
(5S or 5R)-3-(3-isopropoxy-4-methoxyphenyl)-1,7-dioxa-2-azaspiro[4.4]non-2-ene (Compound No. 309),
(5S or 5R)-3-[3-(Cyclopropylmethoxy)-4-methoxyphenyl]-1,7-dioxa-2-azaspiro[4.4]non-2-ene (Compound No. 310),
2-(Cyclopropylmethoxy)-4-[(5S or 5R)-1,7-dioxa-2-azaspiro[4.4]non-2-en-3-yl]phenol (Compound No. 311),
4-[(5S or 5R)-1,7-Dioxa-2-azaspiro[4.4]non-2-en-3-yl]-2-isopropoxyphenol (Compound No. 312),
(5S or 5R)-3-[3-(cyclopentyloxy)-4-(difluoromethoxy)phenyl]-1,7-dioxa-2-azaspiro[4.4]non-2-ene (Compound No. 313),
(5S or 5R)-3-[3-(Cyclopropylmethoxy)-4-(difluoromethoxy)phenyl]-1,7-dioxa-2-azaspiro[4.4]non-2-ene (Compound No. 314),
(5S or 5R)-3-[4-(difluoromethoxy)-3-isopropoxyphenyl]-1,7-dioxa-2-azaspiro[4.4]non-2-ene (Compound No. 315),
(5R or 5S)-3-[4-(difluoromethoxy)-3-isopropoxyphenyl]-1,7-dioxa-2-azaspiro[4.4]non-2-ene (Compound No. 316),
2-(Cyclopropylmethoxy)-4-[(5R or 5S)-1,7-dioxa-2-azaspiro[4.4]non-2-en-3-yl]phenol (Compound No. 317),
4-[(5R or 5S)-1,7-Dioxa-2-azaspiro[4.4]non-2-en-3-yl]-2-isopropoxyphenol (Compound No. 318),
(5R or 5S)-3-[3-(Cyclopropylmethoxy)-4-(difluoromethoxy)phenyl]-1,7-dioxa-2-azaspiro[4.4]non-2-ene (Compound No. 319),
(5R or 5S)-3-[4-(difluoromethoxy)-3-isopropoxyphenyl]-1,7-dioxa-2-azaspiro[4.4]non-2-ene (Compound No. 320),
Hydrochloride salt of 3-{4-(difluoromethoxy)-3-[(3S)-pyrrolidin-3-yloxy]phenyl}-1,7-dioxa-2-azaspiro[4.4]non-2-ene (Compound No. 321),
Hydrochloride salt of 3- {4-(difluoromethoxy)-3-[(2S)-pyrrolidin-2-ylmethoxy]phenyl} - 1,7-dioxa-2-azaspiro[4.4] non-2-ene (Compound No. 322),
Hydrochloride salt of 3-{4-(difluoromethoxy)-3-[(2*R*)-pyrrolidin-2-ylmethoxy]phenyl}-1,7-dioxa-2-azaspiro[4.4] non-2-ene (Compound No. 323),
3-[4-(Difluoromethoxy)-3-{[(2*R*)-1-propionylpyrrolidin-2-yl]methoxy}phenyl]-1,7-dioxa-2-azaspiro[4.4]non-2-ene (Compound No. 324),
3-[3-{[(2*S*)-1-acetylpyrrolidin-2-yl]methoxy}-4-(difluoromethoxy)phenyl]-1,7-dioxa-2-azaspiro[4.4]non-2-ene (Compound No. 325),
3-[3-{[(3S)-1-benzoylpyrrolidin-3-yl]oxy}-4-(difluoromethoxy)phenyl]-1,7-dioxa-2-azaspiro[4.4]non-2-ene (Compound No. 326),
3-[4-(Difluoromethoxy)-3-{[(3S)-1-propionylpyrrolidin-3-yl]oxy}phenyl]-1,7-dioxa-2-azaspiro[4.4]non-2-ene

(Compound No. 327),
(5S or 5R)-3-[3-(Benzyloxy)-4-(difluoromethoxy)phenyl]-1,7-dioxa-2-azaspiro[4.4]non-2-ene (Compound No. 328),
2-(Benzyloxy)-4-[(5S or 5R)-1,7-dioxa-2-azaspiro[4.4]non-2-en-3-yl]phenol (Compound No. 329),
(5S or 5R)-3-[3-(Benzyloxy)-4-methoxyphenyl]-1,7-dioxa-2-azaspiro[4.4]non-2-ene (Compound No. 330),
3-{4-(Difluoromethoxy)-3-[(1-propionylpiperidin-4-yl)oxy]phenyl}-1,7-dioxa-2-azaspiro[4.4]non-2-ene (Compound No. 331),
3-[4-(Difluoromethoxy)-3-{[1-(4-fluorobenzoyl)piperidin-4-yl]oxy}phenyl]-1,7-dioxa-2-azaspiro[4.4]non-2-ene (Compound No. 332),
3-[3-{[1-(Cyclopropylcarbonyl)piperidin-4-yl]oxy}-4-(difluoromethoxy)phenyl]-1,7-dioxa-2-azaspiro[4.4]non-2-ene (Compound No. 333),
3-[3-{[1-(Cyclopentylcarbonyl)piperidin-4-yl]oxy}-4-(difluoromethoxy)phenyl]-1,7-dioxa-2-azaspiro[4.4]non-2-ene (Compound No. 334),
3-[4-(Difluoromethoxy)-3-({1-[(trifluoromethyl)sulfonyl]piperidin-4-yl}oxy)phenyl]-1,7-dioxa-2-azaspiro[4.4]non-2-ene (Compound No. 335),
3-{3-[(1-Acetylpiperidin-3-yl)oxy]-4-(difluoromethoxy)phenyl}-1,7-dioxa-2-azaspiro[4.4]non-2-ene (Compound No. 336),
3-{4-(Difluoromethoxy)-3-[(1-propionylpiperidin-3-yl)oxy]phenyl}-1,7-dioxa-2-azaspiro[4.4]non-2-ene (Compound No. 337),
3-[4-(Difluoromethoxy)-3-{[1-(4-fluorobenzoyl)piperidin-3-yl]oxy}phenyl]-1,7-dioxa-2-azaspiro[4.4]non-2-ene (Compound No. 338),
3-[3-{[1-(Cyclopropylcarbonyl)piperidin-3-yl]oxy}-4-(difluoromethoxy)phenyl] -1,7-dioxa-2-azaspiro[4.4]non-2-ene (Compound No. 339),
3-[3-{[1-(Cyclopentylcarbonyl)piperidin-3-yl]oxy}-4-(difluoromethoxy)phenyl]-1,7-dioxa-2-azaspiro[4.4]non-2-ene (Compound No. 340),
3-[4-(Difluoromethoxy)-3-{[1-(ethylsulfonyl)piperidin-3-yl]oxy}phenyl]-1,7-dioxa-2-azaspiro[4.4]non-2-ene (Compound No. 341),
3-[3-(Benzyloxy)-4-(2,2,2-trifluoroethoxy)phenyl]-1,7-dioxa-2-azaspiro[4.4]non-2-ene (Compound No. 342),
2-(Difluoromethoxy)-5 - [(5*S or* 5*R*)-1,7-dioxa-2-azaspiro[4.4]non-2-en-3-yl]pheno (Compound No. 343),
5-[(5R or 5S)-1,7-Dioxa-2-azaspiro[4.4]non-2-en-3-yl]-2-methoxyphenol (Compound No. 344).

**3.** The pharmaceutical composition of claim 1 , wherein the one or more muscarinic receptor antagonists (MRA) are selected from tiotropium salts, methantheline, ipratropium, propantheline, dicyclomine, scopolamine, telenzepine, benztropine and atropine.

**4.** The pharmaceutical composition of claim 1, wherein the one or more muscarinic receptor antagonists (MRA) are selected from
2-Cyclopentyl-2-hydroxy-*N*-[2-(1*H*-imidazo1-1-yl)ethyl]-2-phenylacetamide (Compound No. 1aa);
1*H*-Imidazol-1-ylmethyl cyclohexyl(hydroxy)(4-methylphenyl)acetate (Compound No. 2aa);
2-(4-Fluorophenyl)-2-hydroxy-*N*-[2-(1*H*-imidazo1-1-yl)ethyl]-2-phenylacetamide (Compound No. 3aa);
2-Cyclobutyl-2-hydroxy-*N*-[2-(1*H*-imidazol-1-yl)ethyl]-2-phenylacetamide (Compound No. 4aa);
2-Cyclopentyl-2-(4-fluorophenyl)-2-hydroxy-*N*-[2-(1*H*-imidazol-1-yl)ethyl]acetamide (Compound No. 5aa);
2-Hydroxy-*N*-[2-(1*H*-imidazol-1-yl)ethyl]-2,2-diphenylacetamide (Compound No. 6aa);
2-Cyclohexyl-2-hydroxy-*N*-[2-(1*H*-imidazo1-1-yl)ethyl]-2-phenylacetamide (Compound No. 7aa);
2-Cyclopentyl-2-hydroxy-*N*-[2-(2-isopropyl-1*H*-imidazol-1-yl)ethyl]-2-phenylacetamide (Compound No. 8aa);
2-Cyclohexyl-2-hydroxy-*N*-[2-(2-isopropyl-1*H*-imidazo1-1-yl)ethyl]-2-phenylacetamide (Compound No. 9aa);
2-Hydroxy-*N*-[2-(2-isopropyl-1*H*-imidazol-1-yl)ethyl]-2-phenyl-2-pyridin-3-ylacetamide (Compound No. 10aa);
2-(4-Fluorophenyl)-2-hydroxy-*N*-[2-(2-isopropyl-1*H*-imidazol-1-yl)ethyl]-2-phenylacetamide (Compound No. 11 aa);
2-Hydroxy-*N*-[2-(2-isopropyl-1*H*-imidazol-1-yl)ethyl]-2,2-diphenylacetamide (Compound No. 12 aa);
2-(2-Methyl-1*H*-imidazo1-1-yl)ethyl cyclopentyl(hydroxy)phenylacetate (Compound No. 13 aa);
2-(2-Methyl-1*H*-imidazol-1-yl) ethyl (2*R*)-cyclopentyl(hydroxy)phenylacetate (Compound No. 14 aa);
1-Cyclopentyl-1-hydroxy-1-(4-methoxyphenyl)-3-(2-methyl-1*H*-imidazol-1-yl)acetone (Compound No. 15 aa);
(2*R*)-2-cyclopentyl-2-hydroxy-*N*-[2-(2-methyl-1*H*-imidazol-1-yl)ethyl]-2-phenylacetamide (Compound No. 16 aa);
2-Hydroxy-N-[2-(1*H*-imidazo1-1-yl)ethyl]-2-phenyl-2-pyridin-3-ylacetamide (Compound No. 17 aa);
2-Cyclopentyl-2-hydroxy-*N*-[2-(2-methyl-1*H*-imidazol-1-yl)ethyl]-2-phenylacetamide (Compound No. 18 aa);
2-Hydroxy-*N*-[2-(2-methyl-1*H*-imidazol-1-yl)ethyl]-2-phenyl-2-pyridin-3-ylacetamide (Compound No. 19 aa);
2-Cyclohexyl-2-hydroxy-*N*-[2-(2-methyl-1*H*-imidazol-1-yl)ethyl]-2-phenylacetamide (Compound No. 20 aa);
2-(4-Fluorophenyl)-2-hydroxy-*N*-[2-(2-methyl-1*H*-imidazol-1-yl)ethyl]-2-phenylacetamide (Compound No. 21 aa);

2-Cyclopentyl-2-(4-fluorophenyl)-2-hydroxy-*N*-[2-(2-methyl-1*H*-imidazol-1-yl)ethyl]acetamide (Compound No. 22 aa);
2-Cyclobutyl-2-hydroxy-*N*-[2-(2-methyl-1*H*-imidazol-1-yl)ethyl]-2-phenylacetamide (Compound No. 23 aa);
2-Hydroxy-*N*-[2-(2-methyl-1*H*-imidazol-1-yl)ethyl]-2,2-diphenylacetamide (Compound No. 24 aa);
3,3,3-Trifluoro-2-hydroxy-*N*-[2-(2-methyl-1*H*-imidazol-1-yl)ethyl]-2-(4-methylphenyl)propanamide (Compound No. 25 aa);
*N*-[2-(2-methyl-1*H*-imidazol-1-yl)ethyl]-2,2-diphenylacetamide (Compound No. 26 aa);
2-Cyclopentyl-*N*-[2-(2-methyl-1*H*-imidazol-1-yl)ethyl]-2-phenylacetamide (Compound No. 27 aa);
2-Cyclopentyl-2-hydroxy-*N*-[2-(2-methyl-1*H*-imidazol-1-yl)ethyl]-2-(4-methylphenyl)acetamide (Compound No. 28 aa);
2-Cyclohexyl-2-hydroxy-*N*-[2-(2-methyl-1*H*-imidazol-1-yl)ethyl]-2-(4-methylphenyl)acetamide (Compound No. 29 aa);
2-Hydroxy-*N*-[2-(2-methyl-1*H*-imidazol-1-yl)ethyl]-2-(4-methylphenyl)-2-phenylacetamide (Compound No. 30 aa);
2-Cyclopentyl-2-hydroxy-*N*-[3-(1*H*-imidazol-1-yl)propyl]-2-phenylacetamide (Compound No. 31 aa);
2-Cyclohexyl-2-hydroxy-*N*-[3-(1*H*-imidazol-1-yl)propyl]-2-phenylacetamide (Compound No. 32 aa);
2-Cyclopentyl-2-hydroxy-*N*-[3-(2-methyl-1*H*-imidazol-1-yl)propyl]-2-phenylacetamide (Compound No. 33 aa);
2-Cyclohexyl-2-hydroxy-*N*-[3-(2-methyl-1*H*-imidazol-1-yl)propyl]-2-phenylacetamide (Compound No. 34 aa);
(2R)-2-(3,3-Difluorocyclopentyl)-2-hydroxy-*N*-[3-(2-methyl-1H-imidazol-l-yl)propyl]-2-phenylacetamide (Compound No. 35 aa);
2-Cyclopentyl-2-hydroxy-*N*-methyl-*N*-[3-(2-methyl-1*H*-imidazol-1-yl)propyl]-2-phenylacetamide (Compound No. 36 aa);
2-Cyclohexyl-2-hydroxy-*N*-methyl-*N*-[3-(2-methyl-1*H*-imidazol-1-yl)propyl]-2-phenylacetamide (Compound No. 37 aa);
2-Cyclopentyl-2-hydroxy-*N*-[2-(1*H*-imidazo1-1-yl)ethyl]-*N*-methyl-2-phenylacetamide (Compound No. 38 aa);
2-Cyclopentyl-2-hydroxy-*N*-[2-(1*H*-imidazo1-4-yl)ethyl]-2-phenylacetamide (Compound No. 39 aa);
*N*-[2-(1-benzyl-1*H*-imidazol-4-yl)ethyl]-2-cyclopentyl-2-hydroxy-2-(4-methylphenyl)acetamide (Compound No. 40 aa);
2-Cyclopentyl-2-hydroxy-*N*-[2-(1*H*-imidazol-4-yl)ethyl]-2-(4-methylphenyl)acetamide (Compound No. 41 aa);
2-Cyclohexyl-2-hydroxy-*N*-[2-(1*H*-imidazo1-4-yl)ethyl]-2-(4-methylphenyl)acetamide (Compound No. 42 aa);
2-Hydroxy-*N*-[2-(1*H*-imidazol-4-yl)ethyl]-2,2-diphenylacetamide (Compound No. 43 aa);
*N*-[2-(1-benzyl-1*H*-imidazol-4-yl)ethyl]-2-cyclohexyl-2-hydroxy-2-(4-methylphenyl)acetamide (Compound No. 44 aa);
*N*-[2-(1-benzyl-1*H*-imidazol-4-yl)ethyl]-2-hydroxy-2,2-diphenylacetamide (Compound No. 45 aa);
1*H*-imidazol-1-ylmethyl cyclopentyl(hydroxy)phenylacetate (Compound No. 46 aa); 1*H*-imidazol-1-ylmethyl cyclohexyl(hydroxy)phenylacetate (Compound No. 47 aa); 1*H*-imidazol-1-ylmethyl (2R)-cyclopentyl(hydroxy)phenylacetate (Compound No. 48 aa); 1*H*-Imidazol-1-ylmethyl cyclopentyl(hydroxy)(4-methoxyphenyl)acetate (Compound No. 49 aa);
1-Cyclohexyl-1-hydroxy-3-(1*H*-imidazol-1-yl)-1-phenylacetone (Compound No. 50 aa); 1-Cyclohexyl-1-hydroxy-3-(2-methyl-1*H*-imidazol-1-yl)-1-phenylacetone (Compound No. 51 aa);
1-Cyclopentyl-1-hydroxy-3-(2-isopropyl-1*H*-imidazol-1-yl)-1-(4-methoxyphenyl)acetone (Compound No. 52 aa);
1-Cyclohexyl-1-hydroxy-3-(2-isopropyl-1*H*-imidazol-1-yl)-1-phenylacetone (Compound No. 53 aa);
1-Cyclohexyl-1-hydroxy-3-(2-methyl-4,5-dihydro-1*H*-imidazol-1-yl)-1-phenylacetone (Compound No. 54 aa);
1-Cyclopentyl-1-hydroxy-1-(4-methoxyphenyl)-3-(2-methyl-4,5-dihydro-1*H*-imidazol-1-yl)acetone (Compound No. 55 aa);
1-Cyclopentyl-1-hydroxy-3-(1*H*-imidazol-1-yl)-1-(4-methoxyphenyl)acetone (Compound No. 56 aa);
2-Cyclohexyl-2-hydroxy-*N*-[2-(1*H*-imidazol-1-yl)ethyl]-*N*-methyl-2-phenylacetamide (Compound No. 57 aa);
2-Cyclopentyl-2-hydroxy-*N*-[2-(1*H*-imidazo1-1-yl)ethyl]-*N*-methyl-2-phenylacetamide (Compound No. 58 aa);
2-(2-Methyl-1*H*-imidazol-1-yl)ethyl cyclopentyl(phenyl)acetate (Compound No. 59 aa); 2-(2-Methyl-1*H*-imidazol-1-yl)ethyl cyclohexyl(hydroxy)phenylacetate (Compound No. 60 aa);
3-(2-Methyl-1*H*-imidazol-1-yl)propyl cyclopentyl(hydroxy) phenylacetate (Compound No. 61 aa);
3-(2-Methyl-1*H*-imidazol-1-yl)propyl (2*R*)-[(1*R*)-3,3-difluorocyclopentyl](hydroxy) phenylacetate (Compound No. 62 aa);
3-(2-Methyl-1*H*-imidazol-1-yl)propyl 2*R*-2-*(1S or 1R)* (3,3-difluorocyclohexyl) (hydroxy)phenylacetate (Compound No. 63 aa);
2-(2-Isopropyl-1*H*-imidazol-1-yl)ethyl cyclohexyl(hydroxy) phenylacetate (Compound No. 64 aa);
2-(1*H*-Imidazol-1-yl)ethyl cyclopentyl(hydroxy)phenylacetate (Compound No. 65 aa); 2-(2-Methyl-1*H*-imidazol-1-yl) ethyl (2*R*)-[(1*S*)-3,3-difluorocyclopentyl] (hydroxy) phenylacetate (Compound No. 66 aa);
2-(1*H*-Imidazol-1-yl)ethyl cyclohexyl(hydroxy)phenylacetate (Compound No. 67 aa);

2-(2-Isopropyl-1*H*-imidazol-1-yl)ethyl cyclopentyl(hydroxy) phenylacetate (Compound No. 68 aa);
2-(2-Methyl-1*H*-imidazol-1-yl)ethyl (2*R*)-[(1*R*)-3,3-difluorocyclopentyl] (hydroxy) phenylacetate (Compound No. 69 aa);
2-(2-Isopropyl-1*H*-imidazol-1-yl)ethyl cycloheptyl(hydroxy) phenylacetate (Compound No. 70 aa);
2-(2-Methyl-1*H*-imidazol-1-yl)ethyl cycloheptyl(hydroxy)phenylacetate (Compound No. 71 aa);
3-Benzyl-1-(2-{[cycloheptyl(hydroxy) phenylacetyl] oxy}ethyl)-2-isopropyl-1*H*-imidazol-3-ium bromide (Compound No. 72 aa);
3-Benzyl-1-[2-({(2*R*)-2-[(1*S*)-3,3-difluorocyclopentyl]-2-hydroxy-2-phenylacetyl}oxy)ethyl]-2-isopropyl-1*H*-imidazol-3-ium bromide (Compound No. 73 aa);
3-Benzyl-l-[2-({(2*R*)-2-[(1*R*)-3,3-difluorocyclopentyl]-2-hydroxy-2-phenylacetyl}oxy) ethyl]-2-isopropyl- 1*H*-imidazol-3-ium bromide (Compound No. 74 aa);
3-(4-Bromobenzyl)-1-[2-({(2*R*)-2-[(1*R*)-3,3-difluorocyclopentyl]-2-hydroxy-2-phenylacetyl}oxy)ethyl]-2-isopropyl-1*H*-imidazol-3-ium bromide (Compound No.75 aa);
3-Benzyl-1-[2-({(2*R*)-2-[(1*R*)-3,3-difluorocyclopentyl]-2-hydroxy-2-phenylacetyl}oxy)ethyl]-2-methyl-1*H*-imidazol-3-ium bromide (Compound No.76 aa);
3-(4-Bromobenzyl)-1-[2-({(2*R*)-2-[(1*R*)-3,3-difluorocyclopentyl]-2-hydroxy-2-phenylacetyl}oxy) ethyl]-2-methyl-1*H*-imidazol-3-ium bromide (Compound No. 77 aa);
3-(4-Fluorobenzyl-1-[2-({(2*R*)-2-[(1*R*)-3,3-difluorocyclopentyl]-2-hydroxy-2-phenylacetyl}oxy) ethyl]-2-methyl-1*H*-imidazol-3-ium bromide (Compound No. 78 aa);
3-Benzyl-1-(2-{[cycloheptyl(hydroxy)phenylacetyl]oxy}ethyl)-2-methyl-1*H*-imidazol-3-ium bromide (Compound No. 79 aa);
3-(4-Bromobenzyl)-1-(2-{[cycloheptyl(hydroxy)phenylacetyl]oxy}ethyl)-2-methyl-1*H*-imidazol-3-ium bromide (Compound No. 80 aa);
1-[2-({(2*R*)-2-[(1*S*)-3,3-difluorocyclopentyl]-2-hydroxy-2-phenylacetyl}oxy)ethyl]-2-isopropyl-3-methyl-1*H*-imidazol-3-ium iodide (Compound No. 81 aa);
1-[2-({(2*R*)-2-[(1*R*)-3,3-difluorocyclopentyl]-2-hydroxy-2-phenylacetyl}oxy)ethyl]-3-(4-fluorobenzyl)-2-isopropyl-1*H*-imidazol-3-ium bromide (Compound No. 82 aa);
1-(2-{[2-Cyclohexyl-2-hydroxy-2-phenylacetyl]oxy}ethyl)-2-isopropyl-3-methyl-1*H*-imidazol-3-ium iodide (Compound No. 83 aa);
1-(2- {[Cyclopentyl (hydroxy) phenylacetyl]oxy}ethyl)-3-methyl-1*H*-imidazol-3-ium iodide (Compound No. 84 aa);
1-[2-({(2*R*)-2-[(1*S*)-3,3-difluorocyclopentyl]-2-hydroxy-2-phenylacetyl}oxy)ethyl]-2,3-dimethyl-1*H*-imidazol-3-ium iodide (Compound No. 85 aa);
1-[2-({(2*R*)-2-[(1*R*)-3,3-difluorocyclopentyl]-2-hydroxy-2-phenylacetyl}oxy)ethyl]-2,3-dimethyl-1*H*-imidazol-3-ium iodide (Compound No. 86 aa);
1-(2- {[Cyclohexyl(hydroxy) phenylacetyl]oxy}ethyl)-3-methyl-1*H*-imidazol-3-ium iodide (Compound No. 87 aa);
1-(2-{[Cyclopentyl(hydroxy)phenylacetyl]oxy}ethyl)-2-isopropyl-3-methyl-1*H*-imidazol-3-ium iodide (Compound No. 88 aa);
1-(2-{[Cyclopentyl(hydroxy)phenylacetyl]amino}ethyl)-2,3-dimethyl-1*H*-imidazol-3-ium iodide (Compound No. 89 aa);
1-(2-{[Cyclohexyl(hydroxy)phenylacetyl]amino}ethyl)-2-isopropyl-3-methyl-1*H*-imidazol-3-ium iodide (Compound No. 90 aa);
1-(2-{[Cycloheptyl(hydroxy)phenylacetyl]oxy}ethyl)-2,3-dimethyl-1*H*-imidazol-3-ium iodide (Compound No. 91 aa) ;
1-(2-{[Cycloheptyl(hydroxy)phenylacetyl]oxy}ethyl)-2-isopropyl-3-methyl-1*H*-imidazol-3-ium iodide (Compound No. 92 aa);
1-[2-({(2*R*)-2-[(1*R*)-3,3-difluorocyclopentyl]-2-hydroxy-2-phenylacetyl}oxy)ethyl]-2-isopropyl-3-methyl-1*H*-imidazol-3-ium iodide (Compound No. 93 aa);
1-(3-{[(2*R*)-2-(3,3-difluorocyclopentyl)-2-hydroxy-2-phenylacetyl]amino}propyl)-2,3-dimethyl-1*H*-imidazol-3-ium iodide (Compound No. 94 aa);
1-(3-{[Cyclopentyl(hydroxy)phenylacetyl]amino}propyl)-2,3-dimethyl-1*H*-imidazol-3-ium iodide (Compound No. 95 aa);
1-(3-{[Cyclohexyl(hydroxy)phenylacetyl]amino}propyl)-2,3-dimethyl-1*H*-imidazol-3-ium iodide (Compound No. 96 aa);
1-(2-{[Cyclopentyl(hydroxy)phenylacetyl](methyl)amino}ethyl)-3-methyl-1H-imidazol-3-ium iodide (Compound No. 97 aa);
1-(3-{[Cyclopentyl(hydroxy)phenylacetyl](methyl)amino}propyl)-2,3-dimethyl-1*H*-imidazol-3-ium iodide (Compound No. 98 aa);
1-(3-{[Cyclopentyl(hydroxy)phenylacetyl]oxy}propyl)-2,3-dimethyl-1*H*-imidazol-3-ium iodide (Compound No. 99 aa);
1-[3-({(2*R*)-2-[(1*S*)-3,3-Difluorocyclopentyl]-2-hydroxy-2-phenylacetyl}oxy)propyl]-2,3-dimethyl-1*H*-imidazol-3-ium

iodide (Compound No. 100 aa);
3-Benzyl-1-[3-({(2*R*)-2-[(1*S*)-3,3-difluorocyclopentyl]-2-hydroxy-2-phenylacetyl}oxy) propyl]-2-methyl-1*H*-imidazol-3-ium bromide (Compound No. 101 aa);
3-(4-Bromobenzyl)-1-[3-({(2*R*)-2-[(1*S*)-3,3-difluorocyclopentyl]-2-hydroxy-2-phenylacetyl}oxy)propyl]-2-methyl-1*H*-imidazol-3-ium bromide (Compound No. 102 aa);
1-[3-({(2*R*)-2-[(1R)-3,3-difluorocyclopentyl]-2-hydroxy-2-phenylacetyl}oxy)propyl]-2,3-dimethyl-1*H*-imidazol-3-ium iodide (Compound No. 103 aa);
3-Benzyl-l-[3-({(2*R*)-2-[(1*R*)-3,3-difluorocyclopentyl]-2-hydroxy-2-phenylacetyl}oxy) propyl]-2-methyl-1*H*-imidazol-3-ium bromide (Compound No. 104 aa);
3-(4-Bromobenzyl)-1-[3-({(2*R*)-2-[(1*R*)-3,3-difluorocyclopentyl]-2-hydroxy-2-phenylacetyl}oxy)propyl]-2-methyl-1*H*-imidazol-3-ium bromide (Compound No. 105 aa);
1-[3-({(*2*R)-2-[(1*S* or 1*R*)-3,3-difluorocyclohexyl]-2-hydroxy-2-phenylacetyl}oxy)propyl]-2,3-dimethyl-1*H*-imidazol-3-ium bromide (Compound No. 106 aa);
3-Benzyl-1-[3-({(2*R*)-2-[(*1R or 1S*)-3,3-difluorocyclohexyl]-2-hydroxy-2-phenylacetyl}oxy)propyl]-2-methyl-1*H*-imidazol-3-ium bromide (Compound No. 107 aa);
3-(4-Bromobenzyl)-1-[3-({(2R)-2-[(*1R or 1S*)-3,3-difluorocyclohexyl]-2-hydroxy-2-phenylacetyl}oxy)propyl]-2-methyl-1*H*-imidazol-3-ium bromide (Compound No. 108 aa);
1-(2-{[Cyclopentyl(hydroxy)phenylacetyl](methyl)amino}ethyl)-2,3-dimethyl-1*H*-imidazol-3-ium iodide (Compound No. 109 aa);
3-(2-Methyl-1H-imidazol-1-yl)propyl (2*R*)-[(1*S*)-3,3-difluorocyclopentyl] (hydroxy)phenylacetate (Compound No. 110 aa);
1-(2-{[Cyclohexyl(hydroxy) phenyl acetyl]oxy}ethyl)-2,3-dimethyl-1*H*-imidazol-3-ium iodide (Compound No 111 aa);
2-(2-Isopropyl-1*H*-imidazol-1-yl)ethyl (2*R*)-[(1*S*)-3,3-difluorocyclopentyl] (hydroxy)phenylacetate (Compound No. 112 aa);
2-(2-Isopropyl-1*H*-imidazol-1-yl)ethyl (2*R*)-[(1*R*)-3,3-difluorocyclopentyl] (hydroxy)phenylacetate (Compound No. 113 aa);
3-(4-Bromobenzyl)-1-[2-({(2R)-2-[(1R)-3,3-difluorocyclohexyl]-2-hydroxy-2-phenylacetyl}oxy)ethyl]-2-methyl-1H-imidazol-3-ium bromide (Compound No. 114aa);
1-[2-({(2R)-2-[(1R)-3,3-Difluorocyclohexyl]-2-hydroxy-2-phenylacetyl}oxy)ethyl]-2,3-dimethyl-1H-imidazol-3-ium iodide (Compound No. 115aa);
1-(2-{[Cycloheptyl(hydroxy)phenylacetyl]oxy}ethyl)-2-methyl-3-(4-methylbenzyl)-1H-imidazol-3-ium bromide (Compound No. 116aa);
1-[2-({(2R)-2-[(1R)-3,3-Difluorocyclopentyl]-2-hydroxy-2-phenylacetyl}oxy)ethyl]-2-methyl-3-(4-methylbenzyl)-1H-imidazol-3-ium bromide (Compound No. 117aa);
1-[3-({(2R)-2-[(1R)-3,3-Difluorocyclohexyl]-2-hydroxy-2-phenylacetyl}oxy)propyl]-2,3-dimethyl-1H-imidazol-3-ium iodide (Compound No. 118aa);
1,1-(3-{[Cycloheptyl(hydroxy)phenylacetyl]oxy}propyl)-2,3-dimethyl-1H-imidazol-3-ium iodide (Compound No. 119aa);
1-(3-{[Cycloheptyl(hydroxy)phenylacetyl]oxy}propyl)-2-methyl-3-(4-methylbenzyl)-1H-imidazol-3-ium bromide (Compound No. 120aa);
3-(4-Bromobenzyl)-1-(3-{[cycloheptyl(hydroxy)phenylacetyl]oxy}propyl)-2-methyl-1H-imidazol-3-ium bromide (Compound No. 121aa);
3-(4-Bromobenzyl)-1-[3-({(2R)-2-[(1R)-3,3-difluorocyclohexyl]-2-hydroxy-2-phenylacetyl}oxy)propyl]-2-methyl-1H-imidazol-3-ium bromide (Compound No. 122aa);
1-[2-({(2R)-2-[(1R)-3,3-Difluorocyclohexyl]-2-hydroxy-2-phenylacetyl}oxy)ethyl]-2-methyl-3-(4-methylbenzyl)-1H-imidazol-3-ium bromide (Compound No. 123aa);
1-[2-({(2R)-2-[(1R)-3,3-Difluorocyclohexyl]-2-hydroxy-2-phenylacetyl}oxy)ethyl]-3-(4-fluorobenzyl)-2-methyl-1H-imidazol-3-ium bromide (Compound No. 124aa);
3-[2-(1,3-Benzodioxol-5-yl)ethyl]-1-(3-[cycloheptyl(hydroxy)phenylacetyl]oxy}propyl)-2-methyl-1H-imidazol-3-ium bromide (Compound No. 125aa);
1-[3-({(2R)-2-[(1R)-3,3-Difluorocyclopentyl]-2-hydroxy-2-phenylacetyl}oxy)propyl]-3-(4-fluorobenzyl)-2-methyl-1H-imidazol-3-ium bromide (Compound No. 126aa);
1-(3-{[Cycloheptyl(hydroxy)phenylacetyl]oxy}propyl)-3-(4-fluorobenzyl)-2-methyl-1H-imidazol-3-ium bromide (Compound No. 127aa);
3-(4-Bromobenzyl)-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 128aa);
3-(4-Fluorobenzyl)-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 129aa);

3-Benzyl-1-[2-({(2R)-2-[(1R)-3,3-difluorocyclopentyl]-2-hydroxy-2-phenylacetyl}oxy)ethyl]-2-methyl-1H-imidazol-3-ium chloride (Compound No. 130aa);

3-Benzyl-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 131aa);

1-[2-({(2R)-2-[(1R)-3,3-Difluorocyclopentyl]-2-hydroxy-2-phenylacetyl}oxy)ethyl]-2,3-dimethyl-1H-imidazol-3-ium bromide (Compound No. 132aa);

1-[3-({(2R)-2-[(1R)-3,3-Difluorocyclopentyl]-2-hydroxy-2-phenylacetyl}oxy)propyl]-3-isopropyl-2-methyl-1H-imidazol-3-ium bromide (Compound No. 133aa);

1-[3-({(2R)-2-[(1R)-3,3-Difluorocyclopentyl]-2-hydroxy-2-phenylacetyl}oxy)propyl]-3-ethyl-2-methyl-1H-imidazol-3-ium iodide (Compound No. 134aa);

3-(Cyclopropylmethyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 135aa);

3-(Cyclohexylmethyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 136aa);

1-[3-({(2R)-2-[(1R)-3,3-Difluorocyclopentyl]-2-hydroxy-2-phenylacetyl}oxy)propyl]-2-methyl-3-[4-(trifluoromethyl)benzyl]-1H-imidazol-3-ium chloride (Compound No. 137aa);

3-(4-Chlorobenzyl)-1-[3-({(2R)-2-[(1R)-3,3-difluorocyclopentyl]-2-hydroxy-2-phenylacetyl}oxy)propyl]-2-methyl-1H-imidazol-3-ium chloride (Compound No. 138aa);

3-(3-Bromobenzyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 139aa);

1-[3-({(2R)-2-[(1R)-3,3-Difluorocyclopentyl]-2-hydroxy-2-phenylacetyl}oxy)propyl]-3-(4-fluorobenzyl)-2-methyl-1H-imidazol-3-ium chloride (Compound No. 140aa);

3-Benzyl-1-[3-({(2R)-2-[(1R)-3,3-difluorocyclopentyl]-2-hydroxy-2-phenylacetyl}oxy)propyl]-2-methyl-1H-imidazol-3-ium chloride (Compound No. 141aa);

1-[3-({(2R)-2-[(1R)-3,3-Difluorocyclopentyl]-2-hydroxy-2-phenylacetyl}oxy)propyl]-2-methyl-3-(2-phenylethyl)-1H-imidazol-3-ium bromide (Compound No. 142aa);

1-[3-({(2R)-2-[(1R)-3,3-Difluorocyclopentyl]-2-hydroxy-2-phenylacetyl}oxy)propyl]-2,3-dimethyl-1H-imidazol-3-ium bromide (Compound No. 143aa);

1-[3-({(2R)-2-[(1R)-3,3-Difluorocyclohexyl]-2-hydroxy-2-phenylacetyl}oxy)propyl]-3-(4-fluorobenzyl)-2-methyl-1H-imidazol-3-ium bromide (Compound No. 144aa);

3-Benzyl-1-{3-[2-cycloheptyl(hydroxy)phenylacetoxy]propyl}-2-methyl-1H-imidazol-3-ium chloride (Compound No. 145aa);

1-(3-{[2-Cycloheptyl(hydroxy)phenylacetyl]oxy}propyl)-2,3-dimethyl-1H-imidazo1-3-ium bromide (Compound No. 146aa);

1-[4-({(2R)-2-[(1R)-3,3-Difluorocyclopentyl]-2-hydroxy-2-phenylacetyl}oxy)butyl]-2,3-dimethyl-1H-imidazol-3-ium bromide (Compound No. 147aa);

3-Benzyl-1-[4-({(2R)-2-[(1R)-3,3-difluorocyclopentyl]-2-hydroxy-2-phenylacetyl}oxy)butyl]-2-methyl-1H-imidazol-3-ium bromide (Compound No. 148aa);

3-Benzyl-1-[2-({(2R)-2-[(1R)-3,3-difluorocyclohexyl]-2-hydroxy-2-phenylacetyl}oxy)ethyl]-2-methyl-1H-imidazol-3-iumn bromide (Compound No. 149aa);

3-Benzyl-1-[3-({(2R)-2-[(1R)-3,3-difluorocyclohexyl]-2-hydroxy-2-phenylacetyl}oxy)propyl]-2-methyl-1H-imidazol-3-ium chloride (Compound No. 150aa);

1-[4-({(2R)-2-[(1R)-3,3-Difluorocyclohexyl]-2-hydroxy-2-phenylacetyl}oxy)butyl]-2,3-dimethyl-1H-imidazol-3-ium bromide (Compound No. 151aa);

3-Benzyl-1-[4-({(2R)-2-[(1R)-3,3-difluorocyclohexyl]-2-hydroxy-2-phenylacetyl}oxy)butyl]-2-methyl-1H-imidazol-3-ium bromide (Compound No. 152aa);

3-(4-*tert*-Butylbenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 153aa);

3-(Biphenyl-4-ylmethyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 154aa);

3-(2,5-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 155aa);

3-(2,4-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 156aa);

3-(3,5-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 157aa);

3-(3,4-Difluorobenzyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 158aa);

1-{3-[2-Cyclopentyl(hydroxy)phenylacetoxy]propyl}-2-methyl-3-(pyridin-4-ylmethyl)-1H-imidazol-3-ium bromide (Compound No. 159aa);

1-[3-({(2R)-2-[(1R)-3,3-Difluorocyclopentyl]-2-hydroxy-2-phenylacetyl}oxy)propyl]-3-(1-ethylpropyl)-2-methyl-1H-imidazol-3-ium bromide (Compound No. 160aa);

3-(1,3-Benzodioxo1-5-ylmethyl)-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 161aa);

1-[3-({(2R)-2-[(1R)-3,3-Difluorocyclopentyl]-2-hydroxy-2-phenylacetyl}oxy)propyl]-2-methyl-3-propyl-1H-imidazol-3-ium bromide (Compound No. 162aa);

1-[3-({(2R)-2-[(1R)-3,3-Difluorocyclopentyl]-2-hydroxy-2-phenylacetyl}oxy)propyl]-3-isobutyl-2-methyl-1H-imidazol-3-ium bromide (Compound No. 163aa);

3-Butyl-1-[3-({(2R)-2-[(1R)-3,3-difluorocyclopentyl]-2-hydroxy-2-phenylacetyl}oxy)propyl]-2-methyl-1H-imidazol-3-ium bromide (Compound No. 164aa); 1-{2-[2-Hydroxy(phenyl)-2-thienylacetoxy]ethyl}-2-methyl-3-(2-phenylethyl)-1H-imidazol-3-ium bromide (Compound No. 165aa);

3-(Cyclopropylmethyl)-1-{2-[2-hydroxy(phenyl)-2-thienylacetoxy]ethyl}-2-methyl-1H-imidazol-3-ium bromide (Compound No. 166aa);

3-Benzyl-1-{2-[2-hydroxy(phenyl)-2-thienylacetoxy]ethyl}-2-methyl-1H-imidazo1-3-ium bromide (Compound No. 167aa);

1-{2-[2-Hydroxy(phenyl)-2-thienylacetoxy]ethyl}-2,3-dimethyl-1H-imidazol-3-ium bromide (Compound No. 168aa);

3-(Cyclopropylmethyl)-1-(2-{2-hydroxy[bis(3-methylphenyl)]acetoxy}ethyl)-2-methyl-1H-imidazol-3-ium bromide (Compound No. 169aa);

1-(2-{2-Hydroxy[bis(3-methylphenyl)]acetoxy}ethyl)-2,3-dimethyl-1H-imidazo1-3-ium bromide (Compound No. 170aa);

3-Benzyl-1-(2-{2-hydroxy[bis(3-methylphenyl)]acetoxy}ethyl)-2-methyl-1H-imidazol-3-ium bromide (Compound No. 171aa);

1-{2-[2,2-Bis(4-fluorophenyl)(hydroxy)acetoxy]ethyl}-2,3-dimethyl-1H-imidazol-3-ium bromide (Compound No. 172aa);

3-Benzyl-1-{2-[2,2-bis(4-fluorophenyl)(hydroxy)acetoxy]ethyl}-2-methyl-1H-imidazo1-3-ium bromide (Compound No. 173aa);

1- {2-[2,2-Bis(4-fluorophenyl)(hydroxy)acetoxy]ethyl}-3-(cyclopropylmethyl)-2-methyl-1H-imidazol-3-ium bromide (Compound No. 174aa);

3-Benzyl-1-{3-[2,2-bis(4-fluorophenyl)(hydroxy)acetoxy]propyl}-2-methyl-1H-imidazol-3-ium bromide (Compound No. 175aa);

1-{3-[2,2-*bis*(4-Fluorophenyl)(hydroxy)acetoxy]propyl}-3-(cyclopropylmethyl)-2-methyl-1H-imidazol-3-ium bromide (Compound No. 176aa);

1-{3-[2,2-*bis*-(4-Fluorophenyl)(hydroxy)acetoxy]propyl}-3-ethyl-2-methyl-1H-imidazol-3-ium bromide (Compound No. 177aa);

1-[3-({(2R)-2-[(1R)-3,3-Difluorocyclopentyl]-2-hydroxy-2-phenylacetyl}oxy)propyl]-2-methyl-3-(3-phenoxypropyl)-1H-imidazol-3-ium bromide (Compound No. 178aa);

1-[3-({(2R)-2-[(1R)-3,3-Difluorocyclohexyl]-2-hydroxy-2-phenylacetyl}oxy)propyl]-2-methyl-3-(3-phenoxypropyl)-1H-imidazol-3-ium bromide (Compound No. 179aa);

1-[3-({(2R)-2-[(1R)-3,3-Difluorocyclohexyl]-2-hydroxy-2-phenylacetyl}oxy)propyl]-2-methyl-3-propyl-1H-imidazol-3-ium bromide (Compound No. 180aa);

1-[3-({(2R)-2-[(1R)-3,3-Difluorocyclohexyl]-2-hydroxy-2-phenylacetyl}oxy)propyl]-3-isopropyl-2-methyl-1H-imidazol-3-ium bromide (Compound No. 181aa);

3-(Cyclopropylmethyl)-1-[3-({(2R)-2-[(1R)-3,3-difluorocyclohexyl]-2-hydroxy-2-phenylacetyl}oxy)propyl]-2-methyl-1H-imidazol-3-ium bromide (Compound No. 182aa);

3-(Cyclohexylmethyl)-1-[3-({(2R)-2-[(1R)-3,3-difluorocyclohexyl]-2-hydroxy-2-phenylacetyl}oxy)propyl]-2-methyl-1H-imidazol-3-ium bromide (Compound No. 183aa);

1-[3-({(2R)-2-[(1R)-3,3-Difluorocyclohexyl]-2-hydroxy-2-phenylacetyl}oxy)propyl]-2-methyl-3-(2-phenylethyl)-1H-imidazol-3-ium bromide (Compound No. 184aa);

1-[3-({(2R)-2-[(1R)-3,3-Difluorocyclohexyl]-2-hydroxy-2-phenylacetyl}oxy)propyl]-2,3-dimethyl-1H-imidazol-3-ium bromide (Compound No. 185aa);

1-{3-[2,2-*bis*(4-Fluorophenyl)(hydroxy)acetoxy]propyl}-2,3-dimethyl-1H-imidazol-3-ium bromide (Compound No. 186aa);

3-(4-Bromobenzyl)-1-(3-{2-hydroxy[bis(3-methylphenyl)]acetoxy}propyl)-2-methyl-1H-imidazol-3-ium bromide (Compound No. 187aa);

3-Benzyl-1-(3-{2-hydroxy[bis(3-methylphenyl)]acetoxy}propyl)-2-methyl-1H-imidazol-3-ium bromide (Compound No. 188aa);

3-(Cyclopropylmethyl)-1-(3-{2-hydroxy[bis(3-methylphenyl)]]acetoxy}propyl)-2-methyl-1H-imidazol-3-ium bromide (Compound No. 189aa);
3-Ethyl-1-(3-{2-hydroxy[bis(3-methylphenyl)]acetoxy}propyl)-2-methyl-1H-imidazo1-3-ium bromide (Compound No. 190aa);
1-(3-{2-Hydroxy[bis(3-methylphenyl)]acetoxy}propyl)-2,3-dimethyl-1H-imidazo1-3-ium bromide (Compound No. 191aa);
3-(4-Bromobenzyl)-1-{3-[2-hydroxy(phenyl)-2-thienylacetoxy]propyl}-2-methyl-1H-imidazol-3-ium bromide (Compound No. 192aa);
3-Benzyl-1-{3-[2-hydroxy(phenyl)-2-thienylacetoxy]propyl}-2-methyl-1H-imidazol-3-ium bromide (Compound No. 193aa);
3-(Cyclopropylmethyl)-1-{3-[2-hydroxy(phenyl)-2-thienylacetoxy]propyl}-2-methyl-1H-imidazol-3-ium bromide (Compound No. 194aa);
3-Ethyl-1-{3-[2-hydroxy(phenyl)-2-thienylacetoxy]propyl}-2-methyl-1H-imidazol-3-ium bromide (Compound No. 195aa);
1-{3-[2-Hydroxy(phenyl)-2-thienylacetoxy]propyl}-2,3-dimethyl-1H-imidazol-3-ium bromide (Compound No. 196aa);
1-[2-({(2R)-2-[(1R)-3,3-Difluorocyclopentyl]-2-hydroxy-2-phenylacetyl}oxy)ethyl]-2-methyl-3-(3-phenoxypropyl)-1H-imidazol-3-ium bromide (Compound No. 197aa);
3-(Cyclopropylmethyl)-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 198aa);
3-Butyl-1-[2-({(2R)-2-[(1R)-3,3-difluorocyclopentyl]-2-hydroxy-2-phenylacetyl}oxy)ethyl]-2-methyl-1H-imidazol-3-ium bromide (Compound No. 199aa);
1-[2-({(2R)-2-[(1R)-3,3-Difluorocyclopentyl]-2-hydroxy-2-phenylacetyl}oxy)ethyl]-2-methyl-3-propyl-1H-imidazol-3-ium bromide (Compound No. 200aa);
1-[2-({(2R)-2-[(1R)-3,3-Difluorocyclopentyl]-2-hydroxy-2-phenylacetyl}oxy)ethyl]-3-ethyl-2-methyl-1H-imidazol-3-ium bromide (Compound No. 201aa);
3-Benzyl-1-(2-[2-hydroxy(di-2-thienyl)acetoxy]ethyl)-2-methyl-1H-imidazol-3-ium bromide (Compound No. 202aa);
3-(Cyclopropylmethyl)-1-{2-[2-hydroxy(di-2-thienyl)acetoxy]ethyl}-2-methyl-1H-imidazol-3-ium bromide (Compound No. 203aa);
3-Ethyl-1-{2-[2-hydroxy(di-2-thienyl)acetoxy]ethyl}-2-methyl-1H-imidazo1-3-ium bromide (Compound No. 204aa);
1-{2-[2-Hydroxy(di-2-thienyl)acetoxy]ethyl}-2,3-dimethyl-1H-imidazol-3-ium bromide (Compound No. 205aa);
1-{3-[2-Hydroxy(di-2-thienyl)acetoxy]propyl}-2-methyl-3-(2-phenylethyl)-1H-imidazol-3-ium bromide (Compound No. 206aa);
1-{3-[2-Hydroxy(di-2-thienyl)acetoxy]propyl}-2-methyl-3-(3-phenoxypropyl)-1H-imidazol-3-ium bromide (Compound No. 207aa);
3-Butyl-1-{3-[2-hydroxy(di-2-thienyl)acetoxy]propyl}-2-methyl-1H-imidazol-3-ium bromide (Compound No. 208aa);
3-Ethyl-1-{3-[2-hydroxy(di-2-thienyl)acetoxy]propyl}-2-methyl-1H-imidazol-3-ium bromide (Compound No. 209aa);
1-{3-[2-Hydroxy(di-2-thienyl)acetoxy]propyl}-2,3-dimethyl-1H-imidazol-3-ium bromide (Compound No. 210aa);
1-(3-{[(2R)-2-Hydroxy-2-phenyl-2-{(1S)-3-[(phenylsulfonyl)amino]cyclopentyl} acetyl]oxy}propyl)-2,3-dimethyl-1H-imidazol-3-ium bromide (Compound No. 211aa);
3-Benzyl-1-(3-{[(2R)-2-hydroxy-2-phenyl-2-{(1S)-3-[(phenylsulfonyl)amino] cyclopentyl}acetyl]oxy}propyl)-2-methyl-1H-imidazol-3-ium bromide (Compound No. 212aa);
3-(4-Bromobenzyl)-1-(3-{[(2R)-2-hydroxy-2-phenyl-2-{(1S)-3-[(phenylsulfonyl)amino] cyclopentyl}acetyl]oxy}propyl)-2-methyl-1H-imidazol-3-ium bromide (Compound No. 213aa);
1-[3-({(2R)-2-[(1S)-3,3-Difluorocyclopentyl]-2-hydroxy-2-phenylacetyl}oxy)propyl]-3-ethyl-2-methyl-1H-imidazol-3-ium bromide (Compound No. 214aa);
1-[3-({(2R)-2-[(1S)-3,3-Difluorocyclopentyl]-2-hydroxy-2-phenylacetyl}oxy)propyl]-2-methyl-3-propyl-1H-imidazol-3-ium bromide (Compound No. 215aa);
3-Butyl-1-[3-({(2R)-2-[(1S)-3,3-difluorocyclopentyl]-2-hydroxy-2-phenylacetyl}oxy)propyl]-2-methyl-1H-imidazol-3-ium bromide (Compound No. 216aa);
1-[3-({(2R)-2-[(1S)-3,3-Difluorocyclopentyl]-2-hydroxy-2-phenylacetyl}oxy)propyl]-2-methyl-3-pentyl-1H-imidazol-3-ium bromide (Compound No. 217aa);
1-[3-({(2R)-2-[(1S)-3,3-Difluorocyclohexyl]-2-hydroxy-2-phenylacetyl}oxy)propyl]-3-ethyl-2-methyl-1H-imidazol-3-ium bromide (Compound No. 218aa);
1-[3-({(2R)-2-[(1S)-3,3-Difluorocyclohexyl]-2-hydroxy-2-phenylacetyl}oxy)propyl]-2-methyl-3-propyl-1H-imidazol-3-ium bromide (Compound No. 219aa);
1-[3-({(2R)-2-[(1S)-3,3-Difluorocyclohexyl]-2-hydroxy-2-phenylacetyl}oxy)propyl]-3-(1-ethylpropyl)-2-methyl-1H-imidazol-3-ium bromide (Compound No. 220aa);
1-[3-({(2R)-2-[(1S)-3,3-Difluorocyclohexyl]-2-hydroxy-2-phenylacetyl}oxy)propyl]-3-isopropyl-2-methyl-1H-imidazol-3-ium bromide (Compound No. 221aa);

1-[3-({(2R)-2-[(1S)-3,3-Difluorocyclohexyl]-2-hydroxy-2-phenylacetyl}oxy)propyl]-2-methyl-3-pentyl-1H-imidazol-3-ium bromide (Compound No. 222aa);

1-[3-({(2R)-2-[(1S)-3,3-Difluorocyclopentyl]-2-hydroxy-2-phenylacetyl}oxy)propyl]-3-isobutyl-2-methyl-1H-imidazol-3-ium bromide (Compound No. 223aa);

1-[3-({(2R)-2-[(1S)-3,3-Difluorocyclopentyl]-2-hydroxy-2-phenylacetyl}oxy)propyl]-3-(1-ethylpropyl)-2-methyl-1H-imidazol-3-ium bromide (Compound No. 224aa);

1-[3-({(2R)-2-[(1S)-3,3-Difluorocyclopentyl]-2-hydroxy-2-phenylacetyl}oxy)propyl]-3-isopropyl-2-methyl-1H-imidazol-3-ium bromide (Compound No. 225 aa);

3-Butyl-1-[3-({(2R)-2-[(1S)-3,3-difluorocyclohexyl]-2-hydroxy-2-phenylacetyl}oxy)propyl]-2-methyl-1H-imidazol-3-ium bromide (Compound No. 226aa);

1-[2-({(2R)-2-[(1S)-3,3-Difluorocyclohexyl]-2-hydroxy-2-phenylacetyl}oxy)ethyl]-3-(1-ethylpropyl)-2-methyl-1H-imidazol-3-ium bromide (Compound No. 227aa);

1-[2-({(2R)-2-[(1S)-3,3-Difluorocyclohexyl]-2-hydroxy-2-phenylacetyl}oxy)ethyl]-2-methyl-3-pentyl-1H-imidazol-3-ium bromide (Compound No. 228aa);

3-Butyl-1-[2-({(2R)-2-[(1S)-3,3-difluorocyclohexyl]-2-hydroxy-2-phenylacetyl}oxy)ethyl]-2-methyl-1H-imidazol-3-ium bromide (Compound No. 229aa);

1-[2-({(2R)-2-[(1S)-3,3-Difluorocyclohexyl]-2-hydroxy-2-phenylacetyl}oxy)ethyl]-2-methyl-3-propyl-1H-imidazol-3-ium bromide (Compound No. 230aa);

1-[2-({(2R)-2-[(1S)-3,3-Difluorocyclohexyl]-2-hydroxy-2-phenylacetyl}oxy)ethyl]-3-ethyl-2-methyl-1H-imidazol-3-ium bromide (Compound No. 231aa);

1-[2-({(2R)-2-[(1S)-3,3-Difluorocyclohexyl]-2-hydroxy-2-phenylacetyl}oxy)ethyl]-2,3-dimethyl-1H-imidazol-3-ium bromide (Compound No. 232aa);

1-[2-({(2R)-2-[(1S)-3,3-Difluorocyclopentyl]-2-hydroxy-2-phenylacetyl}oxy)ethyl]-3-(1-ethylpropyl)-2-methyl-1H-imidazol-3-ium bromide (Compound No. 233aa);

1-[2-({(2R)-2-[(1S)-3,3-Difluorocyclopentyl]-2-hydroxy-2-phenylacetyl}oxy)ethyl]-3-isopropyl-2-methyl-1H-imidazol-3-ium bromide (Compound No. 234aa);

1-[2-({(2R)-2-[(1S)-3,3-Difluorocyclopentyl]-2-hydroxy-2-phenylacetyl}oxy)ethyl]-2-methyl-3-pentyl-1H-imidazol-3-ium bromide (Compound No. 235aa);

3-Butyl-1-[2-({(2R)-2-[(1S)-3,3-difluorocyclopentyl]-2-hydroxy-2-phenylacetyl}oxy)ethyl]-2-methyl-1H-imidazol-3-ium bromide (Compound No. 236aa);

1-[2-({(2R)-2-[(1S)-3,3-Difluorocyclopentyl]-2-hydroxy-2-phenylacetyl)oxy)ethyl]-2-methyl-3-propyl-1H-imidazol-3-ium bromide (Compound No. 237aa);

1-[2-({(2R)-2-[(1S)-3,3-Difluorocyclopentyl]-2-hydroxy-2-phenylacetyl}oxy)ethyl]-3-ethyl-2-methyl-1H-imidazol-3-ium bromide (Compound No. 238aa);

2-(2-Methyl-1H-imidazol-l-yl)ethyl(2R)-[(1R)-3,3-difluorocyclohexyl](hydroxy) phenylacetate (Compound No. 239aa);

3-(2-Methyl-1*H*-imidazol-1-yl)propyl (2*R*)- [(1*R*)-3,3-difluorocyclohexyl](hydroxy) phenylacetate (Compound No. 240aa);

3-(2-Methyl-1H-imidazol-1-yl)propyl cycloheptyl(hydroxy)phenylacetate (Compound No. 24 1 aa);

2-(2-Methyl-1H-imidazol-1-yl)ethyl(2R)-[(1S)-3,3-difluorocyclohexyl](hydroxy) phenylacetate (Compound No. 242aa);

4-(2-Methyl-1H-imidazol-1-yl)butyl(2R)-[(1R)-3,3-difluorocyclopentyl](hydroxy) phenylacetate (Compound No. 243aa);

4-(2-Methyl-1H-imidazol-1-yl)butyl(2R)-[(1R)-3,3-difluorocyclohexyl](hydroxy) phenylacetate (Compound No. 244aa);

2-(2-Methyl-1*H*-imidazol-1-yl)ethyl hydroxy(phenyl)-2-thienylacetate (Compound No. 245aa);

2-(2-Methyl-1*H*-imidazol-1-yl)ethyl hydroxy[bis(3-methylphenyl)]acetate (Compound No. 246aa);

2-(2-Methyl-1H-imidazol-1-yl)ethyl bis(4-fluorophenyl)(hydroxy)acetate (Compound No. 247aa);

3-(2-Methyl-1H-imidazol-1-yl)propylbis(4-fluorophenyl)(hydroxy)acetate (Compound No. 248aa);

3-(2-Methyl-1H-imidazo1-1-yl)propylhydroxy[bis(3-methylphenyl)]acetate(Compound No. 249aa);

3-(2-Methyl-1*H*-imidazol-1-yl)propyl hydroxy(phenyl)2-thienylacetate(Compound No. 250aa);

2-(2-Methyl-1H-imidazol-1-yl)ethyl hydroxy(di-2-thienyl)acetate (Compound No. 251aa);

3-(2-Methyl-1H-imidazol-1-yl)propyl hydroxy(di-2-thienyl)acetate (Compound No. 252aa);

3-(2-Methyl-1H-imidazol-1-yl)propyl (2R)-hydroxy(phenyl) {(1S)-3-[(phenylsulfonyl) amino] cyclopentyl} acetate (Compound No. 253aa);

3-(2-Methyl-1H-imidazol-1-yl)propyl (2R)-[(1S)-3,3-difluorocyclohexyl](hydroxy) phenylacetate (Compound No. 254aa);

3-butyl-1-[3-({(2*R*)-2-[(1*R*)-3,3-difluorocyclohexyl]-2-hydroxy-2-phenylacetyl}oxy)propyl]-2-methyl-1*H*-imidazol-3-ium bromide (Compound No. 255aa);
1-[3-({(2*R*)-2-[(1*R*)-3,3-difluorocyclohexyl]-2-hydroxy-2-phenylacetyl}oxy)propyl]-2-methyl-3-pentyl-1*H*-imidazol-3-ium bromide(Compound No. 256aa);
1-[3-( { (2*R*)- 2-[(1*R*)- 3,3- difluorocyclohexyl]- 2- hydroxy- 2- phenylacetyl} oxy) propyl]- 3-(1- ethylpropyl)- 2- methyl-1*H*-imidazol-3-ium bromide(Compound No. 257aa);
1-[3-( { (2*R*)- 2-[(1*R*)- 3,3- difluorocyclohexyl]- 2- hydroxy- 2- phenylacetyl} oxy) propyl]- 2- methyl- 3-(2- methylpropyl)-1*H*-imidazol-3-ium bromide(Compound No. 258aa);
1-(3-{[(3,3-difluorocyclopentyl)(hydroxy)thiophen-2-ylacetyl]oxy}propyl)-3-ethyl-2-methyl-1*H*-imidazol-3-ium bromide(Compound No. 259aa);
1-(3-{[(3,3-difluorocyclopentyl)(hydroxy)thiophen-2-ylacetyl]oxy}propyl)-2,3-dimethyl-1*H*-imidazol-3-ium bromide (Compound No. 260aa); and
3-benzyl-1-(3-{[(3,3-difluorocyclopentyl)(hydroxy)thiophen-2-ylacetyl]oxy}propyl)-2-methyl-1*H*-imidazol-3-ium bromide(Compound No. 261aa).

**5.** The pharmaceutical composition of claim 1, wherein the one or more β2-agonists are selected from albuterol, salbutamol, biltolterol, pirbuterol, levosalbutamol, tulobuterol, terbutaline, bambuterol, metaproterenol, fenoterol, salmeterol, carmoterol, arformoterol, formoterol, or their pharmaceutically acceptable salts or solvates thereof.

**6.** The pharmaceutical composition of claim 1, wherein the one or more corticosteroids are selected from alclometasone, amcinonide, amelometasone, beclometasone, betamethasone, budesonide, ciclesonide, clobetasol, cloticasone, cyclomethasone, deflazacort, deprodone, dexbudesonide, diflorasone, difluprednate, fluticasone, flunisolide, halometasone, halopredone, hydrocortisone, hydrocortisone, methylprednisolone, mometasone, prednicarbate, prednisolone, rimexolone, tixocortol, triamcinolone, ulobetasol, or pharmaceutically acceptable salts or solvates thereof.

**7.** The pharmaceutical composition of claim 1, wherein one or more PDE-IV inhibitor_and one or more muscarinic receptor antagonists (MRA) are present in a ratio from 1:10 to 10:1.

**8.** The pharmaceutical composition of claim 1, wherein one or more PDE-IV inhibitor and one or more β2-agonist are present in a ratio from 1:10 to 10:1.

**9.** The pharmaceutical composition of claim 1, wherein one or more PDE-IV inhibitor and one or more p38 MAP Kinase inhibitors are present in a ratio from 1:10 to 10:1.

**10.** The pharmaceutical composition of claim 1, wherein one or more PDE-IV inhibitor and one or more corticosteroids are present in a ratio from 1:10 to 10:1.

**11.** A method of treating autoimmune, inflammatory or allergic diseases or disorders comprising administering one or more pharmaceutical compositions of claim 1.

**12.** The method of claim 11, wherein the autoimmune, inflammatory or allergic diseases or disorders are selected from respiratory disorder, asthma, chronic bronchitis, chronic obstructive pulmonary disease, whooping cough, eosinophilic granuloma, psoriasis and other benign or malignant proliferative skin diseases, eczema, inflammatory bowel disease, endotoxic shock, anaphylactic shock, laminitis in horses, septic shock, ulcerative colitis, Crohn's disease, reperfusion injury of the myocardium and brain, inflammatory arthritis, perodontitis, chronic glomerulonephritis, atopic dermatitis, urticaria, adult respiratory distress syndrome, infant respiratory distress syndrome, transplant rejection, rhinitis, pruritus, diabetes insipidus, eye diseases, allergic rhinitis, allergic conjunctivitis, vernal conjunctivitis, arterial restenosis, ortherosclerosis, atherosclerosis, neurogenic inflammation, pain, cough, rheumatoid arthritis, osteoporosis, osteoarthritis, inflammation, ankylosing spondylitis, transplant rejection, graft versus host disease, hypersecretion of gastric acid, bacterial, fungal induced sepsis, viral induced sepsis, fungal induced septic shock, viral induced septic shock, inflammation-mediated chronic tissue degeneration, cytokine-mediated chronic tissue degeneration, osteoarthritis, cancer, cachexia, muscle wasting, depression memory impairment, tumor growth, cancerous invasion of normal tissues Hashimoto's thyroiditis (underactive thyroid), Graves' disease (overactive thyroid), Lupus and acquired immuno deficiency syndrome.

European Patent Office

# EUROPEAN SEARCH REPORT

Application Number
EP 08 15 4898

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>Y | WO 2007/045980 A (RANBAXY LAB LTD [IN]; RAY ABHIJIT [IN]; DASTIDAR SUNANDA G [IN]; SHIRU) 26 April 2007 (2007-04-26)<br>* claims 1-11 *<br>----- | 1-3,5-12<br><br><br><br>4 | INV.<br>A61K31/4174<br>A61K31/422<br>A61K31/46<br>A61K31/57<br>A61K45/06<br>A61K31/519<br>A61P29/00<br>A61P37/00<br>A61P11/00 |
| X<br><br><br><br><br>Y | WO 2007/134828 A (NOVARTIS AG [CH]; NOVARTIS PHARMA GMBH [AT]; BRUCE IAN [GB]; HAYLER JU) 29 November 2007 (2007-11-29)<br>* claims 1,2; compound 10 *<br>* page 26, lines 12-16 - line 25 *<br>----- | 1,11,12<br><br><br><br><br>4 | |
| Y | WO 2007/077510 A (RANBAXY LAB LTD [IN]; KUMAR NARESH [IN]; KAUR JASKIRAN [IN]; PALLE VEN) 12 July 2007 (2007-07-12)<br>* compounds 1-113 *<br>* page 26, lines 3-12 *<br>* pages 59-61 *<br>----- | 1,2,4,<br>11,12 | |
| Y | WO 2007/045979 A (RANBAXY LAB LTD [IN]; RAY ABHIJIT [IN]; DASTIDAR SUNANDA G [IN]; SHIRU) 26 April 2007 (2007-04-26)<br>* claim 1; compounds 1AA-167AA *<br>----- | 1,2,4,<br>11,12 | TECHNICAL FIELDS SEARCHED (IPC)<br>A61K |

The present search report has been drawn up for all claims

| Place of search | Date of completion of the search | Examiner |
|---|---|---|
| Munich | 12 September 2008 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 08 15 4898

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2008

| Patent document cited in search report | Publication date | Patent family member(s) | Publication date |
|---|---|---|---|
| WO 2007045980 A | 26-04-2007 | AU 2006305620 A1 | 26-04-2007 |
| | | AU 2008203254 A1 | 14-08-2008 |
| | | CA 2626628 A1 | 26-04-2007 |
| | | EP 1948167 A1 | 30-07-2008 |
| WO 2007134828 A | 29-11-2007 | AR 061057 A1 | 30-07-2008 |
| WO 2007077510 A | 12-07-2007 | AU 2006334107 A1 | 12-07-2007 |
| WO 2007045979 A | 26-04-2007 | AU 2006305619 A1 | 26-04-2007 |
| | | CA 2626612 A1 | 26-04-2007 |
| | | EP 1948164 A1 | 30-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60605344 B
- US 4285937 A
- EP 1344526 A
- WO 2602DEL2005 A
- US 60598621 B
- US 60630517 B
- US 1098DEL2005 A
- US 211DEL2005 A
- WO 9847892 A
- WO 0043384 A
- WO 9827098 A
- WO 9725048 A
- US 3705233 A
- US 3644353 A
- US 3642896 A
- US 3700681 A
- US 4579985 A
- US 3994974 A
- US 3937838 A
- US 4419364 A
- US 5126375 A
- US 5243076 A
- US 4992474 A
- US 4011258 A
- US 3312590 A
- US 3983233 A
- US 3929768 A
- US 3721687 A
- US 3436389 A
- US 3506694 A
- US 3639434 A
- US 3992534 A
- US 3928326 A
- US 3980778 A
- US 3780177 A
- US 3652554 A
- US 3947478 A
- US 4076708 A
- US 4124707 A
- US 4158055 A
- US 4298604 A
- US 4335121 A
- US 4081541 A
- US 4226862 A
- US 4290962 A
- US 4587236 A
- US 4472392 A
- US 4472393 A
- US 4242334 A
- US 4014909 A
- US 4098803 A
- US 4619921 A
- US 5482934 A
- US 5837699 A
- US 5889015 A
- US 5278156 A
- US 5015746 A
- US 5976573 A
- US 6337324 A
- US 6057307 A
- US 6723713 A
- US 6127353 A
- US 6180781 A
- WO 0232899 A
- WO 0232898 A
- US 5565473 A
- US 5583152 A
- US 4859692 A
- US 4780469 A


### Non-patent literature cited in the description


- **Lambrecht et al.** *Trends in Pharmacol. Sci.,* 1989, 60
- **Birdsall et al.** *Trends in Pharmacol. Sci.,* 1983, vol. 4, 459
- **Coruzzi et al.** *Arch. Int. Pharmacodyn. Ther.,* 1989, vol. 302, 232
- **Kawashima et al.** *Gen. Pharmacol.,* 1990, vol. 21, 17